# EUROPEAN PATENT SPECIFICATION

(11) **EP 2 935 611 B1**
(45) Date of publication and mention of the grant of the patent: **07.07.2021**
(21) Application number: 14790777.8
(22) Date of filing: 14.10.2014
(51) Int. Cl.: C12P 21/00, C12P 21/08, C07K 16/32, A61K 47/68, A61K 39/395, A61P 35/00, A61P 43/00, A61K 39/00

(54) **GLYCOENGINEERED ANTIBODY, ANTIBODY-CONJUGATE AND METHODS FOR THEIR PREPARATION**
DURCH GLYCOENGINEERING ERHALTENE ANTIKÖRPER, ANTIKÖRPER-KONJUGAT, UND VERFAHREN ZU IHRER HERSTELLUNG
ANTICORPS OBTENU PAR GLYCO-INGÉNIERIE, CONJUGUÉ D'ANTICORPS, ET PROCÉDÉS POUR LEUR PRÉPARATION

(30) Priority: 14.10.2013 EP 13188585; 23.04.2014 EP 14165581
(43) Date of publication of application: 28.10.2015
(73) Proprietor: SynAffix B.V., 5342 CC Oss (NL)
(72) Inventor: VAN DELFT, Floris Louis, NL-6524 KZ Nijmegen (NL); VAN GEEL, Remon, 5396 PM Lithoijen (NL); WIJDEVEN, Maria Antonia, NL-6663 HR Lent (NL)
(74) Representative: Nederlandsch Octrooibureau
(86) International application number: PCT/NL2014/050717
(87) International publication number: WO 2015/057066

(56) References cited:
- WO-A1-2014/065661
- MERCER ET AL: "Use of novel mutant galactosyltransferase for the bioconjugation of terminal N-acetylglucosamine (GlcNAc) residues on live cell surface", BIOCONJUGATE CHEMISTRY, vol. 24, 22 December 2012 (2012-12-22), pages 144-152, XP055061203,
- BOEGGEMAN ET AL: "Site specific conjugation of fluoroprobes to the remodeled Fc N-glycans of monoclonal antibodies using mutant glycosyltransferases: Application for cell surface antigen detection", BIOCONJUGATE CHEMISTRY, vol. 20, 2009, pages 1228-1236, XP055061205, cited in the application
- BOURGEAU: "Dévelopement d'un système de régénération d'UDP-GalNAc pour la glycosylation enzymatique d'oligosaccharides et de peptides d'intérêt thérapeutique", HAL - archives ouvertes Thesis (University of Orléans, France), 2007, pages 1, 32-58, XP002735036, Retrieved from the Internet: URL:https://tel.archives-ouvertes.fr/tel-0 0160999/document [retrieved on 2015-01-27]
- WANG ET AL: "Enrichment and site mapping of O-linked N-acetylglucosamine by a combination of chemical/enzymatic tagging, photochemical cleavage, and electron transfer dissociation mass spectrometry", MOLECULAR & CELLULAR PROTEOMICS, vol. 9, 2010, pages 153-160, XP002735035,
- N.N.: "A new site specific antibody conjugation using bacterial transglutaminase", Innate Pharma ADC Summit, San Francisco, 2013, 15 October 2013 (2013-10-15), pages 1-29, XP002735090, Retrieved from the Internet: URL:http://innate-pharma.com/sites/default /files/iph_adc_summit_2013.pdf [retrieved on 2015-01-27]
- SHARMA ET AL: "Design and synthesis of LNA based mercaptoacetamido-linked nucleoside dimmers", CARBOHYDRATE NEWS LETTER (INDIA), vol. 14, December 2013 (2013-12), page 15, XP002734784,
- ROCHEFORT ET AL: "Metabolic exploitation of the sialic acid biosynthetic pathway to generate site-specific labeled antibodies", GLYCOBIOLOGY, vol. 24, 22 October 2013 (2013-10-22), pages 62-69, XP002735091,
- None

### SEQUENCE LISTING

<110> SynAffix B.V.
<120> Glycoengineered antibody, antibody-conjugate and methods for their preparation
<130> P6047951PCT
<150> EP 14165581.1
   <151> 2014-04-23
<150> EP 13188585.7
   <151> 2013-10-14
<160> 24
<170> PatentIn version 3.3
<210> 1
   <211> 39
   <212> DNA
   <213> artificial
<220>
   <223> primer sequence
<400> 1
   cagcgacata tgtcgctgac cgcatgccct gaggagtcc 39
<210> 2
   <211> 36
   <212> DNA
   <213> artificial
<220>
   <223> primer sequence
<400> 2
   gacacctcca aagttctgca cgtaaggtag gctaaa 36
<210> 3
   <211> 39
   <212> DNA
   <213> artificial
<220>
   <223> primer sequence
<400> 3
   ctgatggatg gatccctagc tcggcgtccc gatgtccac 39
<210> 4
   <211> 36
   <212> DNA
   <213> artificial
<220>
   <223> primer sequence
<400> 4
   ccttacgtgc agaactttgg aggtgtctct gctcta 36
<210> 5
   <211> 36
   <212> DNA
   <213> artificial
<220>
   <223> primer sequence
<400> 5
   gacacctcca aaaaactgca cgtaaggtag gctaaa 36
<210> 6
   <211> 36
   <212> DNA
   <213> artificial
<220>
   <223> primer sequence
<400> 6
   ccttacgtgc agttttttgg aggtgtctct gctcta 36
<210> 7
   <211> 36
   <212> DNA
   <213> artificial
<220>
   <223> primer sequence
<400> 7
   gacacctcca aacatctgca cgtaaggtag gctaaa 36
<210> 8
   <211> 36
   <212> DNA
   <213> artificial
<220>
   <223> primer sequence
<400> 8
   ccttacgtgc agatgtttgg aggtgtctct gctcta 36
<210> 9
   <211> 36
   <212> DNA
   <213> artificial
<220>
   <223> primer sequence
<400> 9
   gacacctcca aaaatctgca cgtaaggtag gctaaa 36
<210> 10
   <211> 36
   <212> DNA
   <213> artificial
<220>
   <223> primer sequence
<400> 10
   ccttacgtgc agatttttgg aggtgtctct gctcta 36
<210> 11
   <211> 36
   <212> DNA
   <213> artificial
<220>
   <223> primer sequence
<400> 11
   gacacctcca aacacctgca cgtaaggtag gctaaa 36
<210> 12
   <211> 36
   <212> DNA
   <213> artificial
<220>
   <223> primer sequence
<400> 12
   ccttacgtgc aggtgtttgg aggtgtctct gctcta 36
<210> 13
   <211> 36
   <212> DNA
   <213> artificial
<220>
   <223> primer sequence
<400> 13
   gacacctcca aacgcctgca cgtaaggtag gctaaa 36
<210> 14
   <211> 36
   <212> DNA
   <213> artificial
<220>
   <223> primer sequence
<400> 14
   ccttacgtgc aggcgtttgg aggtgtctct gctcta 36
<210> 15
   <211> 36
   <212> DNA
   <213> artificial
<220>
   <223> primer sequence
<400> 15
   gacacctcca aagccctgca cgtaaggtag gctaaa 36
<210> 16
   <211> 36
   <212> DNA
   <213> artificial
<220>
   <223> primer sequence
<400> 16
   ccttacgtgc agggctttgg aggtgtctct gctcta 36
<210> 17
   <211> 402
   <212> PRT
   <213> Bos taurus
<400> 17
   Met Lys Phe Arg Glu Pro Leu Leu Gly Gly Ser Ala Ala Met Pro Gly
   1               5                   10                  15
   Ala Ser Leu Gln Arg Ala Cys Arg Leu Leu Val Ala Val Cys Ala Leu
               20                  25                  30
   His Leu Gly Val Thr Leu Val Tyr Tyr Leu Ala Gly Arg Asp Leu Arg
           35                  40                  45
   Arg Leu Pro Gln Leu Val Gly Val His Pro Pro Leu Gln Gly Ser Ser
       50                  55                  60
   His Gly Ala Ala Ala Ile Gly Gln Pro Ser Gly Glu Leu Arg Leu Arg
   65                  70                  75                  80
   Gly Val Ala Pro Pro Pro Pro Leu Gln Asn Ser Ser Lys Pro Arg Ser
                   85                  90                  95
   Arg Ala Pro Ser Asn Leu Asp Ala Tyr Ser His Pro Gly Pro Gly Pro
               100                 105                 110
   Gly Pro Gly Ser Asn Leu Thr Ser Ala Pro Val Pro Ser Thr Thr Thr
           115                 120                 125
   Arg Ser Leu Thr Ala Cys Pro Glu Glu Ser Pro Leu Leu Val Gly Pro
       130                 135                 140
   Met Leu Ile Glu Phe Asn Ile Pro Val Asp Leu Lys Leu Ile Glu Gln
   145 150 155 160
   Gln Asn Pro Lys Val Lys Leu Gly Gly Arg Tyr Thr Pro Met Asp Cys
   165 170 175
   Ile Ser Pro His Lys Val Ala Ile Ile Ile Leu Phe Arg Asn Arg Gln
   180 185 190
   Glu His Leu Lys Tyr Trp Leu Tyr Tyr Leu His Pro Met Val Gln Arg
   195 200 205
   Gln Gln Leu Asp Tyr Gly Ile Tyr Val Ile Asn Gln Ala Gly Glu Ser
   210 215 220
   Met Phe Asn Arg Ala Lys Leu Leu Asn Val Gly Phe Lys Glu Ala Leu
   225 230 235 240
   Lys Asp Tyr Asp Tyr Asn Cys Phe Val Phe Ser Asp Val Asp Leu Ile
   245 250 255
   Pro Met Asn Asp His Asn Thr Tyr Arg Cys Phe Ser Gln Pro Arg His
   260 265 270
   Ile Ser Val Ala Met Asp Lys Phe Gly Phe Ser Leu Pro Tyr Val Gln
   275 280 285
   Tyr Phe Gly Gly Val Ser Ala Leu Ser Lys Gln Gln Phe Leu Ser Ile
   290 295 300
   Asn Gly Phe Pro Asn Asn Tyr Trp Gly Trp Gly Gly Glu Asp Asp Asp
   305 310 315 320
   Ile Tyr Asn Arg Leu Ala Phe Arg Gly Met Ser Val Ser Arg Pro Asn
   325 330 335
   Ala Val Ile Gly Lys Cys Arg Met Ile Arg His Ser Arg Asp Lys Lys
   340 345 350
   Asn Glu Pro Asn Pro Gln Arg Phe Asp Arg Ile Ala His Thr Lys Glu
   355 360 365
   Thr Met Leu Ser Asp Gly Leu Asn Ser Leu Thr Tyr Met Val Leu Glu
   370 375 380
   Val Gln Arg Tyr Pro Leu Tyr Thr Lys Ile Thr Val Asp Ile Gly Thr
   385 390 395 400
   Pro Ser
<210> 18
   <211> 402
   <212> PRT
   <213> artificial
<220>
   <223> bos taurus GalT Y289N
<400> 18
   Met Lys Phe Arg Glu Pro Leu Leu Gly Gly Ser Ala Ala Met Pro Gly
   1 5 10 15
   Ala Ser Leu Gln Arg Ala Cys Arg Leu Leu Val Ala Val Cys Ala Leu
   20 25 30
   His Leu Gly Val Thr Leu Val Tyr Tyr Leu Ala Gly Arg Asp Leu Arg
   35 40 45
   Arg Leu Pro Gln Leu Val Gly Val His Pro Pro Leu Gln Gly Ser Ser
   50 55 60
   His Gly Ala Ala Ala Ile Gly Gln Pro Ser Gly Glu Leu Arg Leu Arg
   65 70 75 80
   Gly Val Ala Pro Pro Pro Pro Leu Gln Asn Ser Ser Lys Pro Arg Ser
   85 90 95
   Arg Ala Pro Ser Asn Leu Asp Ala Tyr Ser His Pro Gly Pro Gly Pro
   100 105 110
   Gly Pro Gly Ser Asn Leu Thr Ser Ala Pro Val Pro Ser Thr Thr Thr
   115 120 125
   Arg Ser Leu Thr Ala Cys Pro Glu Glu Ser Pro Leu Leu Val Gly Pro
   130 135 140
   Met Leu Ile Glu Phe Asn Ile Pro Val Asp Leu Lys Leu Ile Glu Gln
   145 150 155 160
   Gln Asn Pro Lys Val Lys Leu Gly Gly Arg Tyr Thr Pro Met Asp Cys
   165 170 175
   Ile Ser Pro His Lys Val Ala Ile Ile Ile Leu Phe Arg Asn Arg Gln
   180 185 190
   Glu His Leu Lys Tyr Trp Leu Tyr Tyr Leu His Pro Met Val Gln Arg
   195 200 205
   Gln Gln Leu Asp Tyr Gly Ile Tyr Val Ile Asn Gln Ala Gly Glu Ser
   210 215 220
   Met Phe Asn Arg Ala Lys Leu Leu Asn Val Gly Phe Lys Glu Ala Leu
   225 230 235 240
   Lys Asp Tyr Asp Tyr Asn Cys Phe Val Phe Ser Asp Val Asp Leu Ile
   245 250 255
   Pro Met Asn Asp His Asn Thr Tyr Arg Cys Phe Ser Gln Pro Arg His
   260 265 270
   Ile Ser Val Ala Met Asp Lys Phe Gly Phe Ser Leu Pro Tyr Val Gln
   275 280 285
   Asn Phe Gly Gly Val Ser Ala Leu Ser Lys Gln Gln Phe Leu Ser Ile
   290 295 300
   Asn Gly Phe Pro Asn Asn Tyr Trp Gly Trp Gly Gly Glu Asp Asp Asp
   305 310 315 320
   Ile Tyr Asn Arg Leu Ala Phe Arg Gly Met Ser Val Ser Arg Pro Asn
   325 330 335
   Ala Val Ile Gly Lys Cys Arg Met Ile Arg His Ser Arg Asp Lys Lys
   340 345 350
   Asn Glu Pro Asn Pro Gln Arg Phe Asp Arg Ile Ala His Thr Lys Glu
   355 360 365
   Thr Met Leu Ser Asp Gly Leu Asn Ser Leu Thr Tyr Met Val Leu Glu
   370 375 380
   Val Gln Arg Tyr Pro Leu Tyr Thr Lys Ile Thr Val Asp Ile Gly Thr
   385 390 395 400
   Pro Ser
<210> 19
   <211> 402
   <212> PRT
   <213> artificial
<220>
   <223> bos taurus GalT Y289F
<400> 19
   Met Lys Phe Arg Glu Pro Leu Leu Gly Gly Ser Ala Ala Met Pro Gly
   1               5                   10                  15
   Ala Ser Leu Gln Arg Ala Cys Arg Leu Leu Val Ala Val Cys Ala Leu
               20                  25                  30
   His Leu Gly Val Thr Leu Val Tyr Tyr Leu Ala Gly Arg Asp Leu Arg
           35                  40                  45
   Arg Leu Pro Gln Leu Val Gly Val His Pro Pro Leu Gln Gly Ser Ser
       50                  55                  60
   His Gly Ala Ala Ala Ile Gly Gln Pro Ser Gly Glu Leu Arg Leu Arg
   65                  70                  75                  80
   Gly Val Ala Pro Pro Pro Pro Leu Gln Asn Ser Ser Lys Pro Arg Ser
                   85                  90                  95
   Arg Ala Pro Ser Asn Leu Asp Ala Tyr Ser His Pro Gly Pro Gly Pro
               100                 105                 110
   Gly Pro Gly Ser Asn Leu Thr Ser Ala Pro Val Pro Ser Thr Thr Thr
           115                 120                 125
   Arg Ser Leu Thr Ala Cys Pro Glu Glu Ser Pro Leu Leu Val Gly Pro
       130                 135                 140
   Met Leu Ile Glu Phe Asn Ile Pro Val Asp Leu Lys Leu Ile Glu Gln
   145                 150                 155                 160
   Gln Asn Pro Lys Val Lys Leu Gly Gly Arg Tyr Thr Pro Met Asp Cys
                   165                 170                 175
   Ile Ser Pro His Lys Val Ala Ile Ile Ile Leu Phe Arg Asn Arg Gln
               180                 185                 190
   Glu His Leu Lys Tyr Trp Leu Tyr Tyr Leu His Pro Met Val Gln Arg
           195                 200                 205
   Gln Gln Leu Asp Tyr Gly Ile Tyr Val Ile Asn Gln Ala Gly Glu Ser
       210                 215                 220
   Met Phe Asn Arg Ala Lys Leu Leu Asn Val Gly Phe Lys Glu Ala Leu
   225                 230                 235                 240
   Lys Asp Tyr Asp Tyr Asn Cys Phe Val Phe Ser Asp Val Asp Leu Ile
   245 250 255
   Pro Met Asn Asp His Asn Thr Tyr Arg Cys Phe Ser Gln Pro Arg His
   260 265 270
   Ile Ser Val Ala Met Asp Lys Phe Gly Phe Ser Leu Pro Tyr Val Gln
   275 280 285
   Phe Phe Gly Gly Val Ser Ala Leu Ser Lys Gln Gln Phe Leu Ser Ile
   290 295 300
   Asn Gly Phe Pro Asn Asn Tyr Trp Gly Trp Gly Gly Glu Asp Asp Asp
   305 310 315 320
   Ile Tyr Asn Arg Leu Ala Phe Arg Gly Met Ser Val Ser Arg Pro Asn
   325 330 335
   Ala Val Ile Gly Lys Cys Arg Met Ile Arg His Ser Arg Asp Lys Lys
   340 345 350
   Asn Glu Pro Asn Pro Gln Arg Phe Asp Arg Ile Ala His Thr Lys Glu
   355 360 365
   Thr Met Leu Ser Asp Gly Leu Asn Ser Leu Thr Tyr Met Val Leu Glu
   370 375 380
   Val Gln Arg Tyr Pro Leu Tyr Thr Lys Ile Thr Val Asp Ile Gly Thr
   385 390 395 400
   Pro Ser
<210> 20
   <211> 402
   <212> PRT
   <213> artificial
<220>
   <223> bos taurus GalT Y289M
<400> 20
   Met Lys Phe Arg Glu Pro Leu Leu Gly Gly Ser Ala Ala Met Pro Gly
   1 5 10 15
   Ala Ser Leu Gln Arg Ala Cys Arg Leu Leu Val Ala Val Cys Ala Leu
   20 25 30
   His Leu Gly Val Thr Leu Val Tyr Tyr Leu Ala Gly Arg Asp Leu Arg
           35                  40                  45
   Arg Leu Pro Gln Leu Val Gly Val His Pro Pro Leu Gln Gly Ser Ser
       50                  55                  60
   His Gly Ala Ala Ala Ile Gly Gln Pro Ser Gly Glu Leu Arg Leu Arg
   65                  70                  75                  80
   Gly Val Ala Pro Pro Pro Pro Leu Gln Asn Ser Ser Lys Pro Arg Ser
                   85                  90                  95
   Arg Ala Pro Ser Asn Leu Asp Ala Tyr Ser His Pro Gly Pro Gly Pro
               100                 105                 110
   Gly Pro Gly Ser Asn Leu Thr Ser Ala Pro Val Pro Ser Thr Thr Thr
           115                 120                 125
   Arg Ser Leu Thr Ala Cys Pro Glu Glu Ser Pro Leu Leu Val Gly Pro
       130                 135                 140
   Met Leu Ile Glu Phe Asn Ile Pro Val Asp Leu Lys Leu Ile Glu Gln
   145                 150                 155                 160
   Gln Asn Pro Lys Val Lys Leu Gly Gly Arg Tyr Thr Pro Met Asp Cys
                   165                 170                 175
   Ile Ser Pro His Lys Val Ala Ile Ile Ile Leu Phe Arg Asn Arg Gln
               180                 185                 190
   Glu His Leu Lys Tyr Trp Leu Tyr Tyr Leu His Pro Met Val Gln Arg
           195                 200                 205
   Gln Gln Leu Asp Tyr Gly Ile Tyr Val Ile Asn Gln Ala Gly Glu Ser
       210                 215                 220
   Met Phe Asn Arg Ala Lys Leu Leu Asn Val Gly Phe Lys Glu Ala Leu
   225                 230                 235                 240
   Lys Asp Tyr Asp Tyr Asn Cys Phe Val Phe Ser Asp Val Asp Leu Ile
                   245                 250                 255
   Pro Met Asn Asp His Asn Thr Tyr Arg Cys Phe Ser Gln Pro Arg His
               260                 265                 270
   Ile Ser Val Ala Met Asp Lys Phe Gly Phe Ser Leu Pro Tyr Val Gln
           275                 280                 285
   Met Phe Gly Gly Val Ser Ala Leu Ser Lys Gln Gln Phe Leu Ser Ile
       290                 295                 300
   Asn Gly Phe Pro Asn Asn Tyr Trp Gly Trp Gly Gly Glu Asp Asp Asp
   305                 310                 315                 320
   Ile Tyr Asn Arg Leu Ala Phe Arg Gly Met Ser Val Ser Arg Pro Asn
                   325                 330                 335
   Ala Val Ile Gly Lys Cys Arg Met Ile Arg His Ser Arg Asp Lys Lys
               340                 345                 350
   Asn Glu Pro Asn Pro Gln Arg Phe Asp Arg Ile Ala His Thr Lys Glu
           355                 360                 365
   Thr Met Leu Ser Asp Gly Leu Asn Ser Leu Thr Tyr Met Val Leu Glu
       370                 375                 380
   Val Gln Arg Tyr Pro Leu Tyr Thr Lys Ile Thr Val Asp Ile Gly Thr
   385                 390                 395                 400
   Pro Ser
<210> 21
   <211> 402
   <212> PRT
   <213> artificial
<220>
   <223> bos taurus GalT Y289I
<400> 21
   Met Lys Phe Arg Glu Pro Leu Leu Gly Gly Ser Ala Ala Met Pro Gly
   1               5                   10                  15
   Ala Ser Leu Gln Arg Ala Cys Arg Leu Leu Val Ala Val Cys Ala Leu
               20                  25                  30
   His Leu Gly Val Thr Leu Val Tyr Tyr Leu Ala Gly Arg Asp Leu Arg
           35                  40                  45
   Arg Leu Pro Gln Leu Val Gly Val His Pro Pro Leu Gln Gly Ser Ser
       50                  55                  60
   His Gly Ala Ala Ala Ile Gly Gln Pro Ser Gly Glu Leu Arg Leu Arg
   65                  70                  75                  80
   Gly Val Ala Pro Pro Pro Pro Leu Gln Asn Ser Ser Lys Pro Arg Ser
                   85                  90                  95
   Arg Ala Pro Ser Asn Leu Asp Ala Tyr Ser His Pro Gly Pro Gly Pro
               100                 105                 110
   Gly Pro Gly Ser Asn Leu Thr Ser Ala Pro Val Pro Ser Thr Thr Thr
           115                 120                 125
   Arg Ser Leu Thr Ala Cys Pro Glu Glu Ser Pro Leu Leu Val Gly Pro
       130                 135                 140
   Met Leu Ile Glu Phe Asn Ile Pro Val Asp Leu Lys Leu Ile Glu Gln
   145                 150                 155                 160
   Gln Asn Pro Lys Val Lys Leu Gly Gly Arg Tyr Thr Pro Met Asp Cys
                   165                 170                 175
   Ile Ser Pro His Lys Val Ala Ile Ile Ile Leu Phe Arg Asn Arg Gln
               180                 185                 190
   Glu His Leu Lys Tyr Trp Leu Tyr Tyr Leu His Pro Met Val Gln Arg
           195                 200                 205
   Gln Gln Leu Asp Tyr Gly Ile Tyr Val Ile Asn Gln Ala Gly Glu Ser
       210                 215                 220
   Met Phe Asn Arg Ala Lys Leu Leu Asn Val Gly Phe Lys Glu Ala Leu
   225                 230                 235                 240
   Lys Asp Tyr Asp Tyr Asn Cys Phe Val Phe Ser Asp Val Asp Leu Ile
                   245                 250                 255
   Pro Met Asn Asp His Asn Thr Tyr Arg Cys Phe Ser Gln Pro Arg His
               260                 265                 270
   Ile Ser Val Ala Met Asp Lys Phe Gly Phe Ser Leu Pro Tyr Val Gln
           275                 280                 285
   Ile Phe Gly Gly Val Ser Ala Leu Ser Lys Gln Gln Phe Leu Ser Ile
       290                 295                 300
   Asn Gly Phe Pro Asn Asn Tyr Trp Gly Trp Gly Gly Glu Asp Asp Asp
   305                 310                 315                 320
   Ile Tyr Asn Arg Leu Ala Phe Arg Gly Met Ser Val Ser Arg Pro Asn
                   325                 330                 335
   Ala Val Ile Gly Lys Cys Arg Met Ile Arg His Ser Arg Asp Lys Lys
   340 345 350
   Asn Glu Pro Asn Pro Gln Arg Phe Asp Arg Ile Ala His Thr Lys Glu
   355 360 365
   Thr Met Leu Ser Asp Gly Leu Asn Ser Leu Thr Tyr Met Val Leu Glu
   370 375 380
   Val Gln Arg Tyr Pro Leu Tyr Thr Lys Ile Thr Val Asp Ile Gly Thr
   385 390 395 400
   Pro Ser
<210> 22
   <211> 402
   <212> PRT
   <213> artificial
<220>
   <223> bos taurus GalT Y289V
<400> 22
   Met Lys Phe Arg Glu Pro Leu Leu Gly Gly Ser Ala Ala Met Pro Gly
   1 5 10 15
   Ala Ser Leu Gln Arg Ala Cys Arg Leu Leu Val Ala Val Cys Ala Leu
   20 25 30
   His Leu Gly Val Thr Leu Val Tyr Tyr Leu Ala Gly Arg Asp Leu Arg
   35 40 45
   Arg Leu Pro Gln Leu Val Gly Val His Pro Pro Leu Gln Gly Ser Ser
   50 55 60
   His Gly Ala Ala Ala Ile Gly Gln Pro Ser Gly Glu Leu Arg Leu Arg
   65 70 75 80
   Gly Val Ala Pro Pro Pro Pro Leu Gln Asn Ser Ser Lys Pro Arg Ser
   85 90 95
   Arg Ala Pro Ser Asn Leu Asp Ala Tyr Ser His Pro Gly Pro Gly Pro
   100 105 110
   Gly Pro Gly Ser Asn Leu Thr Ser Ala Pro Val Pro Ser Thr Thr Thr
   115 120 125
   Arg Ser Leu Thr Ala Cys Pro Glu Glu Ser Pro Leu Leu Val Gly Pro
       130                 135                 140
   Met Leu Ile Glu Phe Asn Ile Pro Val Asp Leu Lys Leu Ile Glu Gln
   145                 150                 155                 160
   Gln Asn Pro Lys Val Lys Leu Gly Gly Arg Tyr Thr Pro Met Asp Cys
                   165                 170                 175
   Ile Ser Pro His Lys Val Ala Ile Ile Ile Leu Phe Arg Asn Arg Gln
               180                 185                 190
   Glu His Leu Lys Tyr Trp Leu Tyr Tyr Leu His Pro Met Val Gln Arg
           195                 200                 205
   Gln Gln Leu Asp Tyr Gly Ile Tyr Val Ile Asn Gln Ala Gly Glu Ser
       210                 215                 220
   Met Phe Asn Arg Ala Lys Leu Leu Asn Val Gly Phe Lys Glu Ala Leu
   225                 230                 235                 240
   Lys Asp Tyr Asp Tyr Asn Cys Phe Val Phe Ser Asp Val Asp Leu Ile
                   245                 250                 255
   Pro Met Asn Asp His Asn Thr Tyr Arg Cys Phe Ser Gln Pro Arg His
               260                 265                 270
   Ile Ser Val Ala Met Asp Lys Phe Gly Phe Ser Leu Pro Tyr Val Gln
           275                 280                 285
   Val Phe Gly Gly Val Ser Ala Leu Ser Lys Gln Gln Phe Leu Ser Ile
       290                 295                 300
   Asn Gly Phe Pro Asn Asn Tyr Trp Gly Trp Gly Gly Glu Asp Asp Asp
   305                 310                 315                 320
   Ile Tyr Asn Arg Leu Ala Phe Arg Gly Met Ser Val Ser Arg Pro Asn
                   325                 330                 335
   Ala Val Ile Gly Lys Cys Arg Met Ile Arg His Ser Arg Asp Lys Lys
               340                 345                 350
   Asn Glu Pro Asn Pro Gln Arg Phe Asp Arg Ile Ala His Thr Lys Glu
           355                 360                 365
   Thr Met Leu Ser Asp Gly Leu Asn Ser Leu Thr Tyr Met Val Leu Glu
       370                 375                 380
   Val Gln Arg Tyr Pro Leu Tyr Thr Lys Ile Thr Val Asp Ile Gly Thr
   385 390 395 400
   Pro Ser
<210> 23
   <211> 402
   <212> PRT
   <213> artificial
<220>
   <223> bos taurus GalT Y289A
<400> 23
   Met Lys Phe Arg Glu Pro Leu Leu Gly Gly Ser Ala Ala Met Pro Gly
   1 5 10 15
   Ala Ser Leu Gln Arg Ala Cys Arg Leu Leu Val Ala Val Cys Ala Leu
   20 25 30
   His Leu Gly Val Thr Leu Val Tyr Tyr Leu Ala Gly Arg Asp Leu Arg
   35 40 45
   Arg Leu Pro Gln Leu Val Gly Val His Pro Pro Leu Gln Gly Ser Ser
   50 55 60
   His Gly Ala Ala Ala Ile Gly Gln Pro Ser Gly Glu Leu Arg Leu Arg
   65 70 75 80
   Gly Val Ala Pro Pro Pro Pro Leu Gln Asn Ser Ser Lys Pro Arg Ser
   85 90 95
   Arg Ala Pro Ser Asn Leu Asp Ala Tyr Ser His Pro Gly Pro Gly Pro
   100 105 110
   Gly Pro Gly Ser Asn Leu Thr Ser Ala Pro Val Pro Ser Thr Thr Thr
   115 120 125
   Arg Ser Leu Thr Ala Cys Pro Glu Glu Ser Pro Leu Leu Val Gly Pro
   130 135 140
   Met Leu Ile Glu Phe Asn Ile Pro Val Asp Leu Lys Leu Ile Glu Gln
   145 150 155 160
   Gln Asn Pro Lys Val Lys Leu Gly Gly Arg Tyr Thr Pro Met Asp Cys
   165 170 175
   Ile Ser Pro His Lys Val Ala Ile Ile Ile Leu Phe Arg Asn Arg Gln
               180                 185                 190
   Glu His Leu Lys Tyr Trp Leu Tyr Tyr Leu His Pro Met Val Gln Arg
           195                 200                 205
   Gln Gln Leu Asp Tyr Gly Ile Tyr Val Ile Asn Gln Ala Gly Glu Ser
       210                 215                 220
   Met Phe Asn Arg Ala Lys Leu Leu Asn Val Gly Phe Lys Glu Ala Leu
   225                 230                 235                 240
   Lys Asp Tyr Asp Tyr Asn Cys Phe Val Phe Ser Asp Val Asp Leu Ile
                   245                 250                 255
   Pro Met Asn Asp His Asn Thr Tyr Arg Cys Phe Ser Gln Pro Arg His
               260                 265                 270
   Ile Ser Val Ala Met Asp Lys Phe Gly Phe Ser Leu Pro Tyr Val Gln
           275                 280                 285
   Ala Phe Gly Gly Val Ser Ala Leu Ser Lys Gln Gln Phe Leu Ser Ile
       290                 295                 300
   Asn Gly Phe Pro Asn Asn Tyr Trp Gly Trp Gly Gly Glu Asp Asp Asp
   305                 310                 315                 320
   Ile Tyr Asn Arg Leu Ala Phe Arg Gly Met Ser Val Ser Arg Pro Asn
                   325                 330                 335
   Ala Val Ile Gly Lys Cys Arg Met Ile Arg His Ser Arg Asp Lys Lys
               340                 345                 350
   Asn Glu Pro Asn Pro Gln Arg Phe Asp Arg Ile Ala His Thr Lys Glu
           355                 360                 365
   Thr Met Leu Ser Asp Gly Leu Asn Ser Leu Thr Tyr Met Val Leu Glu
       370                 375                 380
   Val Gln Arg Tyr Pro Leu Tyr Thr Lys Ile Thr Val Asp Ile Gly Thr
   385                 390                 395                 400
   Pro Ser
<210> 24
   <211> 402
   <212> PRT
   <213> artificial
<220>
   <223> bos taurus GalT Y289G
<400> 24
   Met Lys Phe Arg Glu Pro Leu Leu Gly Gly Ser Ala Ala Met Pro Gly
   1               5                   10                  15
   Ala Ser Leu Gln Arg Ala Cys Arg Leu Leu Val Ala Val Cys Ala Leu
               20                  25                  30
   His Leu Gly Val Thr Leu Val Tyr Tyr Leu Ala Gly Arg Asp Leu Arg
           35                  40                  45
   Arg Leu Pro Gln Leu Val Gly Val His Pro Pro Leu Gln Gly Ser Ser
       50                  55                  60
   His Gly Ala Ala Ala Ile Gly Gln Pro Ser Gly Glu Leu Arg Leu Arg
   65                  70                  75                  80
   Gly Val Ala Pro Pro Pro Pro Leu Gln Asn Ser Ser Lys Pro Arg Ser
                   85                  90                  95
   Arg Ala Pro Ser Asn Leu Asp Ala Tyr Ser His Pro Gly Pro Gly Pro
               100                 105                 110
   Gly Pro Gly Ser Asn Leu Thr Ser Ala Pro Val Pro Ser Thr Thr Thr
           115                 120                 125
   Arg Ser Leu Thr Ala Cys Pro Glu Glu Ser Pro Leu Leu Val Gly Pro
       130                 135                 140
   Met Leu Ile Glu Phe Asn Ile Pro Val Asp Leu Lys Leu Ile Glu Gln
   145                 150                 155                 160
   Gln Asn Pro Lys Val Lys Leu Gly Gly Arg Tyr Thr Pro Met Asp Cys
                   165                 170                 175
   Ile Ser Pro His Lys Val Ala Ile Ile Ile Leu Phe Arg Asn Arg Gln
               180                 185                 190
   Glu His Leu Lys Tyr Trp Leu Tyr Tyr Leu His Pro Met Val Gln Arg
           195                 200                 205
   Gln Gln Leu Asp Tyr Gly Ile Tyr Val Ile Asn Gln Ala Gly Glu Ser
       210                 215                 220
   Met Phe Asn Arg Ala Lys Leu Leu Asn Val Gly Phe Lys Glu Ala Leu
   225                 230                 235                 240
   Lys Asp Tyr Asp Tyr Asn Cys Phe Val Phe Ser Asp Val Asp Leu Ile
                   245                 250                 255
   Pro Met Asn Asp His Asn Thr Tyr Arg Cys Phe Ser Gln Pro Arg His
               260                 265                 270
   Ile Ser Val Ala Met Asp Lys Phe Gly Phe Ser Leu Pro Tyr Val Gln
           275                 280                 285
   Gly Phe Gly Gly Val Ser Ala Leu Ser Lys Gln Gln Phe Leu Ser Ile
       290                 295                 300
   Asn Gly Phe Pro Asn Asn Tyr Trp Gly Trp Gly Gly Glu Asp Asp Asp
   305                 310                 315                 320
   Ile Tyr Asn Arg Leu Ala Phe Arg Gly Met Ser Val Ser Arg Pro Asn
                   325                 330                 335
   Ala Val Ile Gly Lys Cys Arg Met Ile Arg His Ser Arg Asp Lys Lys
               340                 345                 350
   Asn Glu Pro Asn Pro Gln Arg Phe Asp Arg Ile Ala His Thr Lys Glu
           355                 360                 365
   Thr Met Leu Ser Asp Gly Leu Asn Ser Leu Thr Tyr Met Val Leu Glu
       370                 375                 380
   Val Gln Arg Tyr Pro Leu Tyr Thr Lys Ile Thr Val Asp Ile Gly Thr
   385                 390                 395                 400
   Pro Ser

## Description

### Technical field of the invention

The present invention relates to antibodies, modified antibodies and antibody-conjugates, in particular to glycoengineered antibodies, modified antibodies and antibody-conjugates. The invention also relates to a method for preparation of the modified antibodies and antibody-conjugates of the invention. The antibodies may be conjugated to an active substance. The invention therefore also relates to antibody-drug conjugates (ADCs) and a method for the preparation thereof.

### Background of the invention

Antibody-conjugates, *i.e.* antibodies conjugated to a molecule of interest via a linker, are known in the art. There is great interest in antibody-conjugates wherein the molecule of interest is a drug, for example a cytotoxic chemical. Antibody-drug-conjugates are known in the art, and consist of a recombinant antibody covalently bound to a cytotoxic chemical via a synthetic linker (S.C. Alley et al, Curr. Opin. Chem. Biol. 2010, 14, 529-537). The main objective of an antibody-drug-conjugate (ADC), also called immunotoxin, is to combine the high specificity of a monoclonal antibody for a tumor-associated antigen with the pharmacological potency of a "small" cytotoxic drug (typically 300 to 1,000 Da). Examples of ADCs include gemtuzumab ozogamicin (Mylotarg; anti-CD33 mAb conjugated to calicheamycin, Pfizer/Wyeth); brentuximab vedotin (SGN-35, Adcetris, a CD30-targeting ADC consisting of brentuximab, covalently linked to MMAE (monomethylauristatin), Seattle Genetics); trastuzumab-DM1 conjugate (T-DM1, Kadcyla).

One advance in the field includes the emergence of extremely potent toxins, in particular taxanes, calicheamycins, maytansins, pyrrolobenzodiazepines, duocarmycins and auristatins. The low nanomolar to picomolar toxicity of these substances is a principal driver improvement over the earlier applied toxins. Another important technological advance involves the use of optimized linkers that are hydrolysable in the cytoplasm, resistant or susceptible to proteases, or resistant to multi-drug resistance efflux pumps that are associated with highly cytotoxic drugs.

ADCs known from the prior art are commonly prepared by conjugation of the linker-toxin to the side chain of amino acid lysine or cysteine, by acylation or alkylation, respectively.

For lysines, conjugation takes place preferentially at lysine side chains with highest steric accessibility, the lowest pKa, or a combination thereof. Disadvantage of this method is that site-control of conjugation is low.

Better control of site-specificity is obtained by alkylation of cysteines, based on the fact that typically no free cysteines are present in an antibody, thereby offering the option of alkylating only those cysteines that are selectively liberated by a reductive step or specifically engineered into the antibody as free cysteines (as in so-called THIOmabs). Selective cysteine liberation by reduction is typically performed by treatment of whole antibody with a reducing agent (*e.g.* TCEP or DTT), leading to conversion of a disulfide bond into two free thiols (mostly in the antibody's hinge region). The liberated thiols are then alkylated with an electrophilic reagent, typically based on a maleimide attached to a linker-toxin, which generally proceeds fast and with high selectivity. With respect to engineering of an additional (free) cysteine into an antibody, enhanced site-control is attained with respect to the location of the added cysteine(s). Also in this strategy alkylation of free cysteines is effected with maleimide chemistry, but full homogeneity is not attained. One most recent report (Okeley et al., Bioconj. Chem. 2013, 24, 1650 - 1655) describes the metabolic incorporation of 6-thiofucose into a monoclonal antibody. However, efficiency of incorporation of 6-thiofucose was found to be only 70%, thus a DAR of 1.3 is attained after maleimide conjugation.

At the same time, a disadvantage of ADCs obtained via alkylation with maleimides is that in general the resulting conjugates may be unstable due to the reverse of alkylation, *i.e.* a retro-Michael reaction, thereby leading to release of linker-toxin from the antibody. It has been described that the stability of the cysteine-maleimide conjugate is highly dependent on the position of the cysteine in the monoclonal antibody. For example, a highly solvent-accessible site typically rapidly loses conjugation in plasma owing to maleimide exchange with reactive thiols in albumin, glutathione or other proteins. In contrast, this undesired exchange reaction is prevented by hydrolysis of the succinimide ring -the result of thiol addition to maleimide -in a partially accessible site with a positively charged environment, while the site with partial solvent-accessibility and neutral charge displayed both properties. Similarly, it was found that the 6-thiofucose maleimide conjugate described above was found to display somewhat enhanced stability with respect to cysteine maleimide conjugates, but an explanation was not provided. In view of the above, conjugation based on cysteine-maleimide alkylation is not an ideal technology for development of ADCs that preferably should not show premature release of toxin.

An alternative strategy to prepare antibody-drug conjugates involves the generation of one or more aldehyde functions on the antibody's glycan structure. All recombinant antibodies, generated in mammalian host systems, contain the conserved N-glycosylation site at asparagine-297, which is modified by a glycan of the complex type. The latter glycan features 0, 1 or 2 terminal galactose units at the non-reducing termini of each N-glycan, which can be applied for the generation of an aldehyde function, either by chemical means (sodium periodate) or by enzymatic means (galactose oxidase). The latter aldehyde function can subsequently be employed for a selective conjugation process, for example by condensation with a functionalized hydroxylamine or hydrazine molecule, thereby generating an oxime-linked or hydrazone-linked antibody conjugate, respectively. However, it is known that oximes and hydrazones, in particular derived from aliphatic aldehydes, show limited stability over time in water or at lower pH. For example, gemtuzumab ozogamicin is an oxime-linked antibody-drug conjugate and is known to suffer from premature deconjugation in vivo.

Antibody-conjugates known in the art generally suffer from several disadvantages. For antibody drug-conjugates, a measure for the loading of the antibody with a toxin is given by the drug-antibody ratio (DAR), which gives the average number of active substance molecules per antibody. However, the DAR does not give any indication regarding the homogeneity of such ADC.

Processes for the preparation of an antibody-conjugate known from the prior art generally result in a product with a DAR between 1.5 and 4, but in fact such a product comprises a mixture of antibody-conjugates with a number of molecules of interest varying from 0 to 8 or higher. In other words, antibody-conjugates known from the prior art generally are formed with a DAR with high standard deviation.

For example, gemtuzumab ozogamicin is a heterogeneous mixture of 50% conjugates (0 to 8 calicheamycin moieties per IgG molecules with an average of 2 or 3, randomly linked to solvent exposed lysine residues of the antibody) and 50% unconjugated antibody (Bross et al., Clin. Cancer Res. 2001, 7, 1490; Labrijn et al., Nat. Biotechnol. 2009 27, 767). But also for brentuximab vedotin, T-DM1 (Kadcyla), brentuximab vedotin (Adcetris), and other ADCs in clinical development, it is still uncontrollable exactly how many drugs are attaching to any given antibody (drug-antibody ratio, DAR) and the ADC is obtained as a statistical distribution of conjugates. Whether the optimal number of drugs per antibody is for example two, four or more, attaching them in a predictable number and in predictable locations through site-specific conjugation with a narrow standard deviation is still problematic.

A versatile strategy that may be generally applicable to all monoclonal antibodies involves the site-specific conjugation to the Fc-attached glycan, which is naturally present in all antibodies expressed in mammalian (or yeast) cell cultures. Several strategies based on this concept are known in the art, such as via oxidation of the terminal galactose or via enzymatic transfer of (unnatural) sialic acid to the same galactose moiety. However, for ADC purpose such a strategy is suboptimal because glycans are always formed as a complex mixture of isoforms, which may contain different levels of galactosylation (G0, G1, G2) and therefore would afford ADCs with poor control of drug-antibody ratio (DAR, see below).

Qasba et al. disclose in WO 2004/063344 and in J. Biol. Chem. 2002, 277, 20833, that mutant galactosyltransferases GalT(Y289L), GalT(Y289I) and GalT(Y289N) can enzymatically attach GalNAc to a non-reducing GlcNAc sugar. For example, GlcNAc is the terminal component of some of the complex N-glycans such as those on monoclonal antibodies (e.g. Rituxan, Remicade, Herceptin).

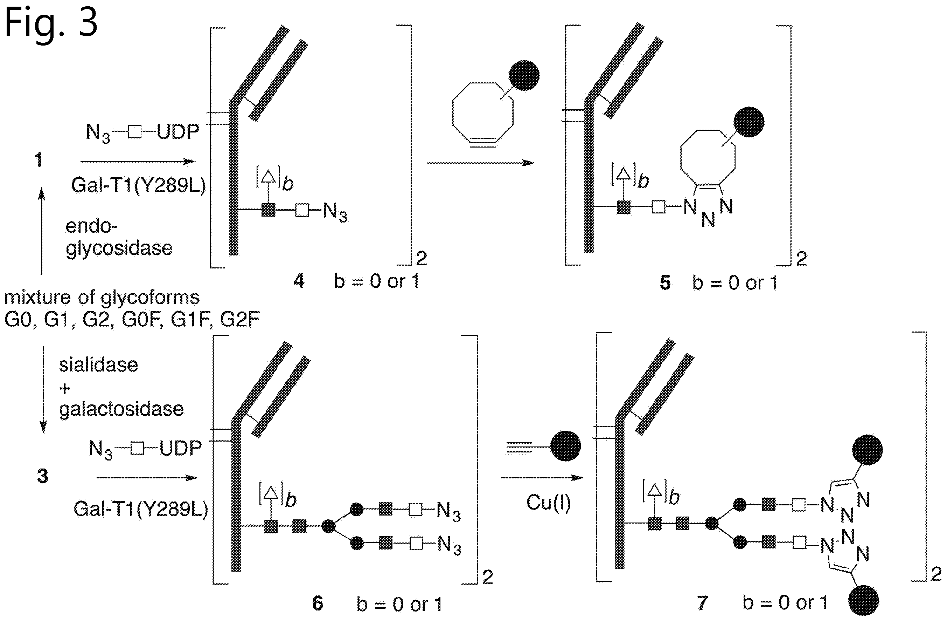

Qasba et al. disclose in Bioconjugate Chem. 2009, 20, 1228, that the process disclosed in WO 2004/063344 also proceeds for non-natural GalNAc-UDP variants substituted on the N-acetyl group. β-Galactosidase treated monoclonal antibodies having a G0 glycoform are fully galactosylated to the G2 glycoform after transfer of a galactose moiety comprising a C2-substituted azidoacetamido moiety (GalNAz) to the terminal GlcNAc residues of the glycan, leading to tetraazido-substituted antibodies, *i.e.* two GalNAz moieties per heavy chain (see Figure 3, conversion of **3** to **6**). The conjugation of said tetraazido-substituted antibodies to a molecule of interest, for example by Staudinger ligation or click reaction with an alkyne (see Figure 3, conversion of **6** to 7) is of potential interest to prepare antibody-drug conjugates with a DAR of 4, for example, but was not disclosed. The transfer of a galactose moiety comprising a C2-substituted keto group (C2-keto-Gal) to the terminal GlcNAc residues of a G0 glycoform glycan, as well as the linking of C2-keto-Gal to aminooxy biotin, is also disclosed. However, as mentioned above, the resulting oxime conjugates may display limited stability due to aqueous hydrolysis.

WO 2007/095506 and WO 2008/029281 (Invitrogen Corporation), disclose that the combination of GalT(Y289L) mutant with C2-substituted azidoacetamido-galactose UDP-derivative (UDP-GalNAz) leads to the incorporation of GalNAz at a terminal non-reducing GlcNAc of a glycan. Subsequent conjugation by Staudinger ligation or with copper-catalyzed click chemistry then provides the respective antibody conjugates wherein a fluorescent alkyne probe is conjugated to an antibody. WO 2007/095506 and WO 2008/029281 further disclose that trimming of the glycan can take place with endo H, thereby hydrolyzing a GlcNAc-GlcNAc glycosidic bond and liberating a GlcNAc for enzymatic introduction of GalNAz.

A disadvantage of the method disclosed in WO 2004/063344 and Bioconjugate Chem. 2009, 20, 1228 is that conjugation of the tetraazido-substituted antibodies to a molecule of interest would lead to an antibody-conjugate with typically two molecules of interest per glycan (provided that said conjugation would proceed with complete conversion). In some cases, for example when the molecule of interest is a lipophilic toxin, the presence of too many molecules of interest per antibody is undesired since this may lead to aggregate formation (BioProcess International 2006, 4, 42-43), in particular when the lipophilic moieties are in proximity.

In WO 2007/133855 (University of Maryland Biotechnology Institute), a chemoenzymatic method for the preparation of a homogeneous glycoprotein or glycopeptide is disclosed, involving a two-stage strategy entailing first trimming of the near-complete glycan tree (under the action of endo A, endo H or endo S) leaving only the core N-acetylglucosamine (GlcNAc) moiety (the so-called GlcNAc-protein), followed by a reglycosylation event wherein, in the presence of a catalyst comprising endoglycosidase (ENGase), an oligosaccharide moiety is transferred to the GlcNAc-protein to yield a homogeneous glycoprotein or glycopeptide. A strategy for azide-functionalized glycoproteins is disclosed, wherein a GlcNAc-protein is reacted in the presence of ENGase with a tetrasaccharide oxazoline containing two 6-azidomannose moieties, thereby introducing two azides simultaneously in the glycan. The azide-functionalized glycoprotein may then be catalytically reacted in a "click chemistry" cycloaddition reaction, in the presence of a catalyst (e.g. a Cu(I) catalyst) with a terminal alkyne bearing a functional moiety X of interest. No actual examples of said click chemistry are disclosed.

In J. Am. Chem. Soc. 2012, 134, 8030, Davis *et al.* disclose the transfer of oligosaccharide oxazolines on a core-fucosylated as well as nonfucosylated core-GlcNAc-Fc domain of intact antibodies, in the presence of glycosynthase EndoS.

In J. Am. Chem. Soc. 2012, 134, 12308, Wang *et al.* disclose the transfer of a tetrasaccharide oxazoline containing two 6-azidomannose moieties on core-fucosylated as well as nonfucosylated core-GlcNAc-Fc domain of intact antibodies (Rituximab) in the presence of glycosynthase mutants EndoS-D233A and EndoS-D233Q.

However, a disadvantage of the glycosynthase strategies disclosed in WO 2007/133855, J. Am. Chem. Soc. 2012, 134, 8030 and J. Am. Chem. Soc. 2012, 134, 12308 is the lengthy and complex synthesis of the required azido-containing oligosaccharide oxazolines.

Rochefort et al. (Glycobiology, 2013, 24, 62-69) disclose the preparation of an antibody cultivated in the presence of N-azidoacetylmannosamine (Ac₄ManNAz), which was introduced at the natural N-linked glycosylation site, and the thus incorporated aizde moiety was conjugated to (PEG)₃-biotin wia a Staudinger reaction.

WO 2014/065661 (Synaffix B.V.) discloses modified antibodies wherein a sugar derivative is linked to a GlcNAc derivative of a glycan, as well as conjugation via the sugar derivative to form antibody-conjugates.

One limitation of the current technologies for the preparation of antibody conjugates via the N-glycan is the inherent dependence of such an approach to (a) naturally existing N-glycosylation site(s). All monoclonal antibodies of IgG-type have at least one N-glycosylation site at (or around) asparagine-297 of the heavy chain. The glycan at N297 is essential to induce effector function (antibody-dependent cellular cytotoxicity, ADCC) by binding to Fc-gamma receptors. The N297 glycan, however is not essential for retaining a long circulation time in blood, which is regulated by interaction of the C_{H}2-C_{H}3 domain interface of the antibody with the FcRn receptor. Apart from the N297 glycosylation site, approximately 20% of monoclonal antibodies harbour a second glycosylation site, typically in the Fab domain. The second glycosylation site, however, is not known to be essential for antibody activity of any kind and may as such be engineered out of the antibody if desirable (as for example applied in the development of trastuzumab).

Wright et al. (EMBO J. 1991, 10, 2717) describe a *de novo* engineering of a glycosylation site at specific position 54 or 60 in the V_{H} of two different antibodies TST2 and TSU7, respectively, and determine the influence of such sites on glycosylation processing, on antigen affinity and binding of the (Fab')₂-fragment derived from the antibody.

In US6254868 and EP97916787, it is described how 10 new glycosylation sites are designed and engineered into a humanized anti-CD22 monoclonal antibody, hLL2. Two CH₁ domain glycosylation sites, HCN1 and HCN5, were identified that were positioned favorably for glycosylation. The new glycosylation sites were applied for site-specific conjugation of chelates and drugs to the (Fab')₂-fragment derived from the antibody. Conjugation was effected , by means of periodate oxidation, then reductive amination, with negligible influence on immunoreactivity. Both the CH₁-appended CHOs conjugated equally efficiently with small chelates, it was concluded that the HCN5-CHOs, due to the structural and positional superiority, appears to be a better conjugation site for large drug complexes then conjugation in the variable domain of the antibody.

Wang et al. (Molecular & Cellular Proteomics, 2010, 9, 153-160) disclose the characterisation of O-GlcNAcylation sites during post-translational modification of proteins, wherein the O-GlcNAc site is tagged with a biotinylation reagent.

On the ADC Summit (San Francisco, 15 Oct 2013, see: URL:http://innate-pharma.com/sites/default/files/iph_adc_summit_2013.pdf), ADCs of the IgG mutants N297S and N297Q, via Q295 or via Q295 and N297Q respectively, were disclosed.

### Summary of the invention

The present invention relates to a process for the preparation of a modified antibody, the process comprising attaching a monosaccharide derivative Su(A)ₓ to a proximal N-linked GlcNAc-residue, in the presence of a catalyst selected from the group consisting of *β*(1,4)-galactosyltransferases, *β*(1,3)-N-galactosyltransferases, *β*(1,4)-galactosyltransferases comprising a mutant catalytic domain and *β*(1,3)-N-galactosyltransferases comprising a mutant catalytic domain; wherein Su(A)ₓ is defined as a monosaccharide derivative Su comprising x functional groups A wherein x is 1, 2, 3 or 4 and wherein A is selected from the group consisting of an azido group, a keto group, an alkynyl group, a thiol group or a precursor thereof, a halogen, a sulfonyloxy group, a halogenated acetamido group, a mercaptoacetamido group and a sulfonylated hydroxyacetamido group, wherein a precursor of a thiol group is -[C(R⁷)₂]ₒSC(O)CH₃, wherein R⁷ is independently selected from the group consisting of hydrogen, halogen and an optionally substituted C₁-C₂₄ alkyl group, and o is 0-24; wherein said proximal N-linked GlcNAc-residue is attached to the N-linked glycosylation site of an IgG antibody comprising one N-linked glycosylation site on the combination of a single heavy chain and single light chain, wherein said N-linked glycosylation site is a mutant N-linked glycosylation site as compared to its wild type counterpart; and wherein the proximal N-linked GlcNAc-residue is optionally fucosylated according to Formula (138) wherein b is 0 or 1.

The invention also relates to a modified IgG antibody comprising one N-linked glycosylation site on the combination of a single heavy chain and single light chain, wherein the N-linked glycosylation site is a mutant N-linked glycosylation site as compared to its wild type counterpart, wherein a proximal N-linked GlcNAc-Su(A)ₓ-substituent is attached to the antibody, wherein the GlcNAc in the N-linked GlcNAc-Su(A)ₓ-substituent is optionally fucosylated, and wherein Su(A)ₓ is defined as a monosaccharide derivative Su comprising x functional groups A wherein x is 1, 2, 3 or 4 and wherein A is selected from the group consisting of an azido group, a keto group, an alkynyl group, a thiol group or a precursor thereof, a halogen, a sulfonyloxy group, a halogenated acetamido group, a mercaptoacetamido group and a sulfonylated hydroxyacetamido group, wherein a precursor of a thiol group is -[C(R⁷)₂]ₒSC(O)CH₃, wherein R⁷ is independently selected from the group consisting of hydrogen, halogen and an optionally substituted C₁-C₂₄ alkyl group, and o is 0-24.

In addition, the invention relates to the use of the modified antibody according to the invention in the preparation of an antibody-conjugate, wherein an antibody-conjugate is defined as an antibody that is conjugated to a molecule of interest D via a linker L.

Also, the invention relates to a process for the preparation of an antibody-conjugate, said process comprising reacting a modified antibody according to the invention with a linker-conjugate, wherein said linker-conjugate comprises a functional group B and one or more molecules of interest, wherein said functional group B is a functional group that is capable of reacting with a functional group A on a glycan of the modified glycoprotein, and wherein functional group A is selected from the group consisting of an azido group, a keto group, an alkynyl group, a thiol group or a precursor thereof, a halogen, a sulfonyloxy group, a halogenated acetamido group, a mercaptoacetamido group and a sulfonylated hydroxyacetamido group, wherein a precursor of a thiol group is -[C(R⁷)₂]ₒSC(O)CH₃, wherein R⁷ is independently selected from the group consisting of hydrogen, halogen and an optionally substituted C₁-C₂₄ alkyl group, and o is 0-24.

The invention further relates to an antibody-conjugate and an antibody-drug conjugate obtainable by the process according to the invention.

In addition, the invention relates to an antibody-conjugate according to the invention, for use as a medicament.

### Description of the figures

Figure 1 shows examples of possible glycosylation profiles of monoclonal antibodies produced in a mammalian host. Only biantennary complex glycans are shown, other options are possible as well, e.g. sialylated glycans and triantennary structures.
Figure 2 shows the different glycoforms of a monoclonal antibody that can be obtained by regular expression followed by trimming with endo-glycosidase (**1**). Glycoform **2** can be obtained by expression of a mAb in a mammalian system in the presence of swainsonine or by expression in an engineered host organism, *e.g*. Pichia. Finally, glycoform **3** can be obtained by trimming of the regular mixture of glycoforms (G0, G1, G2, G0F, G1F and G2F) upon combined action of sialidase and galactosidase.
Figure 3 shows the enzymatic conversion of the mixture of glycoforms of a mAb into GlcNAc-terminated mAb **1** or **3** upon treatment with endo-glycosidase or a mixture of sialidase and galactosidase, respectively. Upon treatment of UDP-GalNAz in the presence of Gal-T1(Y289L), one or two GalNAz moieties per glycosylation site are introduced, respectively. The azide moieties in **4** and **6** serve as attachment point for functional group introduction by e.g. strain-promoted cycloaddition **(4→5)** or copper-catalyzed click reaction **(6→7).**
Figure 4 shows the structures of azido-modified galactose derivatives **(9-11)** for transfer onto a GlcNAc-terminated sugar under the action of a galactosyl transferase (or a mutant thereof).
Figure 5 shows the structures of other galactose derivatives **(12-27)** for transfer onto a GlcNAc-terminated sugar under the action of a galactosyl transferase (or a mutant thereof).
Figure 6 shows different glycoforms of a monoclonal antibody, e.g. IgG, upon removal of the native glycosylation site at N297, *e.g.* Asn to Gln mutation, and the *de novo* engineering of new glycosylation site at another site of the mAb, which may be on any position in the heavy or light chain.
Figure 7 shows the structures of modified UDP-galactose nucleotides **28-31** for enzymatic incorporation onto GlcNAc-terminated glycans upon the action of a galactosyltransferase.
Figure 8 shows the reaction scheme for the synthesis of BCN-MMAF conjugate **(34).**
Figure 9 shows the reaction scheme for the synthesis of BCN-maytansinoid conjugate **(35).**
Figure 10 shows (A) the mass spectral profile of trastuzumab mutant-(N297Q, L196N) as isolated after expression in CHO and (B) the mass spectral profile of trastuzumab-(N297Q, L196N) after treatment with endo F3 (or endo F2).
Figure 11 shows (A) the mass spectral profile of trastuzumab mutant (N297Q, L196N) after treatment with endo F3 (or endo F2) and after GalT1-mediated transfer of GalNAz onto the terminal GlcNAc and (B) the mass spectral profile of antibody drug conjugate resulting from conjugation of azide-charged trastuzumab mutant N297Q, L196N after treatment with BCN-vc-PABA-MMAF **34.**
Figure 12 shows the in vitro cytotoxicity of a range of ADCs against SK-Br-3 cell line.
Figure 13 shows the in vitro cytotoxicity of a range of ADCs against SK-OV-3 cell line.
Figure 14 shows the in vitro cytotoxicity of a range of ADCs against MDA-MB-231 cell line (negative control).
Figure 15 shows the in vivo efficacy profile of a range of ADCs, based on glyco-mutants of trastuzumab and conjugated to vc-PABA-maytansin.

### Detailed description

### Definitions

The verb "to comprise" as is used in this description and in the claims and its conjugations is used in its non-limiting sense to mean that items following the word are included, but items not specifically mentioned are not excluded.

In addition, reference to an element by the indefinite article "a" or "an" does not exclude the possibility that more than one of the element is present, unless the context clearly requires that there is one and only one of the elements. The indefinite article "a" or "an" thus usually means "at least one".

The compounds disclosed in this description and in the claims may comprise one or more asymmetric centres, and different diastereomers and/or enantiomers may exist of the compounds. The description of any compound in this description and in the claims is meant to include all diastereomers, and mixtures thereof, unless stated otherwise. In addition, the description of any compound in this description and in the claims is meant to include both the individual enantiomers, as well as any mixture, racemic or otherwise, of the enantiomers, unless stated otherwise. When the structure of a compound is depicted as a specific enantiomer, it is to be understood that the invention of the present application is not limited to that specific enantiomer.

The compounds may occur in different tautomeric forms. The compounds according to the invention are meant to include all tautomeric forms, unless stated otherwise. When the structure of a compound is depicted as a specific tautomer, it is to be understood that the invention of the present application is not limited to that specific tautomer.

Unsubstituted alkyl groups have the general formula CₙH₂ₙ₊₁ and may be linear or branched. Unsubstituted alkyl groups may also contain a cyclic moiety, and thus have the concomitant general formula CₙH₂ₙ₋₁. Optionally, the alkyl groups are substituted by one or more substituents further specified in this document. Examples of alkyl groups include methyl, ethyl, propyl, 2-propyl, t-butyl, 1-hexyl, 1-dodecyl, etc.

An aryl group comprises six to twelve carbon atoms and may include monocyclic and bicyclic structures. Optionally, the aryl group may be substituted by one or more substituents further specified in this document. Examples of aryl groups are phenyl and naphthyl.

Arylalkyl groups and alkylaryl groups comprise at least seven carbon atoms and may include monocyclic and bicyclic structures. Optionally, the arylalkyl groups and alkylaryl may be substituted by one or more substituents further specified in this document. An arylalkyl group is for example benzyl. An alkylaryl group is for example 4-*t*-butylphenyl.

Heteroaryl groups comprise at least two carbon atoms (i.e. at least C₂) and one or more heteroatoms N, O, P or S. A heteroaryl group may have a monocyclic or a bicyclic structure. Optionally, the heteroaryl group may be substituted by one or more substituents further specified in this document. Examples of suitable heteroaryl groups include pyridinyl, quinolinyl, pyrimidinyl, pyrazinyl, pyrazolyl, imidazolyl, thiazolyl, pyrrolyl, furanyl, triazolyl, benzofuranyl, indolyl, purinyl, benzoxazolyl, thienyl, phospholyl and oxazolyl.

Heteroarylalkyl groups and alkylheteroaryl groups comprise at least three carbon atoms (i.e. at least C₃) and may include monocyclic and bicyclic structures. Optionally, the heteroaryl groups may be substituted by one or more substituents further specified in this document.

Where an aryl group is denoted as a (hetero)aryl group, the notation is meant to include an aryl group and a heteroaryl group. Similarly, an alkyl(hetero)aryl group is meant to include an alkylaryl group and a alkylheteroaryl group, and (hetero)arylalkyl is meant to include an arylalkyl group and a heteroarylalkyl group. A C₂ - C₂₄ (hetero)aryl group is thus to be interpreted as including a C₂ - C₂₄ heteroaryl group and a C₆ - C₂₄ aryl group. Similarly, a C₃ - C₂₄ alkyl(hetero)aryl group is meant to include a C₇ - C₂₄ alkylaryl group and a C₃ - C₂₄ alkylheteroaryl group, and a C₃ - C₂₄ (hetero)arylalkyl is meant to include a C₇ - C₂₄ arylalkyl group and a C₃ - C₂₄ heteroarylalkyl group.

Unless stated otherwise, alkyl groups, alkenyl groups, alkenes, alkynes, (hetero)aryl groups, (hetero)arylalkyl groups and alkyl(hetero)aryl groups may be substituted with one or more substituents selected from the group consisting of C1 - C₁₂ alkyl groups, C₂ - C₁₂ alkenyl groups, C₂ - C₁₂ alkynyl groups, C₃ - C₁₂ cycloalkyl groups, C₅ - C₁₂ cycloalkenyl groups, C₈ - C₁₂ cycloalkynyl groups, C₁ - C₁₂ alkoxy groups, C₂ - C₁₂ alkenyloxy groups, C₂ - C₁₂ alkynyloxy groups, C₃ - C₁₂ cycloalkyloxy groups, halogens, amino groups, oxo and silyl groups, wherein the silyl groups can be represented by the formula (R¹⁰)₃Si-, wherein R¹⁰ is independently selected from the group consisting of C₁ - C₁₂ alkyl groups, C₂ - C₁₂ alkenyl groups, C₂ - C₁₂ alkynyl groups, C₃ - C₁₂ cycloalkyl groups, C₁ - C₁₂ alkoxy groups, C₂ - C₁₂ alkenyloxy groups, C₂ - C₁₂ alkynyloxy groups and C₃ - C₁₂ cycloalkyloxy groups, wherein the alkyl groups, alkenyl groups, alkynyl groups, cycloalkyl groups, alkoxy groups, alkenyloxy groups, alkynyloxy groups and cycloalkyloxy groups are optionally substituted, the alkyl groups, the alkoxy groups, the cycloalkyl groups and the cycloalkoxy groups being optionally interrupted by one of more hetero-atoms selected from the group consisting of O, N and S.

An alkynyl group comprises a carbon-carbon triple bond. An unsubstituted alkynyl group comprising one triple bond has the general formula CₙH₂ₙ₋₃. A terminal alkynyl is an alkynyl group wherein the triple bond is located at a terminal position of a carbon chain. Optionally, the alkynyl group is substituted by one or more substituents further specified in this document, and/or interrupted by heteroatoms selected from the group of oxygen, nitrogen and sulphur. Examples of alkynyl groups include ethynyl, propynyl, butynyl, octynyl, etc.

A cycloalkynyl group is a cyclic alkynyl group. An unsubstituted cycloalkynyl group comprising one triple bond has the general formula CₙH₂ₙ₋₅. Optionally, a cycloalkynyl group is substituted by one or more substituents further specified in this document. An example of a cycloalkynyl group is cyclooctynyl.

A heterocycloalkynyl group is a cycloalkynyl group interrupted by heteroatoms selected from the group of oxygen, nitrogen and sulphur. Optionally, a heterocycloalkynyl group is substituted by one or more substituents further specified in this document. An example of a heterocycloalkynyl group is azacyclooctynyl.

A (hetero)aryl group comprises an aryl group and a heteroaryl group. An alkyl(hetero)aryl group comprises an alkylaryl group and an alkylheteroaryl group. A (hetero)arylalkyl group comprises a arylalkyl group and a heteroarylalkyl groups. A (hetero)alkynyl group comprises an alkynyl group and a heteroalkynyl group. A (hetero)cycloalkynyl group comprises an cycloalkynyl group and a heterocycloalkynyl group.

A (hetero)cycloalkyne compound is herein defined as a compound comprising a (hetero)cycloalkynyl group.

Several of the compounds disclosed in this description and in the claims may be described as fused (hetero)cycloalkyne compounds, *i.e.* (hetero)cycloalkyne compounds wherein a second ring structure is fused, i.e. annelated, to the (hetero)cycloalkynyl group. For example in a fused (hetero)cyclooctyne compound, a cycloalkyl (e.g. a cyclopropyl) or an arene (e.g. benzene) may be annelated to the (hetero)cyclooctynyl group. The triple bond of the (hetero)cyclooctynyl group in a fused (hetero)cyclooctyne compound may be located on either one of the three possible locations, *i.e.* on the 2, 3 or 4 position of the cyclooctyne moiety (numbering according to "IUPAC Nomenclature of Organic Chemistry", Rule A31.2). The description of any fused (hetero)cyclooctyne compound in this description and in the claims is meant to include all three individual regioisomers of the cyclooctyne moiety.

When an alkyl group, a (hetero)aryl group, alkyl(hetero)aryl group, a (hetero)arylalkyl group, a (hetero)cycloalkynyl group is optionally substituted, said groups are independently optionally substituted with one or more substituents independently selected from the group consisting of C₁ - C₁₂ alkyl groups, C₂ - C₁₂ alkenyl groups, C₂ - C₁₂ alkynyl groups, C₃ - C₁₂ cycloalkyl groups, C₁ - C₁₂ alkoxy groups, C₂ - C₁₂ alkenyloxy groups, C₂ - C₁₂ alkynyloxy groups, C₃ - C₁₂ cycloalkyloxy groups, halogens, amino groups, oxo groups and silyl groups, wherein the alkyl groups, alkenyl groups, alkynyl groups, cycloalkyl groups, alkoxy groups, alkenyloxy groups, alkynyloxy groups and cycloalkyloxy groups are optionally substituted, the alkyl groups, the alkoxy groups, the cycloalkyl groups and the cycloalkoxy groups being optionally interrupted by one of more hetero-atoms selected from the group consisting of O, N and S, wherein the silyl groups are represented by the formula (R⁶)₃Si-, wherein R⁶ is independently selected from the group consisting of C₁ - C₁₂ alkyl groups, C₂ - C₁₂ alkenyl groups, C₂ - C₁₂ alkynyl groups, C₃ - C₁₂ cycloalkyl groups, C₁ - C₁₂ alkoxy groups, C₂ - C₁₂ alkenyloxy groups, C₂ - C₁₂ alkynyloxy groups and C₃ - C₁₂ cycloalkyloxy groups, wherein the alkyl groups, alkenyl groups, alkynyl groups, cycloalkyl groups, alkoxy groups, alkenyloxy groups, alkynyloxy groups and cycloalkyloxy groups are optionally substituted, the alkyl groups, the alkoxy groups, the cycloalkyl groups and the cycloalkoxy groups being optionally interrupted by one of more hetero-atoms selected from the group consisting of O, N and S.

The general term "sugar" is herein used to indicate a monosaccharide, for example glucose (Glc), galactose (Gal), mannose (Man) and fucose (Fuc). The term "sugar derivative" is herein used to indicate a derivative of a monosaccharide sugar, i.e. a monosaccharide sugar comprising substituents and/or functional groups. Examples of a sugar derivative include amino sugars and sugar acids, e.g. glucosamine (GlcNH₂), galactosamine (GalNH₂) N-acetylglucosamine (GlcNAc), N-acetylgalactosamine (GalNAc), sialic acid (Sia) which is also referred to as N-acetylneuraminic acid (NeuNAc), and N-acetylmuramic acid (MurNAc), glucuronic acid (GlcA) and iduronic acid (IdoA). Examples of a sugar derivative also include compounds herein denoted Su(A)ₓ, wherein Su is a sugar or a sugar derivative, and wherein Su comprises x functional groups A.

The term "nucleotide" herein refers to a molecule that is composed of a nucleobase, a five-carbon sugar (either ribose or deoxyribose) and one, two or three phosphate groups. Without the phosphate group, the nucleobase and sugar compose a nucleoside. A nucleotide can thus also be called a nucleoside monophosphate, a nucleoside diphosphate or a nucleoside triphosphate. The nucleobase may be adenine, guanine, cytosine, uracil or thymine. Examples of a nucleotide include uridine diphosphate (UDP), guanosine diphosphate (GDP), thymidine diphosphate (TDP), cytidine diphosphate (CDP) and cytidine monophosphate (CMP).

The term "protein" is herein used in its normal scientific meaning. Herein, polypeptides comprising about 10 or more amino acids are considered proteins. A protein may comprise natural, but also unnatural amino acids.

The term "glycoprotein" herein refers to a protein comprising one or more monosaccharide or oligosaccharide chains ("glycans") covalently bonded to the protein. A glycan may be attached to a hydroxyl group on the protein (O-linked-glycan), *e.g.* to the hydroxyl group of serine, threonine, tyrosine, hydroxylysine or hydroxyproline, or to an amide function on the protein (*N*-glycoprotein), *e.g.* asparagine or arginine, or to a carbon on the protein (*C*-glycoprotein), *e.g*. tryptophan. A glycoprotein may comprise more than one glycan, may comprise a combination of one or more monosaccharide and one or more oligosaccharide glycans, and may comprise a combination of N-linked, O-linked and C-linked glycans. It is estimated that more than 50% of all proteins have some form of glycosylation and therefore qualify as glycoprotein. Examples of glycoproteins include PSMA (prostate-specific membrane antigen), CAL (candida antartica lipase), gp41, gp120, EPO (erythropoietin), antifreeze protein and antibodies.

The term "glycan" herein refers to a monosaccharide or oligosaccharide chain that is linked to a protein. The term glycan thus refers to the carbohydrate-part of a glycoprotein. The glycan is attached to a protein via the C-1 carbon of one sugar, which may be without further substitution (monosaccharide) or may be further substituted at one or more of its hydroxyl groups (oligosaccharide). A naturally occurring glycan typically comprises 1 to about 10 saccharide moieties. However, when a longer saccharide chain is linked to a protein, said saccharide chain is herein also considered a glycan.

A glycan of a glycoprotein may be a monosaccharide. Typically, a monosaccharide glycan of a glycoprotein consists of a single N-acetylglucosamine (GlcNAc), glucose (Glc), mannose (Man) or fucose (Fuc) covalently attached to the protein.

A glycan may also be an oligosaccharide. An oligosaccharide chain of a glycoprotein may be linear or branched. In an oligosaccharide, the sugar that is directly attached to the protein is called the core sugar. In an oligosaccharide, a sugar that is not directly attached to the protein and is attached to at least two other sugars is called an internal sugar. In an oligosaccharide, a sugar that is not directly attached to the protein but to a single other sugar, i.e. carrying no further sugar substitutents at one or more of its other hydroxyl groups, is called the terminal sugar. For the avoidance of doubt, there may exist multiple terminal sugars in an oligosaccharide of a glycoprotein, but only one core sugar.

A glycan may be an O-linked glycan, an N-linked glycan or a C-linked glycan. In an O-linked glycan a monosaccharide or oligosaccharide glycan is bonded to an O-atom in an amino acid of the protein, typically via a hydroxyl group of serine (Ser) or threonine (Thr). In an N-linked glycan a monosaccharide or oligosaccharide glycan is bonded to the protein via an N-atom in an amino acid of the protein, typically via an amide nitrogen in the side chain of asparagine (Asn) or arginine (Arg). In a C-linked glycan a monosaccharide or oligosaccharide glycan is bonded to a C-atom in an amino acid of the protein, typically to a C-atom of tryptophan (Trp).

The end of an oligosaccharide that is directly attached to the protein is called the reducing end of a glycan. The other end of the oligosaccharide is called the non-reducing end of a glycan.

For O-linked glycans, a wide diversity of chains exists. Naturally occurring O-linked glycans typically feature a serine or threonine-linked α-O-GalNAc moiety, further substituted with galactose, sialic acid and/or fucose. The hydroxylated amino acid that carries the glycan substitution may be part of any amino acid sequence in the protein.

For N-linked glycans, a wide diversity of chains exist. Naturally occurring N-linked glycans typically feature an asparagine-linked β-N-GlcNAc moiety, in turn further substituted at its 4-OH with β-GlcNAc, in turn further substituted at its 4-OH with β-Man, in turn further substituted at its 3-OH and 6-OH with α-Man, leading to the glycan pentasaccharide Man₃GlcNAc₂. The core GlcNAc moiety may be further substituted at its 6-OH by α-Fuc. The pentasaccharide Man₃GlcNAc₂ is the common oligosaccharide scaffold of nearly all *N*-linked glycoproteins and may carry a wide variety of other substituents, including but not limited to Man, GlcNAc, Gal and sialic acid. The asparagine that is substituted with the glycan on its side-chain is typically part of the sequence Asn-X-Ser/Thr, with X being any amino acid but proline and Ser/Thr being either serine or threonine.

The term "antibody" is herein used in its normal scientific meaning. An antibody is a protein generated by the immune system that is capable of recognizing and binding to a specific antigen. An antibody is an example of a glycoprotein. The term antibody herein is used in its broadest sense and specifically includes monoclonal antibodies, polyclonal antibodies, dimers, multimers, multispecific antibodies (e.g. bispecific antibodies), antibody fragments, and double and single chain antibodies. The term "antibody" is herein also meant to include human antibodies, humanized antibodies, chimeric antibodies and antibodies specifically binding cancer antigen. The term "antibody" is meant to include whole antibodies, but also fragments of an antibody, for example an antibody Fab fragment, (Fab')₂, Fv fragment or Fc fragment from a cleaved antibody, a scFv-Fc fragment, a minibody, a diabody or a scFv. Furthermore, the term includes genetically engineered antibodies and derivatives of an antibody. Antibodies, fragments of antibodies and genetically engineered antibodies may be obtained by methods that are known in the art. Suitable marketed antibodies include, amongst others, abciximab, rituximab, basiliximab, palivizumab, infliximab, trastuzumab, alemtuzumab, adalimumab, tositumomab-¹³¹I, cetuximab, ibrituximab tiuxetan, omalizumab, bevacizumab, natalizumab, ranibizumab, panitumumab, eculizumab, certolizumab pegol, golimumab, canakinumab, catumaxomab, ustekinumab, tocilizumab, ofatumumab, denosumab, belimumab, ipilimumab and brentuximab.

The terms "treatment," "treating," and the like refer to obtaining a desired pharmacologic and/or physiologic effect. The effect may be prophylactic in terms of completely or partially preventing a disease or symptom thereof and/or may be therapeutic in terms of a partial or complete cure for a disease and/or adverse affect attributable to the disease. "Treatment," as used herein, covers any treatment of a disease in a mammal, particularly in a human, and includes preventing the disease from occurring in a subject which may be predisposed to the disease but has not yet been diagnosed as having it; inhibiting the disease, i.e., arresting its development; relieving the disease, i.e., causing regression of the disease.

### Process for the preparation of a modified antibody

The present invention relates to a process for the preparation of a modified antibody, the process comprising attaching a monosaccharide derivative Su(A)ₓ to a proximal N-linked GlcNAc-residue, in the presence of a suitable catalyst; wherein Su(A)ₓ is defined as a monosaccharide derivative Su comprising x functional groups A wherein x is 1, 2, 3 or 4 and wherein A is selected from the group consisting of an azido group, a keto group, an alkynyl group, a thiol group or a precursor thereof, a halogen, a sulfonyloxy group, a halogenated acetamido group, a mercaptoacetamido group and a sulfonylated hydroxyacetamido group; wherein said proximal N-linked GlcNAc-residue is attached to the N-linked glycosylation site of an IgG antibody comprising one N-linked glycosylation site on the combination of a single heavy chain and single light chain, wherein said N-linked glycosylation site is a mutant N-linked glycosylation site as compared to its wild type counterpart; and wherein said proximal N-linked GlcNAc-residue is optionally fucosylated.

The catalyst is selected from the group consisting of *β*(1,4)-galactosyltransferases, *β*(1,3)-N-galactosyltransferases, *β*(1,4)-galactosyltransferases comprising a mutant catalytic domain and *β*(1,3)-N-galactosyltransferases comprising a mutant catalytic domain.

The modified antibody that is obtainable by the process according to the invention is described in more detail below.

In the process for the preparation of a modified antibody according to the invention, a monosaccharide derivative Su(A)ₓ is attached to a proximal N-linked GlcNAc-residue, in the presence of a galactosyltransferase. Said proximal N-linked GlcNAc-residue is attached, via an N-glycosidic bond, to the N-linked glycosylation site of an IgG antibody comprising one, and no more than one, N-linked glycosylation site on the combination of a single heavy chain and single light chain, wherein said N-linked glycosylation site is a mutant N-linked glycosylation site as compared to its wild type counterpart.

When an IgG antibody is a whole antibody, the antibody is typically composed of two immunoglobulin (Ig) heavy chains and two Ig light chains. The Ig heavy chains comprise a constant region, composed of the constant domains C_{H}1, C_{H}2 and C_{H}3, and a variable region, V_{H}. The Ig light chains are composed of a variable region V_{L} and a constant region C_{L}.

Herein, the term "the combination of a single heavy chain and single light chain" refers to the combination of one heavy and one light Ig chain. Since a whole antibody is a symmetrical dimer of two heavy and two light chains, a whole antibody is composed of two of said combinations of a single heavy chain and single light chain. The term "combination of a single heavy chain and single light chain" is herein merely used as a means to define the parts of an IgG antibody where said N-glycosylation sites may be present, and herein does not refer to e.g. a reduced IgG antibody, unless otherwise stated.

The term "an IgG antibody comprising one N-linked glycosylation site on the combination of a single heavy chain and single light chain" herein thus defines that one, and no more than one, N-linked glycosylation site is present in the C_{H}1, C_{H}2, C_{H}3 or V_{H} domain of the heavy chain, or in the C_{L} or V_{L} domain of the light chain of said combination.

It is to be understood that, when the IgG antibody is a whole antibody, since the combination of a single heavy chain and single light chain comprises one N-linked glycosylation site, the whole antibody thus comprises two, and no more than two, N-linked glycosylation sites, one on each combination of a single heavy chain and single light chain.

As defined above, the term "antibody" herein not only refers to whole antibodies, but also to antibody fragments, and said IgG antibody comprising one N-linked glycosylation site on the combination of a single heavy chain and single light chain may also be an antibody fragment. For example an Fc fragment comprising one N-linked glycosylation site on the C_{H}2 or C_{H}3 domain is herein also deemed an IgG antibody comprising one N-linked glycosylation site on the combination of a single heavy chain and single light chain, even though said fragment does not comprise a light chain domain. Examples of antibody fragments that herein may be deemed an IgG antibody comprising one N-linked glycosylation site on the combination of a single heavy chain and single light chain include Fc fragments, Fab fragments, (Fab')₂ fragments, Fab' fragments, scFv fragments, reduced antibodies, diabodies, triabodies, tetrabodies, etc., provided that said fragments comprise one, and only one, N-linked glycosylation site.

In a preferred embodiment, the IgG antibody comprising one N-linked glycosylation site on the combination of a single heavy chain and single light chain is a whole antibody. When an IgG antibody comprising one N-linked glycosylation site on the combination of a single heavy chain and single light chain is a whole antibody, i.e. an antibody comprising two heavy chains and two light chains, then the whole antibody comprises two N-linked glycosylation sites.

The term "N-linked glycosylation site" herein refers to a site on an antibody where a mono- or oligosaccharide is attached to the antibody via an N-glycosidic bond. A mono- or oligosaccharide that is attached to an antibody is herein also referred to as a glycan.

The Fc regions of IgG antibodies bear a highly conserved N-glycosylation site. A conserved N-linked glycosylation site may also be referred to as a native N-linked glycosylation site. The N-glycans attached to this conserved site are predominantly core-fucosylated diantennary structures of the complex type. In addition, small amounts of these N-glycans also bear bisecting GlcNAc and α-2,6-linked sialic acid residues. The native glycosylation site in IgG is present at (or around) asparagine 297, also referred to as Asn297 or N297.

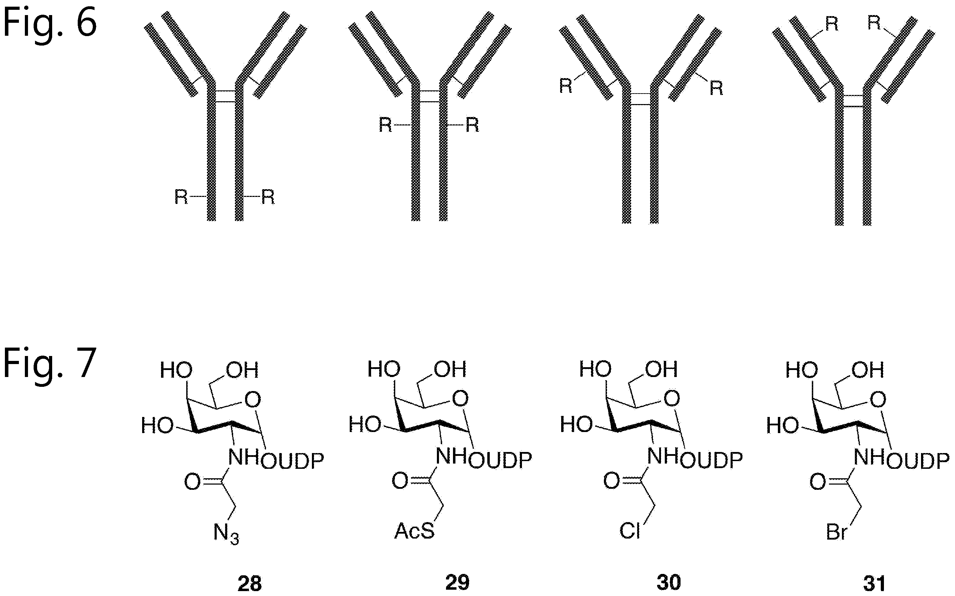

Figure 6 shows several examples of an IgG antibody comprising one N-linked glycosylation site on the combination of a single heavy chain and single light chain, neither of which glycosylation sites is the conserved glycosylation site at N297.

The term "proximal N-linked GlcNAc-residue", herein also referred to as "core-GlcNAc-substituent", refers to a GlcNAc-residue that is covalently bonded to the N-glycosylation site of the IgG antibody comprising one N-linked glycosylation site on the combination of a single heavy chain and single light chain via an N-glycosidic bond, preferably via C1 of said GlcNAc residue to an amide group in the side chain of an antibody amino acid, more preferably to an amide in the side chain of an Asparagine or arginine amino acid. Said proximal N-linked GlcNAc-residue or core-GlcNAc-substituent is optionally fucosylated. A proximal N-linked GlcNAc-residue is according to formula (**138**):

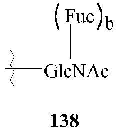

As was described above, in the process for the preparation of a modified antibody according to the invention, a monosaccharide derivative Su(A)ₓ is attached to a proximal N-linked GlcNAc-residue, in the presence of a galactosyltransferase.

In a preferred embodiment, said process comprises contacting an IgG antibody comprising one N-linked glycosylation site on the combination of a single heavy chain and single light chain, wherein a proximal N-linked GlcNAc-residue is attached to the antibody at said glycosylation site, with Su(A)ₓ-P in the presence of a suitable catalyst; wherein the N-linked glycosylation site is a mutant N-linked glycosylation site as compared to its wild type counterpart; wherein Su(A)ₓ-P is a sugar derivative Su comprising x functional groups A wherein x is 1, 2, 3 or 4 and wherein A is selected from the group consisting of an azido group, a keto group, an alkynyl group, a thiol group or a precursor thereof, a halogen, a sulfonyloxy group, a halogenated acetamido group, a mercaptoacetamido group and a sulfonylated hydroxyacetamido group; wherein P is a nucleotide; and wherein a suitable catalyst is defined as a catalyst wherefore Su(A)ₓ-P is a substrate. A preferred embodiment of the process, wherein said IgG antibody is a whole antibody, is shown in Scheme 1.

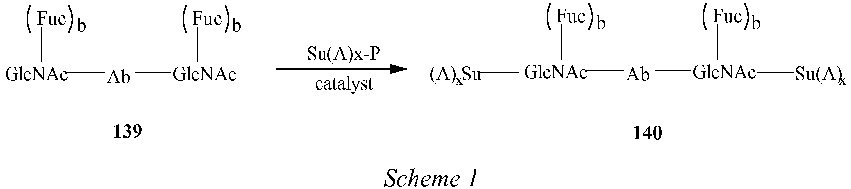

In Scheme 1, Ab refers to the IgG antibody comprising one, and no more than one, N-linked glycosylation site on the combination of a single heavy chain and single light chain, wherein the N-linked glycosylation site is a mutant N-linked glycosylation site as compared to its wild type counterpart, b is 0 (non-fucosylated) or 1 (fucosylated) and Su(A)x-P is as defined above.

Since the IgG antibody comprises one, and no more than one, N-linked glycosylation site on the combination of a single heavy chain and single light chain, and a whole antibody consists of two of said combinations, when the IgG antibody is a whole antibody, said whole antibody comprises two, and no more than two, N-glycosylation sites.

The process for the preparation of a modified antibody according to the invention is performed in the presence of a suitable catalyst, preferably by the action of a suitable catalyst. A suitable catalyst is defined as a catalyst, wherefore Su(A)ₓ-P is a substrate. The catalyst is selected from the group consisting of *β*(1,4)-galactosyltransferases, *β*(1,3)-N-galactosyltransferases, *β*(1,4)-galactosyltransferases comprising a mutant catalytic domain and *β*(1,3)-N-galactosyltransferases comprising a mutant catalytic domain.

When the catalyst is a galactosyltransferase without a mutant domain, said galactosyltransferase preferably is a wild-type galactosyltransferase. When the enzyme is a galactosyltransferase comprising a mutant catalytic domain, said mutant GalT domain may be present within a full-length GalT enzyme, but it may also be present in a recombinant molecule comprising a catalytic domain.

In one embodiment, the catalyst is a wild-type galactosyltransferase, i.e. a wild-type *β*(1,4)-galactosyltransferase or a wild-type *β*(1,3)-N-galactosyltransferase, and even more preferably a wild-type *β*(1,4)-galactosyltransferase. *β*(1,4)-Galactosyltransferase is herein further referred to as GalT. Even more preferably, the *β*(1,4)-galactosyltransferase is selected from the group consisting of a bovine *β*(1,4)-Gal-T1, a human *β*(1,4)-Gal-T1, a human *β*(1,4)-Gal-T2, a human *β*(1,4)-Gal-T3 and a human *β*(1,4)-Gal-T4. Even more preferably, the *β*(1,4)-galactosyltransferase is a *β*(1,4)-Gal-T1. When the catalyst is a wild-type *β*(1,3)-N-galactosyltransferase, a human *β*(1,3)-Gal-T5 is preferred.

This embodiment wherein the catalyst is a wild-type galactosyltransferase is particularly preferred when a functional group A in sugar derivative Su(A)ₓ is present on C2 or C6, preferably C6, of said sugar derivative. In this embodiment, it is further preferred that Su(A)ₓ comprises one functional group A, i.e. preferably x is 1. P, Su(A)ₓ and Su(A)ₓ-P are described in more detail below.

In a specific aspect of the process according to the disclosure, the process comprises contacting an IgG antibody comprising a proximal N-linked GlcNAc-residue with Su(A)ₓ-P in the presence of a suitable catalyst; wherein the proximal N-linked GlcNAc residue of said antibody is optionally fucosylated; wherein a suitable catalyst is defined as a galactosyltransferase or a galactosyltransferase comprising a mutant catalytic domain, wherefore Su(A)ₓ-P is a substrate; wherein Su(A)ₓ is a sugar derivative comprising x functional groups A wherein x is 1, 2, 3 or 4 and A is independently selected from the group consisting of an azido group, a keto group, an alkynyl group, a thiol group or a precursor thereof, a halogen, a sulfonyloxy group, a halogenated acetamido group, a mercaptoacetamido group and a sulfonylated hydroxyacetamido group; wherein P is a nucleotide; with the proviso that when the catalyst is a wild-type galactosyltransferase, then Su(A)ₓ-P comprises one functional group A (i.e. x is 1), and said functional group A is present on C2 or C6, preferably C6, of Su(A)ₓ.

Accordingly, in a specific embodiment, the process for the preparation of a modified antibody comprises contacting an IgG antibody comprising a proximal N-linked GlcNAc-residue with Su(A)ₓ-P in the presence of a suitable catalyst; wherein the proximal N-linked GlcNAc-residue is optionally fucosylated; wherein a suitable catalyst is defined as a wild-type galactosyltransferase wherefore Su(A)ₓ-P is a substrate; wherein Su(A)ₓ is a sugar derivative comprising x functional groups A wherein x is 1, A is present on C2 or C6, preferably C6, of sugar derivative Su and A is independently selected from the group consisting of an azido group, a keto group, an alkynyl group, a thiol group or a precursor thereof, a halogen, a sulfonyloxy group, a halogenated acetamido group, a mercaptoacetamido group and a sulfonylated hydroxyacetamido group; wherein P is a nucleotide.

The wild-type galactosyltransferase in this specific embodiment is a *β*(1,4)-galactosyltransferase or a *β*(1,3)-N-galactosyltransferase, more preferably a *β*(1,4)-galactosyltransferase. Even more preferably, the wild-type a *β*(1,4)-galactosyltransferase is a wild-type human GalT, more preferably a wild-type human GalT selected from the group consisting of a wild-type human *β*4-Gal-T1, a wild-type human *β*(1,4)-Gal-T2, a wild-type human *β*(1,4)-Gal-T3 and a wild-type human *β*(1,4)-Gal-T4.

In another embodiment of the process for the preparation of an antibody-conjugate according to the invention, the catalyst is a galactosyltransferase comprising a mutant catalytic domain, i.e. a *β*(1,4)-galactosyltransferase comprising a mutant catalytic domain or a *β*(1,3)-N-galactosyltransferase comprising a mutant catalytic domain, more preferably a *β*(1,4)-galactosyltransferase comprising a mutant catalytic domain. *β*(1,4)-Galactosyltransferase is herein further referred to as GalT.

In a preferred embodiment the catalyst is a *β*(1,3)-N-galactosyltransferase comprising a mutant catalytic domain, and preferably said *β*(1,3)-N-galactosyltransferase is a human *β*(1-3)-Gal-T5.

More preferably, the catalyst is a *β*(1,4)-N-galactosyltransferase comprising a mutant catalytic domain, more preferably, a *β*(1,4)-galactosyltransferase I comprising a mutant catalytic domain, and even more preferably selected from the group consisting of a bovine *β*(1,4)-Gal-T1, a human *β*4-Gal-T1, a human *β*(1,4)-Gal-T2, a human *β*(1,4)-Gal-T3 and a human *β*(1,4)-Gal-T4, all comprising a mutant catalytic domain.

Most preferably the catalyst is a bovine *β*(1,4)-Gal-T1 comprising a mutant catalytic domain.

Several suitable catalysts for the process for the preparation of modified antibody according to the invention are known in the art. A suitable catalyst is for example a catalyst that comprises a mutant catalytic domain from a *β*(1,4)-galactosyltransferase I. A catalytic domain herein refers to an amino acid segment that folds into a domain that is able to catalyze the linkage of the specific sugar derivative nucleotide Su(A)ₓ-P to the terminal non-reducing GlcNAc-glycan in a specific process according to the invention. *β*(1,4)-galactosyltransferase I is herein further referred to as GalT. Such mutant GalT catalytic domains are for example disclosed in J. Biol. Chem. 2002, 277, 20833 and WO 2004/063344 (National Institutes of Health). J. Biol. Chem. 2002, 277, 20833 and WO 2004/063344 disclose Tyr-289 mutants of bovine *β*(1,4)-Gal-T1, which are referred to as Y289L, Y289N and Y289I. The method of preparation of said mutant catalytic domains Y289L, Y289N and Y289I is disclosed in detail in WO 2004/063344, p. 34,1. 6 - p. 36,1. 2.

Mutant GalT domains that catalyze the formation of a glucose-*β*(1,4)-N-acetylglucosamine bond are disclosed in WO 2004/063344 on p. 10, 1, 25 - p. 12, 1. 4. Mutant GalT domains that catalyze the formation of an N-acetylgalactosamine-*β*(1,4)-N-acetylglucosamine bond are disclosed in WO 2004/063344 on p. 12,1, 6 - p. 13, 1. 2. Mutant GalT domains that catalyze the formation of a N-acetylglucosamine-*β*(1,4)-N-acetylglucosamine bond and a mannose-*β*(1,4)-N-acetylglucosamine bond are disclosed in WO 2004/063344 on p. 12,1, 19 - p. 14,1. 6.

The disclosed mutant GalT domains may be included within full-length GalT enzymes, or in recombinant molecules containing the catalytic domains, as is disclosed in WO 2004/063344 on p. 14,1, 31 - p. 16,1. 28.

Another mutant GalT domain is for example Y284L, disclosed by Bojarová et al., Glycobiology 2009, 19, 509, wherein Tyr284 is replaced by leucine.

Another mutant GalT domain is for example R228K, disclosed by Qasba et al., Glycobiology 2002, 12, 691, wherein Arg228 is replaced by lysine.

The catalyst may also comprise a mutant catalytic domain from a bovine *β*(1,4)-galactosyltransferase, selected from the group consisting of GalT Y289N, GalT Y289F, GalT Y289M, GalT Y289V, GalT Y289G, GalT Y289I and GalT Y289A, preferably selected from the group consisting of GalT Y289F and GalT Y289M. GalT Y289N, GalT Y289F, GalT Y289M, GalT Y289V, GalT Y289G, GalT Y289I and GalT Y289A may be provided via site-directed mutagenesis processes, in a similar manner as disclosed in WO 2004/063344, in Qasba et al., Prot. Expr. Pur. 2003, 30, 219 and in Qasba et al., J. Biol. Chem. 2002, 277, 20833 for Y289L, Y289N and Y289I. In GalT Y289N the tyrosine amino acid (Y) at position 289 is replaced by an asparagine (N) amino acid, in GalT Y289F the tyrosine amino acid (Y) at position 289 is replaced by a phenylalanine (F) amino acid, in GalT Y289M said tyrosine is replaced by a methionine (M) amino acid, in GalT Y289V by a valine (V) amino acid, in GalT Y289G by a glycine (G) amino acid, in GalT Y289I by an isoleucine (I) amino acid and in Y289A by an analine (A) amino acid.

In a preferred embodiment of the process for the preparation of a modified antibody according to the invention, said catalyst is a catalyst comprising a mutant catalytic domain from a *β*(1,4)-galactosyltransferase, preferably from a bovine β(1,4)-Gal-T1.

Preferably, the catalyst is a catalyst comprising a mutant catalytic domain from a *β*(1,4)-galactosyltransferase, preferably selected from the group consisting of bovine *β*(1,4)-Gal-T1 GalT Y289L, GalT Y289N, GalT Y289I, GalT Y289F, GalT Y289M, GalT Y289V, GalT Y289G and GalT Y289A, more preferably selected from the group consisting of bovine *β*(1,4)-Gal-T1 GalT Y289L, GalT Y289N and GalT Y289I.

In a further preferred embodiment, said catalyst is a catalyst comprising a GalT mutant catalytic domain selected from the group consisting of Y289L, Y289N, Y289I, Y284L, R228K, Y289F, Y289M, Y289V, Y289G and Y289A, preferably selected from the group consisting of Y289L, Y289N, Y289I, Y284L and R228K. In another preferred embodiment, said catalyst is a catalyst comprising a bovine *β*(1,4)-Gal-T1 mutant catalytic domain selected from the group consisting of Y289F, Y289M, Y289V, Y289G and Y289A. More preferably said catalyst is a catalyst comprising a GalT mutant catalytic domain selected from the group consisting of Y289L, Y289N and Y289I, and most preferably said catalyst is a catalyst comprising a GalT mutant catalytic domain selected from the group consisting of Y289L.

Another type of suitable catalysts is a catalyst based on *α*(1,3)-N-galactosyltransferase (further referred to as α3Gal-T), preferably *α*(1,3)-N-acetylgalactosaminyltransferase (further referred to as α3GalNAc-T), as disclosed in WO 2009/025646. Mutation of α3Gal-T can broaden donor specificity of the enzyme, and make it an α3GalNAc-T. Mutation of α3GalNAc-T can broaden donor specificity of the enzyme. Polypeptide fragments and catalytic domains of *α*(1,3)-N-acetylgalactosaminyltransferases are disclosed in WO 2009/025646 on p. 26, 1. 18 - p. 47, 1. 15 and p. 77,1. 21 - p. 82, l. 4.

The process for the preparation of a modified antibody is preferably performed in a suitable buffer solution, such as for example phosphate, buffered saline (e.g. phosphate-buffered saline, tris-buffered saline), citrate, HEPES, tris and glycine. Suitable buffers are known in the art. Preferably, the buffer solution is phosphate-buffered saline (PBS) or tris buffer.

The process is preferably performed at a temperature in the range of about 4 to about 50°C, more preferably in the range of about 10 to about 45°C, even more preferably in the range of about 20 to about 40°C, and most preferably in the range of about 30 to about 37°C.

The process is preferably performed a pH in the range of about 5 to about 9, preferably in the range of about 5.5 to about 8.5, more preferably in the range of about 6 to about 8. Most preferably, the process is performed at a pH in the range of about 7 to about 8.

Su(A)ₓ is defined as a sugar derivative comprising x functional groups A, wherein x is 1, 2, 3 or 4 and wherein A is independently selected from the group consisting of an azido group, a keto group an alkynyl group, a thiol group or a precursor thereof, a halogen, a sulfonyloxy group, a halogenated acetamido group, a mercaptoacetamido group and a sulfonylated hydroxyacetamido group.

A Su(A)ₓ-moiety may also be referred to as a "modified sugar". A modified sugar is herein defined as a sugar or a sugar derivative, said sugar or sugar derivative comprising 1, 2, 3 or 4 functional groups A, wherein A is selected from the group consisting of an azido group, a keto group an alkynyl group, a thiol group or a precursor thereof, a halogen, a sulfonyloxy group, a halogenated acetamido group, a mercaptoacetamido group and a sulfonylated hydroxyacetamido group.

When a modified sugar or sugar derivative comprises e.g. an azido group, said sugar or sugar derivative may be referred to as an azido-modified sugar or sugar derivative. When a modified sugar or sugar derivative comprises e.g. a keto group, said sugar or sugar derivative may be referred to as a keto-modified sugar or sugar derivative. When a modified sugar or sugar derivative comprises e.g. an alkynyl group, said sugar or sugar derivative may be referred to as an alkynyl-modified sugar or sugar derivative. When a modified sugar or sugar derivative comprises e.g. a thiol group, said sugar or sugar derivative may be referred to as a thiol-modified sugar or sugar derivative. When a modified sugar or sugar derivative comprises e.g. a thiol-precursor group, said sugar or sugar derivative may be referred to as a thiol-precursor-modified sugar or sugar derivative. When a modified sugar or sugar derivative comprises e.g. a halogen, said sugar or sugar derivative may be referred to as a halogen-modified sugar or sugar derivative. When a modified sugar or sugar derivative comprises e.g. a sulfonyloxy group, said sugar or sugar derivative may be referred to as a sulfonyloxy-modified sugar or sugar derivative.

An azido group is herein defined as a -[C(R⁷)₂]ₒN₃ group, wherein R⁷ is independently selected from the group consisting of hydrogen, halogen and an (optionally substituted) C₁ - C₂₄ alkyl group, and o is 0 - 24. Preferably R⁷ is hydrogen or a C₁, C₂, C₃ or C₄ alkyl group, more preferably R⁷ is hydrogen or -CH₃. Preferably o is 0 - 10, more preferably 0, 1, 2, 3, 4, 5 or 6. More preferably, R⁷ is hydrogen, -CH₃ or a C₂ alkyl group and/or o is 0, 1, 2, 3 or 4. Even more preferably R⁷ is hydrogen and o is 1 or 2. Most preferably o is 0.

A keto group is herein defined as a -[C(R⁷)₂]ₒC(O)R⁶ group, wherein R⁶ is an optionally substituted methyl group or an optionally substituted C₂ - C₂₄ alkyl group, R⁷ is independently selected from the group consisting of hydrogen, halogen, methyl and R⁶, and o is 0 - 24, preferably 0 - 10, and more preferably 0, 1, 2, 3, 4, 5 or 6. Preferably, R⁷ is hydrogen. In a preferred embodiment, R⁶ is an optionally substituted C₂ - C₂₄ alkyl group. When Su(A)ₓ is derived from an amino sugar, A is a keto group bonded to the amino sugar N-atom and o is 0 (i.e. when Su(A) comprises an-NC(O)R⁶ substituent), R⁶ is an optionally substituted C₂ - C₂₄ alkyl group.

An alkynyl group is preferably a terminal alkynyl group or a (hetero)cycloalkynyl group as defined above. In one embodiment the alkynyl group is a -[C(R⁷)₂]ₒC≡C-R⁷ group, wherein R⁷ and o are as defined above; R⁷ is preferably hydrogen. More preferably, o is 0, 1, 2, 3, 4, 5 or 6 and R⁷ is hydrogen. Most preferably o is 0.

A thiol group is herein defined as a -[C(R⁷)₂]ₒSH group, wherein R⁷ is independently selected from the group consisting of hydrogen, halogen and an (optionally substituted) C₁ - C₂₄ alkyl group, and o is 0 - 24. Preferably R⁷ is hydrogen or a C₁, C₂, C₃ or C₄ alkyl group, more preferably R⁷ is hydrogen or -CH₃. Preferably o is 0 - 10, more preferably 0, 1, 2, 3, 4, 5 or 6. More preferably, R⁷ is hydrogen, -CH₃ or a C₂ alkyl group and/or o is 0, 1, 2, 3 or 4. Even more preferably R⁷ is hydrogen and o is 0, 1, 2 or 3, more preferably o is 0, 1 or 2, most preferably o is 0. In a particularly preferred embodiment, R⁷ is hydrogen and o is 0. In another particularly preferred embodiment, R⁷ is hydrogen and o is 1. In another particularly preferred embodiment, R⁷ is hydrogen and o is 2. In another particularly preferred embodiment, R⁷ is hydrogen and o is 3.

A precursor of a thiol group is herein defined as a -[C(R⁷)₂]ₒSC(O)CH₃ group, wherein R⁷ and o, as well as their preferred embodiments, are as defined above for a thiol group. In a particularly preferred embodiment, R⁷ is hydrogen and o is 0. In another particularly preferred embodiment, R⁷ is hydrogen and o is 1. In another particularly preferred embodiment, R⁷ is hydrogen and o is 2. In another particularly preferred embodiment, R⁷ is hydrogen and o is 3. Most preferably, said thiol-precursor is -SC(O)CH₃ (-SAc). In step (4) of the process for the preparation of an antibody-conjugate according to the invention, a sugar derivative Su(A)ₓ wherein A is a precursor of a thiol group may be used. During said process, the thiol-precursor is converted to a thiol group.

A halogen is herein defined as F, Cl, Br or I. Preferably, said halogen is Cl, Br or I, more preferably Cl.

A sulfonyloxy group is herein defined as a -[C(R⁷)₂]ₒOS(O)₂R⁸ group, wherein R⁷ and o are as defined above for a thiol group, and R⁸ is selected from the group consisting of C₁ - C₂₄ alkyl groups, C₇ - C₂₄ alkylaryl groups and C₇ - C₂₄ arylalkyl groups. R⁸ is preferably a C₁ - C₄ alkyl group, C₇ - C₁₂ alkylaryl group or a C₇ - C₁₂ arylalkyl group, more preferably -CH₃, -C₂H₅, a C₃ linear or branched alkyl group or a C₇ alkylaryl group. R⁸ may also be a C₁ - C₂₄ aryl group, preferably a phenyl group. R⁸ is most preferably a methyl group, an ethyl group, a phenyl group or a p-tolyl group. Preferably R⁷ is hydrogen or a C₁, C₂, C₃ or C₄ alkyl group, more preferably R⁷ is hydrogen or -CH₃. Preferably o is 0 - 10, more preferably 0, 1, 2, 3, 4, 5 or 6. More preferably, R⁷ is hydrogen, -CH₃ or a C₂ alkyl group and/or o is 0, 1, 2, 3 or 4. Even more preferably R⁷ is hydrogen and o is 1 or 2, most preferably o is 0. R⁸ is preferably a C₁ - C₄ alkyl group, a C₇ - C₁₂ alkylaryl group or a C₇ - C₁₂ arylalkyl group, more preferably -CH₃, -C₂H₅, a C₃ linear or branched alkyl group or a C₇ alkylaryl group. It is also preferred that R⁸ is a phenyl group. Most preferably the sulfonyloxy group is a mesylate group, -OS(O)₂CH₃, a benzenesulfonate group (-OS(O)₂(C₆H₅)) or a tosylate group (-OS(O)₂(C₆H₄CH₃)).

A halogenated acetamido group is herein defined as an -NHC(O)[C(R⁷)₂]ₒX group, wherein R⁷ is independently selected from the group consisting of hydrogen, halogen and an (optionally substituted) C₁ - C₂₄ alkyl group, X is F, Cl, Br or I, and o is 0 - 24. Preferably R⁷ is hydrogen or a C₁, C₂, C₃ or C₄ alkyl group, more preferably R⁷ is hydrogen or -CH₃, most preferably hydrogen. Preferably o is 0 to 10, more preferably 1, 2, 3, 4, 5 or 6, even more preferably 1, 2, 3 or 4 and most preferably o is 1. More preferably, R⁷ is hydrogen, -CH₃ or a C₂ alkyl group and/or o is 1, 2, 3 or 4 and most preferably R⁷ is hydrogen and o is 1. Preferably, X is Cl or Br, more preferably X is Cl. Most preferably, R⁷ is hydrogen, X is Cl and o is 1.

A mercaptoacetamido group is herein defined as an -NHC(O)[C(R⁷)₂]ₒSH group, wherein R⁷ is independently selected from the group consisting of hydrogen, halogen and an (optionally substituted) C₁ - C₂₄ alkyl group and o is 0 - 24. Preferably R⁷ is hydrogen or a C₁, C₂, C₃ or C₄ alkyl group, more preferably R⁷ is hydrogen or -CH₃, most preferably hydrogen. Preferably o is 0 to 10, more preferably 1, 2, 3, 4, 5 or 6, even more preferably 1, 2, 3 or 4 and most preferably o is 2, 3 or 4. More preferably, R⁷ is hydrogen, -CH₃ or a C₂ alkyl group and/or o is 1, 2, 3 or 4. Most preferably, R⁷ is hydrogen and o is 1. Preferred examples include a mercaptoethanoylamido group, a mercaptopropanoylamido group, a mercaptobutanoylamido group and a mercapto-pentanoylamido group, preferably a mercaptopropanoylamido group.

A sulfonated hydroxyacetamido group is herein defined as a -NHC(O)[C(R⁷)₂] ₒOS(O)₂R⁸ group, wherein R⁷ is independently selected from the group consisting of hydrogen, halogen and an (optionally substituted) C₁ - C₂₄ alkyl group, R⁸ is selected from the group consisting of C₁ - C₂₄ alkyl groups, C₆ - C₂₄ aryl groups, C₇ - C₂₄ alkylaryl groups and C₇ - C₂₄ arylalkyl groups, and o is 0 - 24. R⁸ is preferably a C₁ - C₄ alkyl group, a C₆ - C₁₂ aryl group, a C₇ - C₁₂ alkylaryl group or a C₇ - C₁₂ arylalkyl group, more preferably -CH₃, -C₂H₅, a C₃ linear or branched alkyl group, a C₆ - C₉ aryl group or a C₇ alkylaryl group. Most preferably the sulfonyloxy group is a mesylate group -OS(O)₂CH₃, a benzenesulfonate group -OS(O)₂(C₆H₅) or a tosylate group -OS(O)₂(C₆H₄CH₃). Preferably R⁷ is hydrogen or a C₁, C₂, C₃ or C₄ alkyl group, more preferably R⁷ is hydrogen or -CH₃, most preferably hydrogen. Preferably o is 0 to 10, more preferably 1, 2, 3, 4, 5 or 6, even more preferably 1, 2, 3 or 4 and most preferably o is 1. More preferably, R⁷ is hydrogen, -CH₃ or a C₂ alkyl group and/or o is 1, 2, 3 or 4. Even more preferably R⁷ is hydrogen and o is 1, 2 or 3. Yet even more preferably, R⁷ is H, o is 1 and R⁸ is a mesylate group, a benzenesulfonate group or a tosylate group. Most preferably, R⁷ is hydrogen, R⁸ is -CH₃ and o is 1.

The sugar derivative Su(A)ₓ comprises one or more functional groups A. When Su(A)ₓ comprises two or more functional groups A, each functional group A is independently selected, i.e. one Su(A)ₓ may comprise different functional groups A, e.g. an azido group and a keto group, etc. In a preferred embodiment, x is 1 or 2, more preferably x is 1. In another preferred embodiment, functional group A is an azido group or a keto group, more preferably an azido group. In another preferred embodiment, functional group A is a thiol group or a halogen, more preferably a halogen. In a further preferred embodiment, x is 1 and A is an azido group, a keto group, a thiol group or a halogen.

Sugar derivative Su(A)ₓ is derived from a sugar or a sugar derivative Su, e.g. an amino sugar or an otherwise derivatized sugar. Examples of sugars and sugar derivatives include galactose (Gal), mannose (Man), glucose (Glc), N-acetylneuraminic acid or sialic acid (Sial) and fucose (Fuc).

An amino sugar is a sugar wherein a hydroxyl (OH) group is replaced by an amine group and examples include glucosamine (GlcNH₂) and galactosamine (GalNH₂). Examples of an otherwise derivatized sugar include N-acetylneuraminic acid (sialic acid, Sia or NeuNAc) or fucose (Fuc).

Sugar derivative Su(A)ₓ is preferably derived from galactose (Gal), mannose (Man), N-acetylglucosamine (GlcNAc), glucose (Glc), N-acetylgalactosamine (GalNAc), fucose (Fuc) and N-acetylneuraminic acid (sialic acid Sia or NeuNAc), preferably from the group consisting of GlcNAc, Glc, Gal and GalNAc. More preferably Su(A)ₓ is derived from Gal or GalNAc, and most preferably Su(A)ₓ is derived from GalNAc.

The one or more functional groups A in Su(A)ₓ may be linked to the sugar or sugar derivative Su in several ways. The one or more functional groups A may be bonded to C2, C3, C4 and/or C6 of the sugar or sugar derivative, instead of a hydroxyl (OH) group. It should be noted that, since fucose lacks an OH-group on C6, if A is bonded to C6 of Fuc, then A takes the place of an H-atom.

In a preferred embodiment, the one or more functional groups A in Su(A)ₓ are present on C2 and/or C6 of the sugar or sugar derivative Su. When a functional group A is present instead of an OH-group on C2 of a sugar or sugar derivative, A is preferably selected from the group consisting of an azido group, a halogenated acetamido group, a mercaptoacetamido group and a sulfonylated hydroxyacetamido group. However, when A is present on C2 of a 2-aminosugar derivative, e.g. GalNAc or GlcNAc, A is preferably selected from the group consisting of an azido group, a halogen, a thiol group or a derivative thereof and a sulfonyloxy group, more preferably from the group consisting of an azido group, halogen, a thiol group and a sulfonyloxy group.

When A is an azido group, it is preferred that A is bonded to C2, C3, C4 or C6. As was described above, the one or more azide substituents in Su(A)ₓ may be bonded to C2, C3, C4 or C6 of the sugar or sugar derivative S, instead of a hydroxyl (OH) group or, in case of 6-azidofucose (6-AzFuc), instead of a hydrogen atom. Alternatively or additionally, the N-acetyl substituent of an amino sugar derivative may be substituted by an azidoacetyl substituent. In a preferred embodiment Su(A)ₓ is selected from the group consisting of 2-azidoacetamidogalactose (GalNAz), 6-azido-6-deoxygalactose (6-AzGal), 6-azido-6-deoxy-2-acetamidogalactose (6-AzGalNAc), 4-azido-4-deoxy-2-acetamidogalactose (4-AzGalNAc), 6-azido-6-deoxy-2-azidoacetamidogalactose (6-AzGalNAz), 2-azidoacetamidoglucose (GlcNAz), 6-azido-6-deoxyglucose (6-AzGlc), 6-azido-6-deoxy-2-acetamidoglucose (6-AzGlcNAc), 4-azido-4-deoxy-2-acetamidoglucose (4-AzGlcNAc) and 6-azido-6-deoxy-2-azidoacetamidoglucose (6-AzGlcNAz), more preferably from the group consisting of GalNAz, 6-AzGal, 4-AzGalNAc, GlcNAz and 6-AzGlcNAc. Examples of Su(A)ₓ-P wherein A is an azido group are shown below.

When A is a keto group, it is preferred that A is bonded to C2 instead of the OH-group of Su. Alternatively A may be bonded to the N-atom of an amino sugar derivative, preferably a 2-amino sugar derivative. The sugar derivative then comprises an -NC(O)R⁶ substituent. R⁶ is preferably a C₂ - C₂₄ alkyl group, optionally substituted. More preferably, R⁶ is an ethyl group. In a preferred embodiment Su(A)ₓ is selected from the group consisting of 2-deoxy-(2-oxopropyl)galactose (2-ketoGal), 2-N-propionylgalactosamine (2-N-propionylGalNAc), 2-N-(4-oxopentanoyl)galactosamine (2-N-LevGal) and 2-N-butyrylgalactosamine (2-N-butyrylGalNAc), more preferably 2-ketoGalNAc and 2-N-propionylGalNAc. Examples of Su(A)ₓ-P wherein A is a keto group are shown below.

When A is an alkynyl group, preferably a terminal alkynyl group or a (hetero)cycloalkynyl group, it is preferred that said alkynyl group is present on a 2-amino sugar derivative. An example of Su(A)x wherein A is an alkynyl group is 2-(but-3-yonic acid amido)-2-deoxy-galactose. An example of Su(A)ₓ-P wherein A is an alkynyl group is shown below.

When A is a thiol group, it is preferred that said thiol group is present on the 6-position of a sugar derivative or on a 2-amino sugar derivative. An example of Su(A)ₓ wherein A is a thiol group is 2-(mercaptoacetamido)-2-deoxy-galactose. Another example of Su(A)ₓ wherein A is a thiol group is 6-mercapto-6-deoxy-galactose.

When A is a halogen, it is preferred that said halogen is present on the 6-position of a sugar derivative or on a 2-amino sugar derivative. An example of Su(A)ₓ wherein A is a halogen is 2-(chloroacetamido)-2-deoxy-galactose. Another example of Su(A)ₓ wherein A is a halogen is 6-iodo-6-deoxy-galactose. Another example of Su(A)ₓ wherein A is a halogen is 6-(chloroacetamido)-6-deoxy-galactose.

When A is a sulfonyloxy group, it is preferred that said sulfonyloxy group is present on the 6-position of a sugar derivative or on a 2-amino sugar derivative. An example of Su(A)ₓ wherein A is a sulfonyloxy group is 2-(methylsulfonyloxyacetamido)-2-deoxy-galactose (2-GalNAcOMs). Another example of SuAₓ wherein A is a sulfonyloxy group is 2-(benzenesulfonyloxyacetamido)-2-deoxy-galactose (2-GalNAcOMs. Another example of Su(A)ₓ wherein A is a sulfonyloxy group is 6-(methylsulfonyl)-galactose.

When A is a halogenated acetamido group, a mercaptoacetamido group or a sulfonylated hydroxyacetamido group it is preferred that said groups are present on the 6-position of a sugar derivative.

P is herein defined as a nucleotide. P is preferably selected from the group consisting of a nucleoside monophosphate and a nucleoside diphosphate, more preferably from the group consisting of uridine diphosphate (UDP), guanosine diphosphate (GDP), thymidine diphosphate (TDP), cytidine diphosphate (CDP) and cytidine monophosphate (CMP), more preferably from the group consisting of uridine diphosphate (UDP), guanosine diphosphate (GDP), cytidine diphosphate and (CDP). Most preferably, P is UDP.

Several compounds of the formula Su(A)ₓ-P, wherein a nucleoside monophosphate or a nucleoside diphosphate P is linked to a sugar derivative Su(A)ₓ, are known in the art. For example Wang et al., Chem. Eur. J. 2010, 16, 13343-13345, Piller et al., ACS Chem. Biol. 2012, 7, 753, Piller et al., Bioorg. Med. Chem. Lett. 2005, 15, 5459-5462 and WO 2009/102820, disclose a number of compounds Su(A)ₓ-P and their syntheses.

Several examples **(9** - **11)** and **(12** - **27)** of azido-, keto-, alkynyl-, halogen, thiol, thiolated acetamid- and halogenated acetamido-substitued sugars and sugar derivatives are shown below, all of which may be converted into their corresponding UDP sugars Su(A)ₓ-UDP (**9b - 11b**) and (**12b - 27b**).

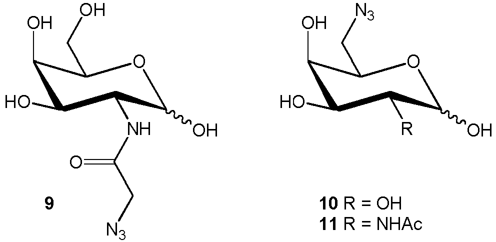

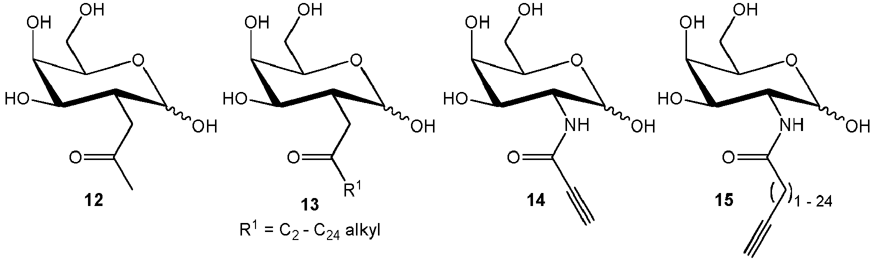

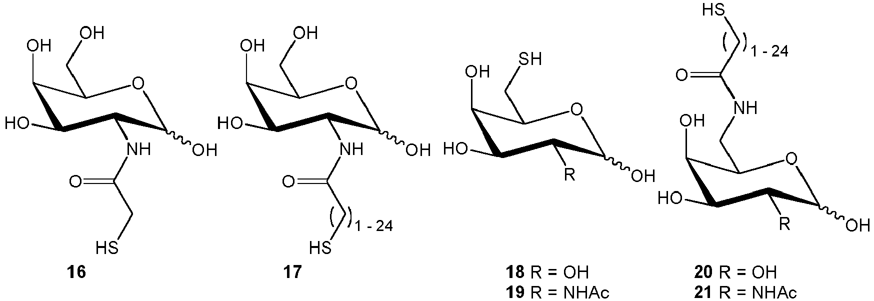

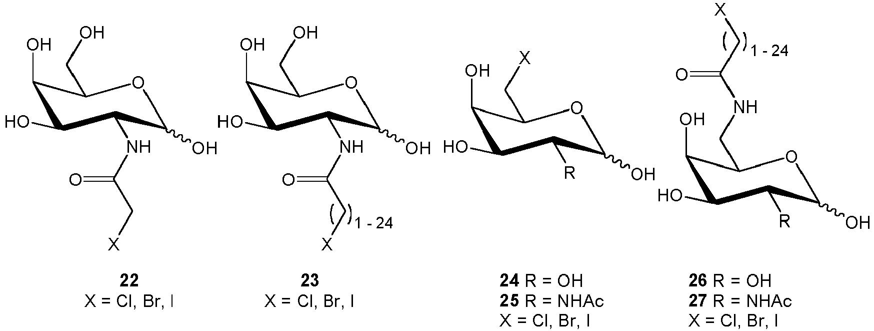

Preferably, Su(A)ₓ-P is selected from the group consisting of GalNAz-UDP (**9b**), 6-AzGal-UDP (**10b**), 6-AzGalNAc-UDP (**11b**), 4-AzGalNAz-UDP, 6-AzGalNAz-UDP, 6-AzGlc-UDP, 6-AzGlcNAz-UDP, 2-ketoGal-UDP (**12b**), 2-N-propionylGalNAc-UDP (**13b**, wherein R¹ is ethyl) and 2-(buty-3-ynoic acid amido)-2-deoxy-galactose-UDP (**15b**, with n=1). More preferably, Su(A)ₓ-P is GalNAz-UDP (**9b**) or 6-AzGalNAc-UDP (**11b**).

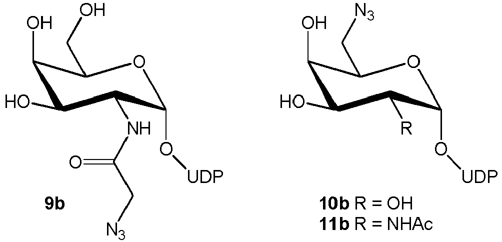

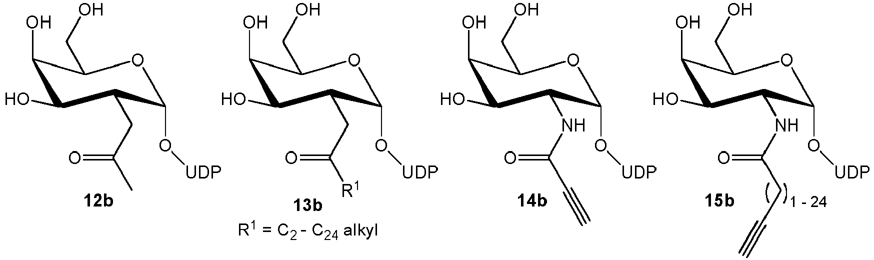

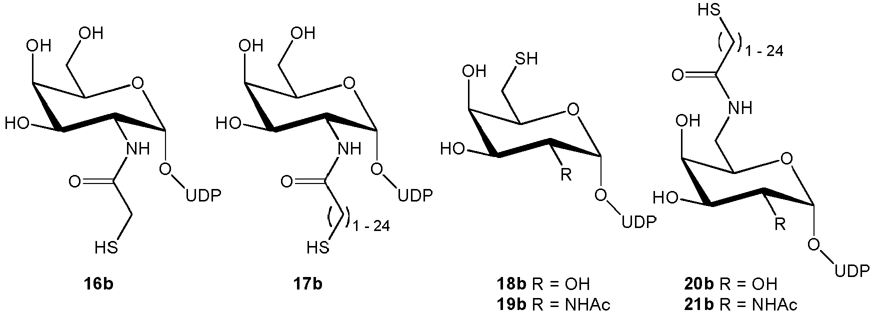

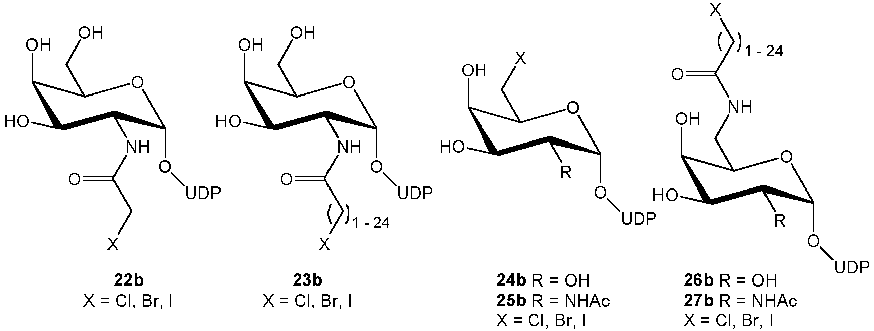

Preferably, Su(A)ₓ-P is GalNAz-UDP (**9b**) or 6-AzGalNAc-UDP (**11b**). The syntheses of GalNAz-UDP (**9b**) and 6-AzGalNAc-UDP (**11b**) are disclosed in Piller et al., Bioorg. Med. Chem. Lett. 2005, 15, 5459-5462 and Wang et al., Chem. Eur. J. 2010, 16, 13343-13345.

The synthesis of 2-ketoGal-UDP (**12b**) is disclosed in Qasba et al., J. Am. Chem. Soc. 2003, 125, 16162, in particular in the Supporting Information.

The synthesis of 2-(but-3-yonic acid amido)-2-deoxy-galactose-UDP (**15b**) is disclosed in WO 2009/102820.

Further examples of Su(A)ₓ-P include 6-A-6-deoxygalactose-UDP (6-A-Gal-UDP), such as 6-chloro-6-deoxygalactose-UDP (6-ClGal-UDP, (**24b**) with X is Cl), 6-thio-6-deoxygalactose-UDP (6-HSGal-UDP, (**18b**)) or 2-A-2-deoxygalactose-UDP (2-A-Gal-UDP), such as 2-chloro-2-deoxygalactose-UDP (2-ClGal-UDP), 2-thio-2-deoxygalactose-UDP (2-HSGal-UDP). Alternatively, A may be indirectly substituted to the sugar derivative as part of an acetamido group that in turn is substituting a hydroxyl group. Examples include 6-A-acetamido-6-deoxygalactose-UDP (6-GalNAcA-UDP), such as 6-chloroacetamido-6-deoxygalactose-UDP (6-GalNAcCl-UDP, (**26b**) with X is Cl), 6-thioacetamido-6-deoxygalactose-UDP (6-GalNAcSH-UDP, (**20b**)) or 2-A-acetamido-2-deoxygalactose-UDP (2-GalNAcA-UDP), such as 2-chloroacetamido-2-deoxygalactose-UDP (2-GalNAcCl-UDP, (**22b**) with X is Cl), 2-thioacetamido-2-deoxygalactose-UDP (2-GalNAcSH-UDP, (**16b**)) and 2-acetylthioacetamido-2-deoxygalactose-UDP (2-GalNAcSAc-UDP). Further examples include 3-thiopropanoylamido-2-deoxygalactose-UDP (2-GalNProSH-UDP) and 4-thiobutanolylamido-2-deoxygalactose-UDP (2-GalNBuSH-UDP). Alternatively, A may be indirectly substituted to the sugar derivative as part of another functional group that in turn is substituting a hydroxyl group or is attached to a hydroxyl group. Examples of such other functional group include an (hetero)alkyl chain or a (hetero)aryl chain.

Preferably, Su(A)ₓ-P is selected from the group consisting of GalNAz-UDP (**9b**), 6-AzGalNAc-UDP (**11b**), 6-GalNAcCl-UDP ((**26b**) with X is Cl), 6-GalNAcSH-UDP (**20b**), 2-GalNAcCl-UDP ((**22b**) with X is Cl), 2-GalNAcSH-UDP (**16b**), 6-ClGal-UDP ((**24b**) with X is Cl), 2-ClGal-UDP, 2-HSGal-UDP and 6-HSGal-UDP (**18b**), or from the group consisting of 2-GalNProSH-UDP and 2-GalNBuSH-UDP.

More preferably, Su(A)ₓ-P is selected from the group consisting of GalNAz-UDP (**9b**), 6-AzGalNAc-UDP (**11b**), 6-GalNAcCl-UDP ((**26b**) with X is Cl), 6-GalNAcSH-UDP (**20b**), 2-GalNAcCl-UDP ((**22b**) with X is Cl), 2-GalNAcSH-UDP (**16b)**, 6-ClGal-UDP ((**24b**) with X is Cl) and 2-ClGal-UDP, or from the group consisting of 2-GalNProSH-UDP and 2-GalNBuSH-UDP.

In a preferred embodiment of the process for the preparation of a modified antibody, Su(A)ₓ comprises 1 or 2 functional groups A, i.e. preferably x is 1 or 2. More preferably, x is 1. In another preferred embodiment, Su is galactose (Gal). In a further preferred embodiment, x is 1 or 2 and Su is Gal, and most preferably, x is 1 and Su is Gal.

In a preferred embodiment, Su(A)ₓ is selected from the group consisting of GalNAz, 6-AzGalNAc, 6-GalNAcCl, 6-GalNAcSH, 2-GalNAcCl, 2-GalNAcSH, 6-ClGal, 2-ClGal, 2-HSGal and 6-HSGal, more preferably form the group consisting of GalNAz, 6-AzGalNAc, 6-GalNAcCl, 6-GalNAcSH, 2-GalNAcCl, 2-GalNAcSH, 6-ClGal-and 2-ClGal. In another preferred embodiment, Su(A)ₓ is selected from the group consisting of 2-GalNProSH and 2-GalNBuSH,

In a further preferred embodiment, x is 1 and Su(A)ₓ is selected from the group consisting of GalNAz, 6-AzGalNAc, 6-GalNAcCl, 6-GalNAcSH, 2-GalNAcCl, 2-GalNAcSH, 6-ClGal, 2-ClGal, 2-HSGal and 6-HSGal, more preferably form the group consisting of GalNAz, 6-AzGalNAc, 6-GalNAcCl, 6-GalNAcSH, 2-GalNAcCl, 2-GalNAcSH, 6-ClGal-and 2-ClGal. In another further preferred embodiment, x is 1 and Su(A)ₓ is selected from the group consisting of 2-GalNProSH and 2-GalNBuSH.

In a preferred embodiment, A is an azide group, a thiol group or a halogen.

In a preferred embodiment wherein A is an azide group, Su(A)ₓ is preferably selected from the group consisting of 2-azidoacetamidogalactose (GalNAz), 6-azido-6-deoxygalactose (6-AzGal), 6-azido-6-deoxy-2-acetamidogalactose (6-AzGalNAc), 4-azido-4-deoxy-2-acetamidogalactose (4-AzGalNAc), 6-azido-6-deoxy-2-azidoacetamidogalactose (6-AzGalNAz), 2-azidoacetamidoglucose (GlcNAz), 6-azido-6-deoxyglucose (6-AzGlc), 6-azido-6-deoxy-2-acetamidoglucose (6-AzGlcNAc), 4-azido-4-deoxy-2-acetamidoglucose (4-AzGlcNAc) and 6-azido-6-deoxy-2-azidoacetamidoglucose (6-AzGlcNAz). In a further preferred embodiment Su(A)ₓ is selected from the group consisting of GalNAz, 6-AzGal, 4-AzGalNAc, GlcNAz and 6-AzGlcNAc. More preferably, x is 1 and Su(A)ₓ is selected from the group consisting of 2-azidoacetamidogalactose (GalNAz), 6-azido-6-deoxygalactose (6-AzGal), 6-azido-6-deoxy-2-acetamidogalactose (6-AzGalNAc), 4-azido-4-deoxy-2-acetamidogalactose (4-AzGalNAc), 6-azido-6-deoxy-2-azidoacetamidogalactose (6-AzGalNAz), 2-azidoacetamidoglucose (GlcNAz), 6-azido-6-deoxyglucose (6-AzGlc), 6-azido-6-deoxy-2-acetamidoglucose (6-AzGlcNAc), 4-azido-4-deoxy-2-acetamidoglucose (4-AzGlcNAc) and 6-azido-6-deoxy-2-azidoacetamidoglucose (6-AzGlcNAz). More preferably, x is 1 and Su(A)ₓ is selected from the group consisting of GalNAz, 6-AzGal, 4-AzGalNAc, GlcNAz and 6-AzGlcNAc.

In a particularly preferred embodiment of the process for the modification of a modified antibody according to the invention, Su(A)ₓ is selected from the group consisting of GalNAz, 6-AzGalNAc, 2-GalNAcSH, 2-GalNAcX, 2-GalNAcOS(O)₂R⁸, 6-GalNAcSH, 6-GalNAcX and 6-GalNAcOS(O)₂R⁸. In another particularly preferred embodiment of the process for the modification of a modified antibody according to the invention, Su(A)ₓ is selected from the group consisting of 2-GalNProSH and 2-GalNBuSH.

In an even more preferred embodiment, x is 1 or 2 and Su(A)ₓ is selected from the group consisting of GalNAz, 6-AzGalNAc, 2-GalNAcSH, 2-GalNAcX, 2-GalNAcOS(O)₂R⁸, 6-GalNAcSH, 6-GalNAcX and 6-GalNAcOS(O)₂R⁸. In another even more preferred embodiment, x is 1 or 2 and Su(A)ₓ is selected from the group consisting of 2-GalNProSH and 2-GalNBuSH.

In a most preferred embodiment, x is 1 and Su(A)ₓ is selected from the group consisting of GalNAz, 6-AzGalNAc, 2-GalNAcSH, 2-GalNAcX, 2-GalNAcOS(O)₂R⁸, 6-GalNAcSH, 6-GalNAcX and 6-GalNAcOS(O)₂R⁸. In another most preferred embodiment, x is 1 and Su(A)ₓ is selected from the group consisting of 2-GalNProSH and 2-GalNBuSH.

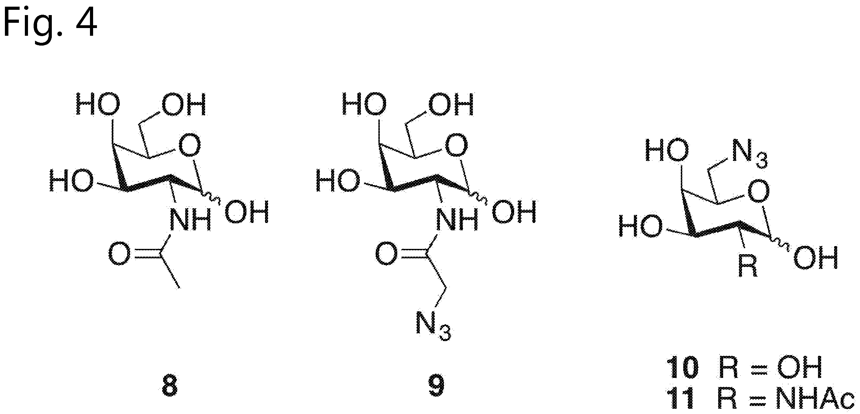

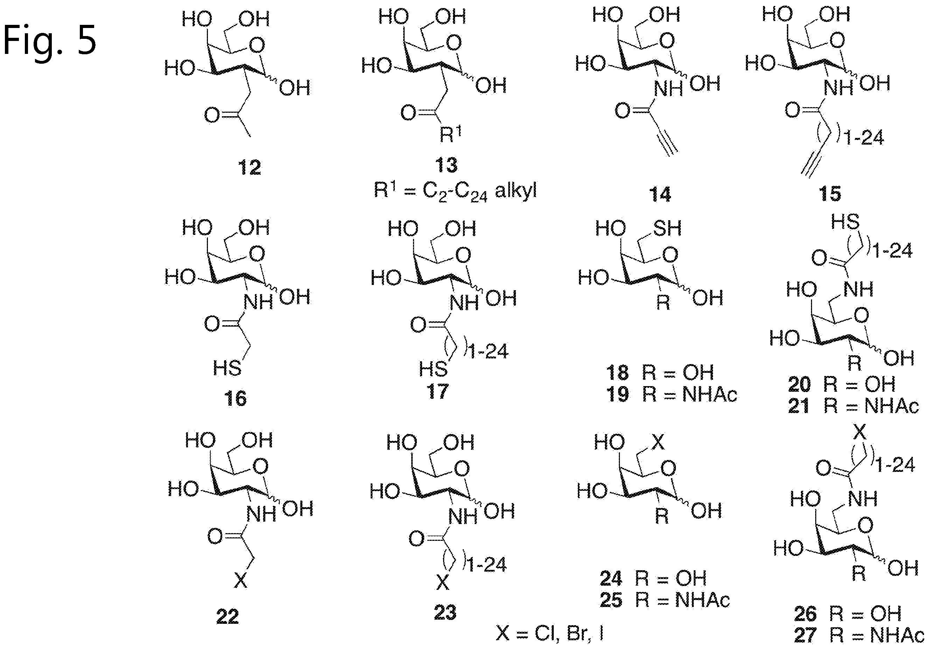

Additional examples of sugars and sugar derivatives are shown in Figures 4 and 5. Figure 4 shows the structures of azido-modified galactose derivatives (9-11), for which the corresponding UDP sugars may be used for transfer onto a GlcNAc-terminated sugar under the action of a galactosyl transferase (or a mutant thereof). Figure 5 shows the structures of other galactose derivatives (**12**-**27**), for which the corresponding UDP sugars may be used for transfer onto a GlcNAc-terminated sugar under the action of a galactosyl transferase (or a mutant thereof).

Figure 7 shows the structures of modified UDP-galactose nucleotides **28-31** for enzymatic incorporation onto GlcNAc-terminated glycans upon the action of a galacto syltransferase.

Several of the sugar derivative nucleotides Su(A)ₓ-P that may be employed in the process for the preparation of a modified antibody according to the invention are a substrate for a wild type galactosyltransferase. For these sugar derivative nucleotides Su(A)ₓ-P, the process according to the invention may be performed in the presence of a wild type galactosyltransferase, preferably a wild type *β*(1,4)-galactosyltransferase, more preferably a wild type *β*(1,4)-galactosyltransferase I, as a catalyst. More preferably, the wild-type a *β*(1,4)-galactosyltransferase is a wild-type human GalT, more preferably a wild-type human GalT selected from the group consisting of a wild-type human *β*4-Gal-T1, a wild-type human *β*(1,4)-Gal-T2, a wild-type human *β*(1,4)-Gal-T3 and a wild-type human *β*(1,4)-Gal-T4.

When a wild type galactosyltransferase is used as a catalyst, it is preferred that Su(A)ₓ-P is selected from the group consisting of Su(A)ₓ-P wherein x is 1 and wherein A is present on C2 or C6, more preferably C6, of the sugar derivative, and wherein A is selected from the group consisting of an azido group, a keto group, an alkynyl group, a thiol group or a precursor thereof, a halogen, a sulfonyloxy group, a halogenated acetamido group, a mercaptoacetamido group and a sulfonylated hydroxyacetamido group. A may be directly substituted to the sugar derivative instead of an hydroxyl group. Examples include 6-A-6-deoxygalactose-UDP (6-A-Gal-UDP), such as 6-azido-6-deoxygalactose-UDP (6-AzGal-UDP, (**10b**)), 6-chloro-6-deoxygalactose-UDP (6-ClGal-UDP, (**24b**) with X is Cl), 6-thio-6-deoxygalactose-UDP (6-HSGal-UDP, (**18b**)) or 2-A-2-deoxygalactose-UDP (2-A-Gal-UDP), such as 2-azido-2-deoxygalactose-UDP (2-AzGal-UDP), 2-chloro-2-deoxygalactose-UDP (2-ClGal-UDP), 2-thio-2-deoxygalactose-UDP (2-HSGal-UDP). Alternatively, A may be indirectly substituted to the sugar derivative as part of an acetamido group that in turn is substituting a hydroxyl group. Examples include 6-A-acetamido-6-deoxygalactose-UDP (6-GalNAcA-UDP), such as 6-azidoacetamido-6-deoxygalactose-UDP (6-GalNAcN₃-UDP), 6-chloroacetamido-6-deoxygalactose-UDP (6-GalNAcCl-UDP, (**26b**) with X is Cl), 6-thioacetamido-6-deoxygalactose-UDP (6-GalNAcSH-UDP, (**20b**)) or 2-A-acetamido-2-deoxygalactose-UDP (2-GalNAcA-UDP), such as 2-azidoacetamido-2-deoxygalactose-UDP (2-GalNAcN₃-UDP, (**9b**)), 2-chloroacetamido-2-deoxygalactose-UDP (2-GalNAcCl-UDP, (**22b**)), 2-thioacetamido-2-deoxygalactose-UDP (2-GalNAcSH-UDP, (**16b**)). Alternatively, A may be indirectly substituted to the sugar derivative as part of another functional group that in turn is substituting a hydroxyl group or is attached to a hydroxyl group. Examples of such other functional group include an (hetero)alkyl chain or a (hetero)aryl chain.

In a particularly preferred embodiment of the process for the preparation of a modified antibody according to the invention, Su(A)ₓ-P is selected from the group consisting of GalNAz-UDP (**9b**), 6-AzGalNAc-UDP (**11b**), 2-GalNAcSH-UDP (**16b**), 2-GalNAcX-UDP (**22b**), 2-GalNAcOS(O)₂R⁸-UDP, 6-GalNAcSH-UDP (**20b**), 6-GalNAcX-UDP (**26b**) and 6-GalNAcOS(O)₂R⁸-UDP, and the catalyst is bovine β(1,4)-Gal-T1 comprising a mutant catalytic domain GalT (Y289L); wherein X is Cl, Br or I; and wherein R⁸ is a methyl group, an ethyl group, a phenyl group or a p-tolyl group. In another particularly preferred embodiment of the process for the preparation of a modified antibody according to the invention, Su(A)ₓ-P is selected from the group consisting of 2-GalNProSH-UDP and 2-GalNBuSH-UDP, and the catalyst is bovine β(1,4)-Gal-T1 comprising a mutant catalytic domain GalT (Y289L); wherein X is Cl, Br or I; and wherein R⁸ is a methyl group, an ethyl group, a phenyl group or a p-tolyl group.

In a further preferred embodiment 2-GalNAcX-UDP is 2-GalNAcCl-UDP or 2-GalNAcBr-UDP, more preferably 2-GalNAcCl-UDP, and 6-GalNAcX-UDP is 6-GalNAcCl-UDP or 6-GalNAcBr-UDP, more preferably 6-GalNAcCl-UDP. In another preferred embodiment, R⁸ in 2-GalNAcOS(O)₂R⁸-UDP is methyl, phenyl or p-tolyl, most preferably methyl, and R⁸ in 6-GalNAcOS(O)₂R⁸-UDP is methyl, phenyl or p-tolyl, most preferably R⁸ is methyl.

In another particularly preferred embodiment of the process for the preparation of a modified antibody according to the invention, Su(A)ₓ-P is selected from the group consisting of 6-AzGalNAc-UDP (**11b**), 6-HSGal-UDP (**18b**), 6-XGal-UDP (**24b**), 6-R⁸S(O)₂OGal-UDP, and the catalyst is a wild-type human GalT; wherein X is Cl, Br or I; and wherein R⁸ is a methyl group, an ethyl group, a phenyl group or a p-tolyl group. X is more preferably Cl or Br, most preferably Cl. R⁸ is more preferably methyl, phenyl or p-tolyl, most preferably methyl. The human GalT is preferably a human *β*4-Gal-T1, a human *β*(1,4)-Gal-T2, a human *β*(1,4)-Gal-T3 and a human *β*(1,4)-Gal-T4.

As was described above, in the process for the modification of a antibody according to the invention, Su(A)ₓ-P may be any sugar derivative nucleotide that is a substrate for a suitable galactosyltransferase catalyst.

The sugar derivative Su(A)ₓ in the proximal N-linked Su(A)ₓGlcNAc-substituent attached to an N-glycosylation site of the IgG antibody may for example be bonded to C4 of the GlcNAc-moiety via a *β*(1,4)-glycosidic bond or to C3 of said GlcNAc-moiety via an *α*(1,3)-glycosidic bond. The proximal N-linked GlcNAc-residue of the Su(A)ₓGlcNAc-substituent is bonded via C1 to the protein or antibody via an N-glycosidic bond, preferably to the amide nitrogen atom in the side chain of an asparagine amino acid of the protein or antibody. The proximal N-linked GlcNAc-residue in said Su(A)ₓGlcNAc-substituent is optionally fucosylated. Whether the sugar derivative Su(A)ₓ in the Su(A)ₓGlcNAc-moiety attached to the antibody is bonded to C4 of said GlcNAc-residue via a *β*(1,4)-glycosidic bond or to C3 of said GlcNAc-moiety via an *α*(1,3)-glycosidic bond depends on the catalyst that was used in step (4) and/or step (5b) of the process according to the disclosure. When said step is performed in the presence of a *β*(1,4)-galactosyltransferase then binding occurs via C1 of Su(A)ₓ and C4 of the proximal GlcNAc-residue via a *β*(1,4)-glycosidic bond. When the process is performed in the presence of a *α*(1,3)-galactosyltransferase then binding occurs via C1 of Su(A)ₓ and C3 of the proximal GlcNAc-residue via an *α*(1,3)-glycosidic bond.

When A is an azido functional group, the IgG antibody comprising a Su(A)ₓGlcNAc-residue that is obtainable by the process for the modification of an antibody according to the invention is referred to as an azide-modified antibody. When A is a keto functional group, the IgG antibody comprising a Su(A)ₓGlcNAc-residue is referred to as a keto-modified antibody. When A is an alkynyl functional group, the IgG antibody comprising a Su(A)ₓGlcNAc-residue is referred to as an alkyne-modified antibody. When A is a thiol group, the IgG antibody comprising a Su(A)ₓGlcNAc-residue is referred to as a thiol-modified antibody. When A is an halogen group, the IgG antibody comprising a Su(A)ₓGlcNAc-residue is referred to as a halogen-modified antibody. When A is an sulfonyloxy group, the IgG antibody comprising a Su(A)ₓGlcNAc-residue is referred to as a sulfonyloxy-modified antibody. When A is a mercaptoacetamido group, the IgG antibody comprising a Su(A)ₓGlcNAc-residue is referred to as a thiolated acetamido-modified antibody. When A is a halogenated acetamido group, the IgG antibody comprising a Su(A)ₓGlcNAc-residue is referred to as a halogenated acetamido-modified antibody. When A is a sulfonylated hydroxyacetamido group, the IgG antibody comprising a Su(A)ₓGlcNAc-residue is referred to as mercaptoacetamido-modified antibody.

The process for the preparation of a modified antibody, i.e. the process of attaching a monosaccharide derivative Su(A)ₓ to a proximal N-linked GlcNAc-residue, is preferably performed in a suitable buffer solution, such as for example phosphate, buffered saline (e.g. phosphate-buffered saline, tris-buffered saline), citrate, HEPES, tris and glycine. Suitable buffers are known in the art. Preferably, the buffer solution is phosphate-buffered saline (PBS) or tris buffer.

The process is preferably performed at a temperature in the range of about 4 to about 50°C, more preferably in the range of about 10 to about 45°C, even more preferably in the range of about 20 to about 40°C, and most preferably in the range of about 30 to about 37°C.

The process is preferably performed a pH in the range of about 5 to about 9, preferably in the range of about 5.5 to about 8.5, more preferably in the range of about 6 to about 8. Most preferably, the process is performed at a pH in the range of about 7 to about 8.

In one embodiment of the process for the preparation of a modified antibody according to the invention comprises the steps of:
(1) providing an IgG antibody comprising one N-linked glycosylation site on the combination of a single heavy chain and single light chain, wherein the N-linked glycosylation site is a mutant N-linked glycosylation site as compared to its wild type counterpart, and wherein a proximal N-linked GlcNAc-residue is attached to the antibody at said glycosylation site; and
(2) attaching a monosaccharide derivative Su(A)ₓ to a proximal N-linked GlcNAc-residue, in the presence of a suitable catalyst; wherein Su(A)ₓ is defined as a monosaccharide derivative Su comprising x functional groups A wherein x is 1, 2, 3 or 4 and wherein A is selected from the group consisting of an azido group, a keto group, an alkynyl group, a thiol group or a precursor thereof, a halogen, a sulfonyloxy group, a halogenated acetamido group, a mercaptoacetamido group and a sulfonylated hydroxyacetamido group; and wherein said proximal N-linked GlcNAc-residue is optionally fucosylated.

In a more specific embodiment of the process for the preparation of a modified antibody according to the invention, in step (1) the IgG antibody comprising one N-linked glycosylation sites on the combination of a single heavy chain and single light chain is provided by a site-specific mutagenesis process.

In another more specific embodiment, the process for the preparation of a modified antibody comprises the steps of:
(1) providing an IgG antibody comprising one N-linked glycosylation site on the combination of a single heavy chain and single light chain, wherein the N-linked glycosylation site is a mutant N-linked glycosylation site as compared to its wild type counterpart; and
(2) trimming the oligosaccharide that is attached to said glycosylation site, by the action of an endo-β-N-acetylglucosaminidase, in order to obtain a proximal N-linked GlcNAc-residue at said glycosylation site; and
(3) attaching a monosaccharide derivative Su(A)ₓ to a proximal N-linked GlcNAc-residue, in the presence of a suitable catalyst; wherein Su(A)ₓ is defined as a monosaccharide derivative Su comprising x functional groups A wherein x is 1, 2, 3 or 4 and wherein A is selected from the group consisting of an azido group, a keto group, an alkynyl group, a thiol group or a precursor thereof, a halogen, a sulfonyloxy group, a halogenated acetamido group, a mercaptoacetamido group and a sulfonylated hydroxyacetamido group;
wherein said proximal N-linked GlcNAc-residue in step (2) and (3) is optionally fucosylated.

In step (1), said IgG antibody may be provided by glycoengineering methods. For example, said IgG antibody may be provided by a process for the preparation of an IgG antibody comprising one, and no more than one, N-linked glycosylation site on the combination of a single heavy chain and single light chain, wherein the N-linked glycosylation site is a mutant N-linked glycosylation site as compared to its wild type counterpart, wherein:
(i) the amino acid chain of the heavy chain is altered to remove the N-glycosylation site at position 297, and wherein the altered amino acid chain of the heavy chain comprises one altered N-glycosylation site as compared to its wild type counterpart; or
(ii) the amino acid chain of the heavy chain is altered to remove the N-glycosylation site at position 297, and wherein the light chain comprises one altered N-glycosylation site as compared to its wild type counterpart.

In a preferred embodiment of steps (i) and (ii), the asparagine at position 297 of the heavy chain is altered to glutamine. Therefore, in this preferred embodiment, the heavy chain comprises the N297Q mutation. In an IgG antibody comprising the N297Q mutation, the conserved N-glycosylation site at N297 is removed.

Therefore, in a preferred embodiment of the process for the preparation of said IgG antibody, in step (1):
(i) the amino acid chain of the heavy chain is altered to remove the N-glycosylation site at position 297, and the altered amino acid chain of the heavy chain comprises one altered N-glycosylation site as compared to its wild type counterpart; or
(ii) the amino acid chain of the heavy chain is altered to remove the N-glycosylation site at position 297, and the light chain comprises one altered N-glycosylation site as compared to its wild type counterpart.

In another preferred embodiment of this process, the heavy chain in step (i) or the light chain in step (ii) comprises one newly introduced glycosylation site. In a further preferred embodiment, the amino acid sequence of the heavy chain in step (i) or the amino acid sequence of the light chain in step (ii) comprises the consensus sequence Asn-X-Ser/Thr, wherein X is any amino acid except Pro. Introduction of said consensus sequence into the amino acid sequence, preferably results in the presence of an N-glycosylation site at the asparagine amino acid of said sequence.

Therefore, in a preferred embodiment of the process for the preparation of said IgG antibody, in step (1):
(i) the amino acid chain of the heavy chain is altered to remove the N-glycosylation site at position 297, and the altered amino acid chain of the heavy chain comprises the consensus sequence Asn-X-Ser/Thr, wherein X is any amino acid except Pro; or
(ii) the amino acid chain of the heavy chain is altered to remove the N-glycosylation site at position 297, and the amino acid of the light chain comprises the consensus sequence Asn-X-Ser/Thr, wherein X is any amino acid except Pro.

Step (2) of this particular embodiment of the process for the preparation of a modified antibody according to the invention comprises the trimming of an oligosaccharide that is attached to a glycosylation site, by the action of a suitable enzyme, in order to obtain a proximal N-linked GlcNAc-residue at said glycosylation site, wherein a suitable enzyme is defined as an enzyme wherefore the oligosaccharide that is to be trimmed is a substrate.

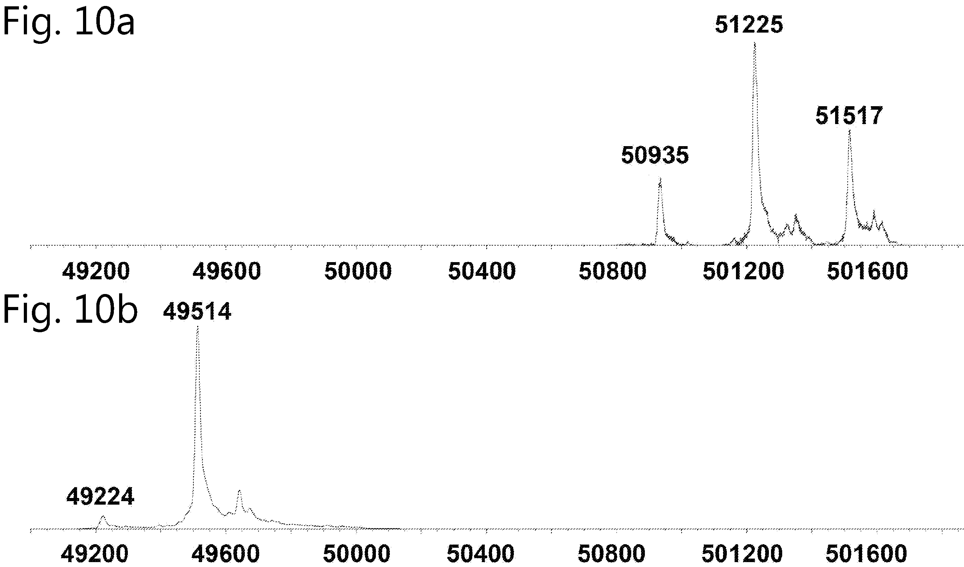

The process for trimming of a trastuzumab glycomutant (N297Q,L196N), as described in example 5, becomes visible from the MS profile depicted in Figure 10, which shows are range of glycoforms on the starting antibody, with main molecular mass of 50935, 51225 and 51517, which converge to two peaks after endoglycosidase trimming, of mass 49224 and 49514. As can be concluded, the homogeneity of the antibody increases as a result of the trimming process.

In the IgG antibody comprising one N-linked glycosylation site on the combination of a single heavy chain and single light chain, an oligosaccharide is attached at the N-glycosylation site, preferably to the amide side chain of an antibody amino acid. The oligosaccharide is attached via an N-glycosidic bond, in most cases to an asparagine (Asn) or arginine (Arg) amino acid side chain. The oligosaccharide attached to the antibody is also referred to as a glycan. A glycan may for example be attached to an antibody via C1 of a GlcNAc-residue, that is bonded to the amide side chain of an asparagine amino acid that is part of the antibody.

Numerous different types of glycans exist. As described above, the Fc regions of IgG antibodies bear a highly conserved N-glycosylation site. The N-glycans attached to this site are predominantly core-fucosylated diantennary structures of the complex type. Another type of glycan is the class of high mannose glycans. High-mannose typically comprises two N-acetylglucosamines and a varying number of mannose residues.

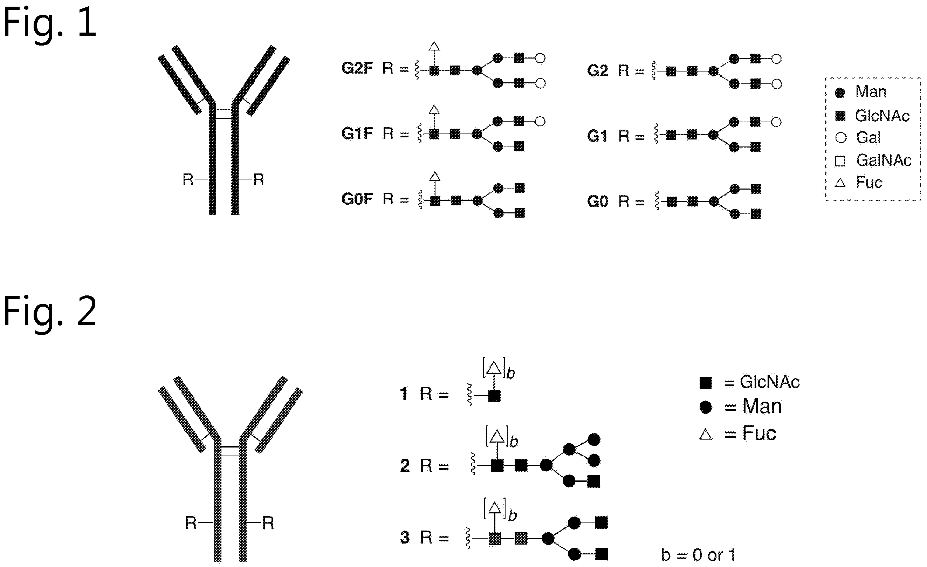

Figure 1 shows a diantennary glycan of the complex type, and the different glycoforms with respect of galactosylation (G0, G1 and G2) and fucosylation (G0F, G1F and G2F).

Figure 2 shows examples of different glycosylation profiles of a monoclonal antibody that may be obtained by regular expression followed by trimming with endoglycosidase (1), or by trimming with α- and β-mannosidases (2). Glycoform 3 can be obtained by trimming of the regular mixture of glycoforms (G0, G1, G2, G0F, G1F and G2F) upon combined action of sialidase and galactosidase.

In a preferred embodiment, in an IgG antibody comprising one N-linked glycosylation sites on the combination of a single heavy chain and single light chain, the oligosaccharide attached at the N-linked glycosylation site is a diantennary glycan of the complex type, and glycoforms thereof.

As described above, a glycan may be bonded to the antibody via a GlcNAc-residue, and this GlcNAc residue may be fucosylated. In Figure 1 and 2, this is denoted by b: when b is 0, said GlcNAc-residue is non-fucosylated and when b is 1, said GlcNAc is fucosylated.

In a large number of glycans, a second GlcNAc-residue is bonded to the GlcNac-residue that is directly bonded to the antibody, as is also seen in Figure 2, (2) and (3). Trimming of an oligosaccharide (glycan) in step (2) of this embodiment of the process according to the invention occurs in between these two GlcNAc-residues. Trimming of a glycan according to step (2) provides a GlcNAc-residue that is covalently bonded to the N-glycosylation site on the IgG antibody. This is also shown in Figure 2 (1). Such a GlcNAc-residue that is covalently bonded to an N-glycosylation site, preferably via an N-glycosidic bond via C1 of said GlcNAc and an amide present in an amino acid side chain of the antibody, is herein referred to as a "proximal N-linked GlcNAc-residue", and also "core-GlcNAc-substituent". Said proximal N-linked GlcNAc-residue or core-GlcNAc-substituent is optionally fucosylated.

In step (2) of this embodiment of the process for the preparation of a modified antibody according to the invention, the trimming of an oligosaccharide that is attached to the N-glycosylation site occurs by the action of a suitable enzyme, and after trimming the oligosaccharide (also referred to as glycan), a fucosylated (b in Figure 1(1) is 1) or a non-fucosylated (b is 0) proximal N-linked GlcNAc-residue (also referred to as core-GlcNAc-substituent) is obtained.

A "suitable enzyme" is defined as an enzyme wherefore the oligosaccharide that is to be trimmed is a substrate. The preferred type of enzyme that is to be used in step (2) depends on the specific oligosaccharide or oligosaccharides that is or are trimmed.

In a preferred embodiment of step (1) of this particular embodiment of the process according to the invention, the enzyme in step (2) is selected from the group of endo-glycosidases.

Endo-glycosidases are capable of cleaving internal glycosidic linkages in glycan structures, which provides another benefit to remodeling and synthetic endeavors. For example, endo-glycosidases can be employed for facile homogenization of heterogeneous glycan populations, when they cleave at predictable sites within conserved glycan regions. One of the most significant classes of endoglycosidases in this respect comprises the endo-β-N-acetylglucosaminidases (EC 3.2.1.96, commonly known as Endos and ENGases), a class of hydrolytic enzymes that remove N-glycans from glycoproteins by hydrolyzing the β-1,4-glycosidic bond in the N,N'-diacetylchitobiose core (reviewed by Wong et al. Chem. Rev. 2011, 111, 4259), leaving a single protein proximal N-linked GlcNAc residue. Endo-β-N-acetylglucosaminidases are found widely distributed through nature with common chemoenzymatic variants including Endo D, which is specific for pauci mannose; Endo A and Endo H, which are specific for high mannose; Endo F subtypes, which range from high mannose to biantennary complex; and Endo M, which can cleave most N-glycan structures (high mannose/complex-type/hybrid-type), except fucosylated glycans, and the hydrolytic activity for the high-mannose type oligosaccharides is significantly higher than that for the complex-and hybrid-type oligosaccharides. These ENGases show specificity toward the distal N-glycan structure and not the protein displaying it, making them useful for cleaving most N-linked glycans from glycoproteins under native conditions.

Endoglycosidases F1, F2, and F3 are most suitable for deglycosylation of native proteins. The linkage specificities of endo F1, F2, and F3 suggest a general strategy for deglycosylation of proteins that may remove all classes of N-linked oligosaccharides without denaturing the protein. Biantennary and triantennary structures can be immediately removed by endoglycosidases F2 and F3, respectively. Oligo- mannose and hybrid structures can be removed by Endo F1.

Endo F3 is unique in that its cleavage is sensitive to the state of peptide linkage of the oligosaccharide, as well as the state of core fucosylation. Endoglycosidase F3 cleaves asparagine-linked biantennary and triantennary complex oligosaccharides. It will cleave non-fucosylated biantennary and triantennary structures at a slow rate, but only if peptide-linked. Core fucosylated biantennary structures are efficient substrates for Endo F3, which activity up to 400-fold. There is no activity on oligomannose and hybrid molecules. See for example Tarentino et al. Glycobiology 1995, 5, 599.

Endo S is a secreted endoglycosidase from *Streptococcus pyogenes,* and also belongs to the glycoside hydrolase family 18, as disclosed by Collin et al. (EMBO J. 2001, 20, 3046). In contrast to the ENGases mentioned above, however, the endo S has a more defined specificity and is specific for cleaving only the conserved N-glycan in the Fc domain of human IgGs (no other substrate has been identified to date), suggesting that a protein-protein interaction between the enzyme and IgG provides this specificity.

Endo S49 is described in WO 2013/037824 (Genovis AB). Endo S49 is isolated from Streptococcus poyogenes NZ131 and is a homologue of Endo S. Endo S49 has a specific endoglycosidase activity on native IgG and cleaves a larger variety of Fc glycans than Endo S.

In a further preferred embodiment, the enzyme in step (2) of this embodiment is an endo-β-N-acetylglucosaminidase. In a further preferred embodiment, the endo-β-N-acetylglucosaminidase is selected from the group consisting of Endo S, Endo S 49, Endo F1, Endo F2, Endo F3, Endo H, Endo M, Endo A, and any combination thereof.

When the oligosaccharide to be trimmed is a diantennary structure of the complex type, the endo-β-N-acetylglucosaminidase is preferably selected from the group consisting of Endo S, Endo S49, Endo F1, Endo F2, Endo F3, and a combination thereof.

When the oligosaccharide to be trimmed is a diantennary structure of the complex type (i.e. according to Figure 2(**3**), and it is present at the IgG conserved N-glycosylation site at N297, the endo-β-N-acetylglucosaminidase is preferably selected from the group consisting of Endo S, Endo S49, Endo F2, Endo F3, and a combination thereof, more preferably from the group consisting of Endo S, Endo S49, and a combination thereof.

When the oligosaccharide to be trimmed is a diantennary structure of the complex type, and it is not present at the IgG conserved N-glycosylation site at N297, the endo-β-N-acetylglucosaminidase is preferably selected from the group consisting of Endo F2, Endo F3, and a combination thereof.

When the oligosaccharide to be trimmed is a high mannose, the endo-β-N-acetylglucosaminidase is preferably selected from the group consisting of Endo H, Endo M, Endo A and Endo F1.

The trimming step (2) of the process according to the invention is preferably performed in a suitable buffer solution, such as for example phosphate, buffered saline (e.g. phosphate-buffered saline, tris-buffered saline), citrate, HEPES, tris and glycine. Suitable buffers are known in the art. Preferably, the buffer solution is phosphate-buffered saline (PBS) or tris buffer.

The process is preferably performed at a temperature in the range of about 4 to about 50°C, more preferably in the range of about 10 to about 45°C, even more preferably in the range of about 20 to about 40°C, and most preferably in the range of about 30 to about 37°C.

The process is preferably performed a pH in the range of about 5 to about 9, preferably in the range of about 5.5 to about 8.5, more preferably in the range of about 6 to about 8. Most preferably, the process is performed at a pH in the range of about 7 to about 8.

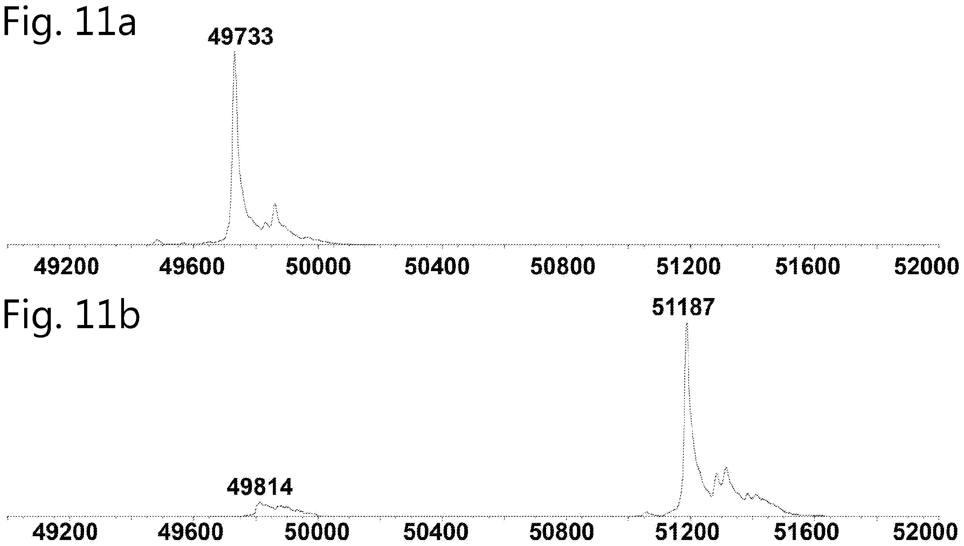

For example, a trastuzumab mutant-(N297Q, L196N) was provided via the process according to the invention described above. Said trastuzumab mutant-(N297Q, L196N) was treated with an endo-β-N-acetylglucosamidase in order to obtain a proximal N-linked GlcNAc residue at the N196 glycosylation site, as described in example 5. Figure 10 shows (A) the mass spectral profile of trastuzumab mutant-(N297Q, L196N) as isolated after expression in CHO and (B) the mass spectral profile of trastuzumab-(N297Q, L196N) after treatment with endo F3 (or endo F2). Subsequently, a monosaccharide derivative Su(A)ₓ, GalNAz, was attached to the proximal N-linked GlcNAc residue. Figure 11 shows (A) the mass spectral profile of trastuzumab mutant (N297Q, L196N) after treatment with endo F3 (or endo F2), as described in example 9, and after GalT1-mediated transfer of GalNAz onto the terminal GlcNAc and (B) the mass spectral profile of antibody drug conjugate resulting from conjugation of azide-charged trastuzumab mutant N297Q, L196N after treatment with BCN-vc-PABA-MMAF **34,** as described in example 14.

### Modified antibody

The present invention further relates to a modified antibody obtainable by the process for the preparation of a modified antibody according to the invention. Said process, as well as preferred embodiments thereof, are described in detail above. The preferred embodiments of the process for the modification of an antibody also apply to the modified antibody obtainable by that process.

In a preferred embodiment, x is 1 or 2, more preferably x is 1, wherein x is as defined above in Su(A)ₓ.

The invention also relates to an IgG antibody comprising one, and no more than one, N-linked glycosylation site on the combination of a single heavy chain and single light chain, wherein the N-linked glycosylation site is a mutant N-linked glycosylation site as compared to its wild type counterpart, wherein a proximal N-linked GlcNAc-Su(A)ₓ-substituent is attached to the antibody, wherein the GlcNAc in the N-linked GlcNAc-Su(A)ₓ-substituent is optionally fucosylated, and wherein Su(A)ₓ is defined as a monosaccharide derivative Su comprising x functional groups A wherein x is 1, 2, 3 or 4 and wherein A is selected from the group consisting of an azido group, a keto group, an alkynyl group, a thiol group or a precursor thereof, a halogen, a sulfonyloxy group, a halogenated acetamido group, a mercaptoacetamido group and a sulfonylated hydroxyacetamido group.

In a preferred embodiment, wherein the IgG antibody is a whole antibody, the modified antibody according to the invention is an antibody according to formula (140): 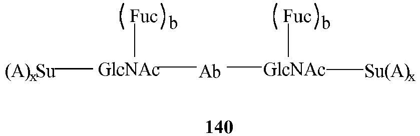 wherein:
Ab represents an IgG antibody comprising one, and no more than one, N-linked glycosylation site on the combination of a single heavy chain and single light chain, wherein the N-linked glycosylation site is a mutant N-linked glycosylation site as compared to its wild type counterpart; Su(A) and x are as defined above; and b is 0 (non-fucosylated) or 1 (fucosylated).

In a preferred embodiment, Su(A)ₓ comprises 1 or 2 functional groups A, i.e. preferably x is 1 or 2. More preferably, x is 1. In another preferred embodiment, Su is galactose (Gal). In a further preferred embodiment, x is 1 or 2 and Su is Gal, and most preferably, x is 1 and Su is Gal.

In a preferred embodiment, Su(A)ₓ is selected from the group consisting of GalNAz, 6-AzGalNAc, 6-GalNAcCl, 6-GalNAcSH, 2-GalNAcCl, 2-GalNAcSH, 6-ClGal, 2-ClGal, 2-HSGal and 6-HSGal, more preferably form the group consisting of GalNAz, 6-AzGalNAc, 6-GalNAcCl, 6-GalNAcSH, 2-GalNAcCl, 2-GalNAcSH, 6-ClGal-and 2-ClGal. In another preferred embodiment, Su(A)ₓ is selected from the group consisting of 2-GalNProSH and 2-GalNBuSH.

In a further preferred embodiment, x is 1 and Su(A)ₓ is selected from the group consisting of GalNAz, 6-AzGalNAc, 6-GalNAcCl, 6-GalNAcSH, 2-GalNAcCl, 2-GalNAcSH, 6-ClGal, 2-ClGal, 2-HSGal and 6-HSGal, more preferably form the group consisting of GalNAz, 6-AzGalNAc, 6-GalNAcCl, 6-GalNAcSH, 2-GalNAcCl, 2-GalNAcSH, 6-ClGal-and 2-ClGal. In another further preferred embodiment, x is 1 and Su(A)ₓ is selected from the group consisting of 2-GalNProSH and 2-GalNBuSH.

In a preferred embodiment of the modified antibody according to the invention, A is an azide group, a thiol group or a halogen. The modified antibody is preferably an azide-modified antibody, a thiol-modified antibody or a halogen-modified antibody. When the antibody is a halogen-modified antibody, it is preferably a chloride-modified antibody, a bromide-modified antibody or an iodide-modified antibody, more preferably a chloride-modified antibody or a bromide-modified antibody, and most preferably a chloride-modified antibody. More preferably, x is 1 and the modified antibody is preferably an azide-modified antibody, a thiol-modified antibody or a halogen-modified antibody (preferably a chloride- or a bromide-modified antibody, most preferably a chloride-modified antibody).

In a preferred embodiment wherein A is an azide group, Su(A)ₓ is preferably selected from the group consisting of 2-azidoacetamidogalactose (GalNAz), 6-azido-6-deoxygalactose (6-AzGal), 6-azido-6-deoxy-2-acetamidogalactose (6-AzGalNAc), 4-azido-4-deoxy-2-acetamidogalactose (4-AzGalNAc), 6-azido-6-deoxy-2-azidoacetamidogalactose (6-AzGalNAz), 2-azidoacetamidoglucose (GlcNAz), 6-azido-6-deoxyglucose (6-AzGlc), 6-azido-6-deoxy-2-acetamidoglucose (6-AzGlcNAc), 4-azido-4-deoxy-2-acetamidoglucose (4-AzGlcNAc) and 6-azido-6-deoxy-2-azidoacetamidoglucose (6-AzGlcNAz). In a further preferred embodiment Su(A)ₓ is selected from the group consisting of GalNAz, 6-AzGal, 4-AzGalNAc, GlcNAz and 6-AzGlcNAc. More preferably, x is 1 and Su(A)ₓ is selected from the group consisting of 2-azidoacetamidogalactose (GalNAz), 6-azido-6-deoxygalactose (6-AzGal), 6-azido-6-deoxy-2-acetamidogalactose (6-AzGalNAc), 4-azido-4-deoxy-2-acetamidogalactose (4-AzGalNAc), 6-azido-6-deoxy-2-azidoacetamidogalactose (6-AzGalNAz), 2-azidoacetamidoglucose (GlcNAz), 6-azido-6-deoxyglucose (6-AzGlc), 6-azido-6-deoxy-2-acetamidoglucose (6-AzGlcNAc), 4-azido-4-deoxy-2-acetamidoglucose (4-AzGlcNAc) and 6-azido-6-deoxy-2-azidoacetamidoglucose (6-AzGlcNAz). More preferably, x is 1 and Su(A)ₓ is selected from the group consisting of GalNAz, 6-AzGal, 4-AzGalNAc, GlcNAz and 6-AzGlcNAc.

In a particularly preferred embodiment of the modified antibody according to the invention, Su(A)ₓ is selected from the group consisting of GalNAz, 6-AzGalNAc, 2-GalNAcSH, 2-GalNAcX, 2-GalNAcOS(O)₂R⁸, 6-GalNAcSH, 6-GalNAcX and 6-GalNAcOS(O)₂R⁸. In another particularly preferred embodiment of the modified antibody according to the invention, Su(A)ₓ is selected from the group consisting of 2-GalNProSH and 2-GalNBuSH.

In an even more preferred embodiment, x is 1 or 2 and Su(A)ₓ is selected from the group consisting of GalNAz, 6-AzGalNAc, 2-GalNAcSH, 2-GalNAcX, 2-GaNAcOS(O)₂R⁸, 6-GalNAcSH, 6-GalNAcX and 6-GalNAcOS(O)₂R⁸. In another even more preferred embodiment, x is 1 or 2 and Su(A)ₓ is selected from the group consisting of 2-GalNProSH and 2-GalNBuSH.

In a most preferred embodiment, x is 1 and Su(A)ₓ is selected from the group consisting of GalNAz, 6-AzGalNAc, 2-GalNAcSH, 2-GalNAcX, 2-GalNAcOS(O)₂R⁸, 6-GalNAcSH, 6-GalNAcX and 6-GalNAcOS(O)₂R⁸. R⁸ and preferred embodiments thereof are as defined above. In another most preferred embodiment, x is 1 and Su(A)ₓ is selected from the group consisting of 2-GalNProSH and 2-GalNBuSH.

### Process for the preparation of an antibody-conjugate.

The invention further relates to the use of a modified antibody according to the invention in the preparation of an antibody-conjugate, wherein an antibody-conjugate is defined as an antibody that is conjugated to a molecule of interest D via a linker L.

An antibody-conjugate is herein defined as an antibody that is conjugated to a molecule of interest D via a linker L. The antibody-conjugate according to the invention may be conjugated to one or to more than one molecule of interest D via said linker L.

A molecule of interest may for example be a reporter molecule, a diagnostic molecule, an active substance, an enzyme, an amino acid (including an unnatural amino acid), a (non-catalytic) protein, a peptide, a polypeptide, an oligonucleotide, a glycan, a (poly)ethylene glycol diamine (e.g. 1,8-diamino-3,6-dioxaoctane or equivalents comprising longer ethylene glycol chains), a polyethylene glycol chain, a polyethylene oxide chain, a polypropylene glycol chain, a polypropylene oxide chain, 1,x-diaminoalkane (wherein x is the number of carbon atoms in the alkane), an azide or a (hetero)cycloalkynyl moiety, preferably a bivalent or bifunctional (hetero)cycloalkynyl moiety. In a preferred embodiment, the molecule of interest is selected from the group consisting of an amino acid (in particular lysine), an active substance, a reporter molecule, an azide and a (hetero)cycloalkynyl moiety.

An active substance is herein defined as a pharmacological and/or biological substance, i.e. a substance that is biologically and/or pharmaceutically active, for example a drug or a prodrug, a diagnostic agent, an amino acid, a protein, a peptide, a polypeptide, a glycan, a lipid, a vitamin, a steroid, a nucleotide, a nucleoside, a polynucleotide, RNA or DNA. Examples of suitable peptide tags include cellpenetrating peptides like human lactoferrin or polyarginine. An example of a suitable glycan is oligomannose.

In a preferred embodiment, the active substance is selected from the group consisting of drugs and prodrugs. More preferably, the active substance is selected from the group consisting of pharmaceutically active compounds, in particular low to medium molecular weight compounds (e.g. about 200 to about 1500 Da, preferably about 300 to about 1000 Da), such as for example cytotoxins, antiviral agents, antibacterials agents, peptides and oligonucleotides. Examples of cytotoxins include colchicine, vinca alkaloids, camptothecins, doxorubicin, daunorubicin, taxanes, calicheamycins, tubulysins, irinotecans, an inhibitory peptide, amanitin, deBouganin, duocarmycins, maytansines, auristatins or pyrrolobenzodiazepines (PBDs). In a preferred embodiment, the cytotoxin is selected from the group consisting of camptothecins, doxorubicin, daunorubicin, taxanes, calicheamycins, duocarmycins, maytansines, auristatins and pyrrolobenzodiazepines (PBDs). In another preferred embodiment, the cytotoxin is selected from the group consisting of colchicine, vinca alkaloids, tubulysins, irinotecans, an inhibitory peptide, amanitin and deBouganin.

A reporter molecule is a molecule whose presence is readily detected, for example a diagnostic agent, a dye, a fluorophore, a radioactive isotope label, a contrast agent, a magnetic resonance imaging agent or a mass label. Examples of a fluorophore include all kinds of Alexa Fluor (e.g. Alexa Fluor 555), cyanine dyes (e.g. Cy3 or Cy5), coumarin derivatives, fluorescein, rhodamine, allophycocyanin and chromomycin.

Examples of radioactive isotope label include ^{99m}Tc, ¹¹¹In, ¹⁸F, ¹⁴C, ⁶⁴Cu, ¹³¹I or ¹²³I, which may or may not be connected via a chelating moiety such as DTPA, DOTA, NOTA or HYNIC.

In the antibody-conjugate according to the invention, the molecule of interest D is conjugated to the antibody via a linker L. Linkers or linking units are well known in the art, and are described in more detail below.

In a preferred embodiment, the process for the preparation of an antibody-conjugate according to the invention comprises reacting an IgG antibody comprising one, and no more than one, N-linked glycosylation site on the combination of a single heavy chain and single light chain, wherein the N-linked glycosylation site is a mutant N-linked glycosylation site as compared to its wild type counterpart and wherein a proximal GlcNASu(A)ₓ-substituent is attached to said N-linked glycosylation site, with a linker-conjugate; wherein said linker-conjugate comprises a functional group B and one or more molecules of interest D, wherein said functional group B is a functional group that is capable of reacting with a functional group A of the proximal GlcNASu(A)ₓc-substituent on said antibody, wherein Su(A)ₓ is a sugar derivative comprising x functional groups A wherein x is 1, 2, 3 or 4 and A is independently selected from the group consisting of an azido group, a keto group, an alkynyl group, a thiol group, a halogen, a sulfonyloxy group, a halogenated acetamido group, a mercaptoacetamido group and a sulfonylated hydroxyacetamido group; and wherein said proximal GlcNAcSu(A)ₓ-substituent is optionally fucosylated.

In a preferred embodiment of the process for the preparation of an antibody-conjugate, Su(A)ₓ comprises 1 or 2 functional groups A, i.e. preferably x is 1 or 2. More preferably, x is 1. In another preferred embodiment, Su is galactose (Gal). In a further preferred embodiment, x is 1 or 2 and Su is Gal, and most preferably, x is 1 and Su is Gal. In these preferred embodiments it is further preferred that the linker-conjugate comprises 1 or 2, and most preferably 1, molecules of interest.

In a preferred embodiment, Su(A)ₓ is selected from the group consisting of GalNAz, 6-AzGalNAc, 6-GalNAcCl, 6-GalNAcSH, 2-GalNAcCl, 2-GalNAcSH, 6-ClGal, 2-ClGal, 2-HSGal and 6-HSGal, more preferably form the group consisting of GalNAz, 6-AzGalNAc, 6-GalNAcCl, 6-GalNAcSH, 2-GalNAcCl, 2-GalNAcSH, 6-ClGal-and 2-ClGal. In another preferred embodiment, Su(A)ₓ is selected from the group consisting of 2-GalNProSH and 2-GalNBuSH. In these preferred embodiments it is further preferred that the linker-conjugate comprises 1 or 2, and most preferably 1, molecules of interest.

In a further preferred embodiment, x is 1 and Su(A)ₓ is selected from the group consisting of GalNAz, 6-AzGalNAc, 6-GalNAcCl, 6-GalNAcSH, 2-GalNAcCl, 2-GalNAcSH, 6-ClGal, 2-ClGal, 2-HSGal and 6-HSGal, more preferably from the group consisting of GalNAz, 6-AzGalNAc, 6-GalNAcCl, 6-GalNAcSH, 2-GalNAcCl, 2-GalNAcSH, 6-ClGal-and 2-ClGal. In another further preferred embodiment, x is 1 and Su(A)ₓ is selected from the group consisting of 2-GalNProSH and 2-GalNBuSH. In these preferred embodiments it is further preferred that the linker-conjugate comprises 1 or 2, and most preferably 1, molecules of interest.

In a preferred embodiment wherein A is an azide group, Su(A)ₓ is preferably selected from the group consisting of 2-azidoacetamidogalactose (GalNAz), 6-azido-6-deoxygalactose (6-AzGal), 6-azido-6-deoxy-2-acetamidogalactose (6-AzGalNAc), 4-azido-4-deoxy-2-acetamidogalactose (4-AzGalNAc), 6-azido-6-deoxy-2-azidoacetamidogalactose (6-AzGalNAz), 2-azidoacetamidoglucose (GlcNAz), 6-azido-6-deoxyglucose (6-AzGlc), 6-azido-6-deoxy-2-acetamidoglucose (6-AzGlcNAc), 4-azido-4-deoxy-2-acetamidoglucose (4-AzGlcNAc) and 6-azido-6-deoxy-2-azidoacetamidoglucose (6-AzGlcNAz). In a further preferred embodiment Su(A)ₓ is selected from the group consisting of GalNAz, 6-AzGal, 4-AzGalNAc, GlcNAz and 6-AzGlcNAc. More preferably, x is 1 and Su(A)ₓ is selected from the group consisting of 2-azidoacetamidogalactose (GalNAz), 6-azido-6-deoxygalactose (6-AzGal), 6-azido-6-deoxy-2-acetamidogalactose (6-AzGalNAc), 4-azido-4-deoxy-2-acetamidogalactose (4-AzGalNAc), 6-azido-6-deoxy-2-azidoacetamidogalactose (6-AzGalNAz), 2-azidoacetamidoglucose (GlcNAz), 6-azido-6-deoxyglucose (6-AzGlc), 6-azido-6-deoxy-2-acetamidoglucose (6-AzGlcNAc), 4-azido-4-deoxy-2-acetamidoglucose (4-AzGlcNAc) and 6-azido-6-deoxy-2-azidoacetamidoglucose (6-AzGlcNAz). More preferably, x is 1 and Su(A)ₓ is selected from the group consisting of GalNAz, 6-AzGal, 4-AzGalNAc, GlcNAz and 6-AzGlcNAc. In these preferred embodiments it is further preferred that the linker-conjugate comprises 1 or 2, and most preferably 1, molecules of interest.

In a particularly preferred embodiment of the modified antibody according to the invention, Su(A)ₓ is selected from the group consisting of GalNAz, 6-AzGalNAc, 2-GalNAcSH, 2-GalNAcX, 2-GalNAcOS(O)₂R⁸, 6-GalNAcSH, 6-GalNAcX and 6-GalNAcOS(O)₂R⁸. In another particularly preferred embodiment of the modified antibody according to the invention, Su(A)ₓ is selected from the group consisting of 2-GalNProSH and 2-GalNBuSH. In an even more preferred embodiment, x is 1 or 2 and Su(A)ₓ is selected from the group consisting of GalNAz, 6-AzGalNAc, 2-GalNAcSH, 2-GalNAcX, 2-GalNAcOS(O)₂R⁸, 6-GalNAcSH, 6-GalNAcX and 6-GalNAcOS(O)₂R⁸. In another even more preferred embodiment, x is 1 or 2 and Su(A)ₓ is selected from the group consisting of 2-GalNProSH and 2-GalNBuSH. In a most preferred embodiment, x is 1 and Su(A)ₓ is selected from the group consisting of GalNAz, 6-AzGalNAc, 2-GalNAcSH, 2-GalNAcX, 2-GalNAcOS(O)₂R⁸, 6-GalNAcSH, 6-GalNAcX and 6-GalNAcOS(O)₂R⁸. In another most preferred embodiment, x is 1 and Su(A)ₓ is selected from the group consisting of 2-GalNProSH and 2-GalNBuSH. In these preferred embodiments it is further preferred that the linker-conjugate comprises 1 or 2, and most preferably 1, molecules of interest. R⁸, and preferred embodiments of R⁸, are as defined above.

The linker-conjugate preferably is of the formula B-L(D)ᵣ, wherein D is a molecule of interest as defined above, and B and L are as defined below, and r is 1 - 20. Preferably r is 1 - 10, more preferably r is 1 - 8, even more preferably r is 1, 2, 3, 4, 5 or 6, even more preferably r is 1, 2, 3 or 4, even more preferably r is 1 or 2, and most preferably r is 1. In other words, the linker-conjugate is preferably linked to 1 or 2, preferably 1 molecule of interest.

Complementary functional groups B for the functional group A on the modified antibody (A is an azido group, a keto group, an alkynyl group, a thiol group, a halogen, a sulfonyloxygroup, a halogenated acetamido group, a mercaptoacetamido group or a sulfonylated hydroxyacetamido group) are known in the art.

When A is an azido group, linking of the azide-modified antibody and the linker-conjugate preferably takes place via a cycloaddition reaction. Functional group B is then preferably selected from the group consisting of alkynyl groups, preferably terminal alkynyl groups, and (hetero)cycloalkynyl groups.

When A is a keto group, linking of the keto-modified antibody with the linker-conjugate preferably takes place via selective conjugation with hydroxylamine derivatives or hydrazines, resulting in respectively oximes or hydrazones. Functional group B is then preferably a primary amino group, e.g. an -NH₂ group, an aminooxy group, e.g. -O-NH₂, or a hydrazinyl group, e.g. -N(H)NH₂. The linker-conjugate is then preferably H₂N-L(D)ᵣ, H₂N-O-L(D)ᵣ or H₂N-N(H)-L(D)ᵣ respectively, wherein L, D and r are as defined above.

When A is an alkynyl group, linking of the alkyne-modified antibody with the linker-conjugate preferably takes place via a cycloaddition reaction, preferably a 1,3-dipolar cycoaddition. Functional group B is then preferably a 1,3-dipole, such as an azide, a nitrone or a nitrile oxide. The linker-conjugate is then preferably N₃-L(D)ᵣ, wherein L, D and r are as defined above.

When A is a thiol group, linking of the thiol-modified antibody with the linker-conjugate preferably takes place via a Michael-type addition reaction. Functional group B is then preferably an N-maleimidyl group for the Michael-type addition, a halogenated acetamido group for the nucleophilic substitution reaction, or a terminal alkene for the thiol-ene reaction. The linker-conjugate is then preferably X-CH₂C(O)NHL(D)ᵣ or X-CH₂C(O)N[L(D)ᵣ]₂ wherein X is F, Cl, Br or I, or a maleimidelinker-conjugate (141) as illustrated below.

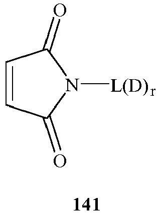

When A is a halogen-modified antibody, a halogenated acetamide-modified antibody, a sulfonyloxy-modified antibody or a mercaptoacetamide-modified antibody, linking of the modified antibody with the linker-conjugate preferably takes place via reaction with a thiol to form a thioether. When A is a halogen, a halogenated acetamido group, a sulfonyloxy group or a mercaptoacetamido group, linking of the modified antibody with the linker-conjugate preferably takes place via reaction with a thiol to form a thioether. In other words, when the modified antibody is a halogen-modified antibody, a halogenated acetamide-modified antibody, a sulfonyloxy-modified antibody or a mercaptoacetamide-modified antibody, linking of the modified antibody with the linker-conjugate preferably takes place via reaction with a thiol to form a thioether.

Functional group B comprises then preferably a thiol group, and a preferred linker-conjugate is HS-L(D)ᵣ. However, functional group B may also comprise an alcohol group or an amine group.

A preferred embodiment of the process for the preparation of an antibody-conjugate according to the invention comprises reacting a modified antibody with a linker-conjugate, wherein:
(a) when the modified antibody is an azido-modified antibody, the linker-conjugate comprises a (hetero)cycloalkynyl group or an alkynyl group, and one or more molecules of interest; or
(b) when the modified antibody is a keto-modified antibody, the linker-conjugate comprises a primary amine group, an aminooxy group or a hydrazinyl group, and one or more molecules of interest; or
(c) when the modified antibody is an alkyne-modified antibody, the linker-conjugate comprises an azido group, a nitrone or a nitrile oxide, and one or more molecules of interest.
(d) when the modified antibody is a thiol-modified antibody or a mercaptoacetamide-modified antibody, the linker-conjugate comprises an N-maleimide group or a halogenated acetamido group or a terminal alkene, and one or more molecules of interest; or
(e) when the modified antibody is a halogen-modified antibody, a halogenated acetamido-modified antibody, a sulfonyloxy-modified antibody or a sulfonylated hydroxyacetamido-modified antibody, the linker-conjugate comprises a thiol group, and one or more molecules of interest.

When said modified antibody is a halogen-modified antibody and functional group B comprises a thiol group, said thiol group may be an aliphatic or an aromatic thiol group. In a preferred embodiment said thiol group is an aromatic thiol group.

In a preferred embodiment, the modified antibody is a thiol-modified antibody and functional group B comprises an N-maleimide group or a halogenated acetamido group.

In a preferred embodiment of the process for the preparation of an antibody-conjugate, linker-conjugate B-L(D)ᵣ is selected from the group consisting of linker-conjugates of formula **(142a), (142b), (143), (144), (141)** or **(145):** 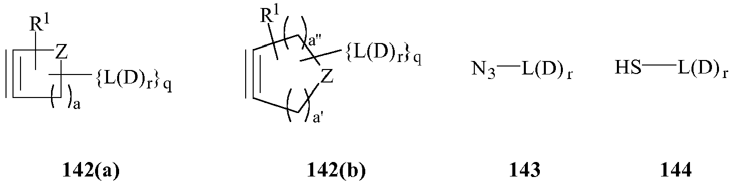 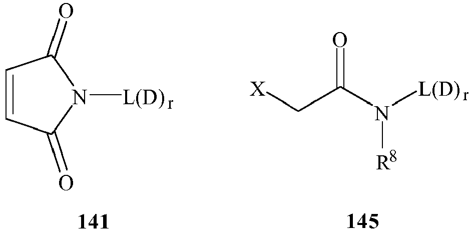 wherein:
L is a linker;
D is a molecule of interest;
r is 1 - 20;
R' is independently selected from the group consisting of hydrogen, halogen, -OR⁵, -NO₂, -CN, -S(O)₂R⁵, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups and wherein the alkyl groups, (hetero)aryl groups, alkyl(hetero)aryl groups and (hetero)arylalkyl groups are optionally substituted, wherein two substituents R¹ may be linked together to form an annelated cycloalkyl or an annelated (hetero)arene substituent, and wherein R⁵ is independently selected from the group consisting of hydrogen, halogen, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups;
Z is C(R¹)₂, O, S or NR², wherein R² is R¹ or L(D)ᵣ, and wherein L, D and r are as defined above;
q is 0 or 1, with the proviso that if q is 0 then Z is N-L(D)ᵣ;
a is 1, 2, 3, 4, 5, 6, 7, 8, 9 or 10;
a' is 0, 1, 2, 3, 4, 5, 6, 7 or 8;
a" is 0,1, 2, 3, 4, 5, 6, 7 or 8;
a'+a"< 10;
X is F, Cl, Br or I; and
R⁸ is R¹ or -L(D)ᵣ, preferably hydrogen, -L(D)ᵣ or a C₁ - C₂₄ alkyl group, more preferably hydrogen, -L(D)ᵣ or a C₁ - C₆ alkyl group.

In another preferred embodiment of the process for the preparation of an antibody-conjugate according to the invention, linker-conjugate B-L(D)ᵣ is selected from the group consisting of linker-conjugates of formula **(142a), (143), (144), (141)** or **(145),** as defined above.

As was described above, it is preferred that r is 1 - 10, more preferably r is 1 - 8, even more preferably r is 1, 2, 3, 4, 5 or 6, even more preferably r is 1, 2, 3 or 4, even more preferably r is 1 or 2, and most preferably r is 1. In other words, the linker-conjugate is preferably linked to 1 or 2, preferably 1 molecule of interest.

In a preferred embodiment, the modified antibody according to the invention is an azide-modified antibody, an alkyne-modified antibody, a halogen-modified antibody or a thiol-modified antibody.

A suitable linker-conjugate for the preparation of a antibody-conjugate according to the invention is a linker-conjugate comprising a functional group B and a molecule of interest. Linkers L, also referred to as linking units, are well known in the art. In a linker-conjugate as described herein, L is linked to a molecule of interest D as well as to a functional group B, as was described above. Numerous methods for linking said functional group B and said molecule of interest D to L are known in the art. As will be clear to a person skilled in the art, the choice of a suitable method for linking a functional group B to one end and a molecule of interest D to another end of a linker depends on the exact nature of the functional group B, the linker L and the molecule of interest D.

A linker may have the general structure F¹-L(F²)ᵣ, wherein F¹ represents either a functional group B or a functional group that is able to react with a functional group F on the functional group B as described above, e.g. a (hetero)cycloalkynyl group, a terminal alkynyl group, a primary amine, an aminooxy group, a hydrazyl group, an azido group, an N-maleimidyl group, an acetamido group or a thiol group. F² represents a functional group that is able to react with a functional group F on the molecule of interest.

Since more than one molecule of interest may be bonded to a linker, more than one functional group F² may be present on L. As was described above, r is 1 to 20, preferably 1 to 10, more preferably 1 to 8, even more preferably 1, 2, 3, 4, 5 or 6, even more preferably 1, 2, 3 or 4 and most preferably, r is 1 or 2.

L may for example be selected from the group consisting of linear or branched C₁-C₂₀₀ alkylene groups, C₂-C₂₀₀ alkenylene groups, C₂-C₂₀₀ alkynylene groups, C₃-C₂₀₀ cycloalkylene groups, C₅-C₂₀₀ cycloalkenylene groups, C₈-C₂₀₀ cycloalkynylene groups, C₇-C₂₀₀ alkylarylene groups, C₇-C₂₀₀ arylalkylene groups, C₈-C₂₀₀ arylalkenylene groups, C₉-C₂₀₀ arylalkynylene groups. Optionally the alkylene groups, alkenylene groups, alkynylene groups, cycloalkylene groups, cycloalkenylene groups, cycloalkynylene groups, alkylarylene groups, arylalkylene groups, arylalkenylene groups and arylalkynylene groups may be substituted, and optionally said groups may be interrupted by one or more heteroatoms, preferably 1 to 100 heteroatoms, said heteroatoms preferably being selected from the group consisting of O, S and NR⁵, wherein R⁵ is independently selected from the group consisting of hydrogen, halogen, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups. Most preferably, the heteroatom is O.

F, F¹ and F² may for example be independently selected from the group consisting of hydrogen, halogen, R⁵, C₄ - C₁₀ (hetero)cycloalkyne groups, -CH=C(R⁵)₂, -C≡CR⁵, -[C(R⁵)₂C(R⁵)₂O]_{q}-R⁵, wherein q is in the range of 1 to 200, -CN, -N₃, -NCX, -XCN, -XR⁵, -N(R⁵)₂, -N(R⁵)₃, -C(X)N(R⁵)₂, -C(R⁵)₂XR⁵, -C(X)R⁵, -C(X)XR⁵, -S(O)R⁵, -S(O)₂R⁵, -S(O)OR⁵, -S(O)₂OR⁵, -S(O)N(R⁵)₂, -S(O)₂N(R⁵)₂, -OS(O)R⁵, -OS(O)₂R⁵, -OS(O)OR⁵, -OS(O)₂OR⁵, -P(O)(R⁵)(OR⁵), -P(O)(OR⁵)₂, -OP(O)(OR⁵)₂, -Si(R⁵)₃, -XC(X)R⁵, -XC(X)XR⁵, -XC(X)N(R⁵)₂, -N(R⁵)C(X)R⁵, -N(R⁵)C(X)XR⁵ and -N(R⁵)C(X)N(R⁵)₂, wherein X is oxygen or sulphur and wherein R⁵ is as defined above.

Examples of suitable linking units include (poly)ethylene glycol diamines (e.g. 1,8-diamino-3,6-dioxaoctane or equivalents comprising longer ethylene glycol chains), polyethylene glycol or polyethylene oxide chains, polypropylene glycol or polypropylene oxide chains and 1,x-diaminoalkanes wherein x is the number of carbon atoms in the alkane.

Another class of suitable linkers comprises cleavable linkers. Cleavable linkers are well known in the art. For example Shabat et al., Soft Matter 2012, 6, 1073, discloses cleavable linkers comprising self-immolative moieties that are released upon a biological trigger, e.g. an enzymatic cleavage or an oxidation event. Some examples of suitable cleavable linkers are peptide-linkers that are cleaved upon specific recognition by a protease, e.g. cathepsin, plasmin or metalloproteases, or glycosidebased linkers that are cleaved upon specific recognition by a glycosidase, e.g. glucoronidase, or nitroaromatics that are reduced in oxygen-poor, hypoxic areas.

As was described above, when the modified antibody is an azide-modified antibody, it is preferred that the linker-conjugate is a (hetero)cycloalkyne linker-conjugate of formula **(142a).** 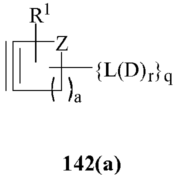 wherein:
L is a linker;
D is a molecule of interest;
r is 1 - 20;
R¹ is independently selected from the group consisting of hydrogen, halogen, -OR⁵, -NO₂, -CN, -S(O)₂R⁵, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups and wherein the alkyl groups, (hetero)aryl groups, alkyl(hetero)aryl groups and (hetero)arylalkyl groups are optionally substituted, wherein two substituents R¹ may be linked together to form an annelated cycloalkyl or an annelated (hetero)arene substituent, and wherein R⁵ is independently selected from the group consisting of hydrogen, halogen, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups;
Z is C(R¹)₂, O, S or NR², wherein R² is R¹ or L(D)ᵣ, and wherein L, D and r are as defined above;
q is 0 or 1, with the proviso that if q is 0 then Z is N-L(D)ᵣ; and
a is 1, 2, 3, 4, 5, 6, 7, 8, 9 or 10.

In a further preferred embodiment, a is 5, i.e. said (hetero)cycloalkynyl group is preferably a (hetero)cyclooctyne group.

In another preferred embodiment, Z is C(R²)₂ or NR². When Z is C(R²)₂ it is preferred that R² is hydrogen. When Z is NR², it is preferred that R² is L(D)ᵣ. In yet another preferred embodiment, r is 1 to 10, more preferably, r is 1, 2, 3, 4, 5 or 6, even more preferably r is 1, 2, 3 or 4, even more preferably r is 1 or 2, and most preferably is 1. In another preferred embodiment, q is 1 or 2, more preferably q is 1. Even more preferably, r is 1 and q is 1, and most preferably, a is 5 and r is 1 and q is 1.

In another further preferred embodiment, when the modified glycoprotein is an azide-modified glycoprotein, the linker-conjugate is a (hetero)cycloalkyne linker-conjugate of formula **(142b):** 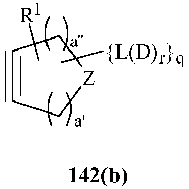 wherein:
L is a linker;
D is a molecule of interest;
r is 1 - 20;
R¹ is independently selected from the group consisting of hydrogen, halogen, -OR⁵, -NO₂, -CN, -S(O)₂R⁵, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups and wherein the alkyl groups, (hetero)aryl groups, alkyl(hetero)aryl groups and (hetero)arylalkyl groups are optionally substituted, wherein two substituents R¹ may be linked together to form an annelated cycloalkyl or an annelated (hetero)arene substituent, and wherein R⁵ is independently selected from the group consisting of hydrogen, halogen, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups;
Z is C(R¹)₂, O, S or NR², wherein R² is R¹ or L(D)ᵣ, and wherein L, D and r are as defined above;
q is 0 or 1, with the proviso that if q is 0 then Z is N-L(D)ᵣ;
a' is 0, 1, 2, 3, 4, 5, 6, 7 or 8;
a" is 0,1, 2, 3, 4, 5, 6, 7 or 8; and
a'+a"< 10.

In a further preferred embodiment, a' + a" is 4, 5, 6 or 7, more preferably a' + a" is 4, 5 or 6 and most preferably a' + a" is 5, i.e. said (hetero)cycloalkynyl group is preferably a (hetero)cyclooctyne group.

In another preferred embodiment, Z is C(R²)₂ or NR². When Z is C(R²)₂ it is preferred that R² is hydrogen. When Z is NR², it is preferred that R² is L(D)ᵣ. In yet another preferred embodiment, r is 1 to 10, more preferably, r is 1, 2, 3, 4, 5 or 6, even more preferably r is 1, 2, 3 or 4, even more preferably r is 1 or 2, and most preferably is 1. In another preferred embodiment, q is 1 or 2, more preferably q is 1. Even more preferably, r is 1 and q is 1, and most preferably, a' + a" is 5 and r is 1 and q is 1.

The L(D)ᵣ substituent may be present on a C-atom in said (hetero)cycloalkynyl group, or, in case of a heterocycloalkynyl group, on the heteroatom of said heterocycloalkynyl group. When the (hetero)cycloalkynyl group comprises substituents, e.g. an annelated cycloalkyl, the L(D)ᵣ substituent may also be present on said substituents.

The methods to connect a linker L to a (hetero)cycloalkynyl group on the one end and to a molecule of interest on the other end, in order to obtain a linker-conjugate, depend on the exact the nature of the linker, the (hetero)cycloalkynyl group and the molecule of interest. Suitable methods are known in the art.

Preferably, the linker-conjugate comprises a (hetero)cyclooctyne group, more preferably a strained (hetero)cyclooctyne group. Suitable (hetero)cycloalkynyl moieties are known in the art. For example DIFO, DIFO2 and DIFO 3 are disclosed in US 2009/0068738. DIBO is disclosed in WO 2009/067663. BARAC is disclosed in J. Am. Chem. Soc. 2010, 132, 3688 - 3690 and US 2011/0207147.

Preferred examples of linker-conjugates comprising a (hetero)cyclooctyne group are shown below.

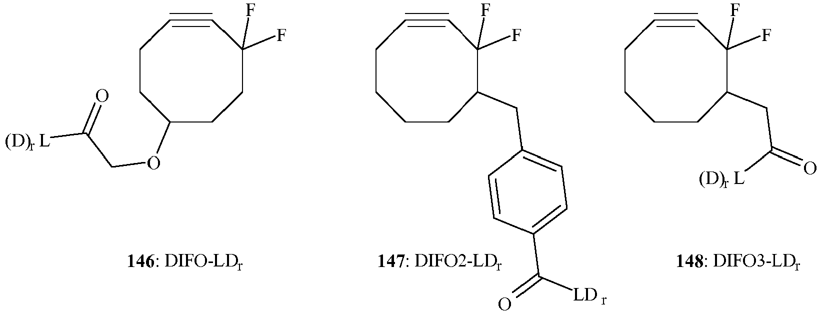

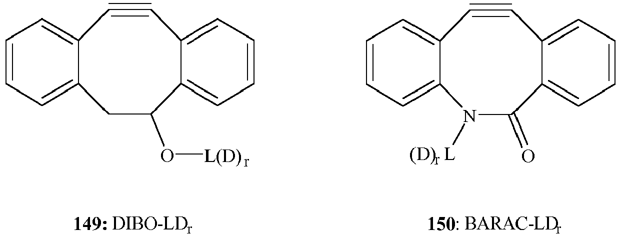

Other cyclooctyne moieties that are known in the art are DIBAC (also known as ADIBO or DBCO) and BCN. DIBAC is disclosed in Chem. Commun. 2010, 46, 97 - 99. BCN is disclosed in WO 2011/136645.

In a preferred embodiment, said linker-conjugate has the Formula **(151):** 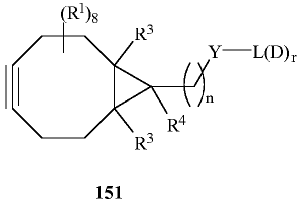 wherein:
R¹, L, D and r are as defined above;
Y is O, S or NR², wherein R² is as defined above;
R³ is independently selected from the group consisting of hydrogen, halogen, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups;
R⁴ is selected from the group consisting of hydrogen, Y-L(D)ᵣ, -(CH₂)ₙ-Y-L(D)ᵣ, halogen, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups, the alkyl groups optionally being interrupted by one of more hetero-atoms selected from the group consisting of O, N and S, wherein the alkyl groups, (hetero)aryl groups, alkyl(hetero)aryl groups and (hetero)arylalkyl groups are independently optionally substituted; and
n is 1, 2, 3, 4, 5, 6, 7, 8, 9 or 10.

In a further preferred embodiment, R¹ is hydrogen. In another preferred embodiment, R³ is hydrogen. In another preferred embodiment, n is 1 or 2. In another preferred embodiment, R⁴ is hydrogen, Y-L(D)ᵣ or -(CH₂)ₙ-Y-L(D)ᵣ. In another preferred embodiment, R² is hydrogen or L(D)ᵣ. In a further preferred embodiment, the linker-conjugate has the Formula **(152):** 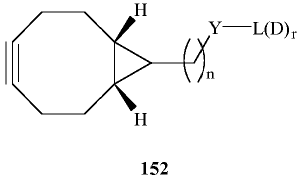 wherein Y, L, D, n and r are as defined above. Preferably, r is 1 or 2, more preferably r is 1.

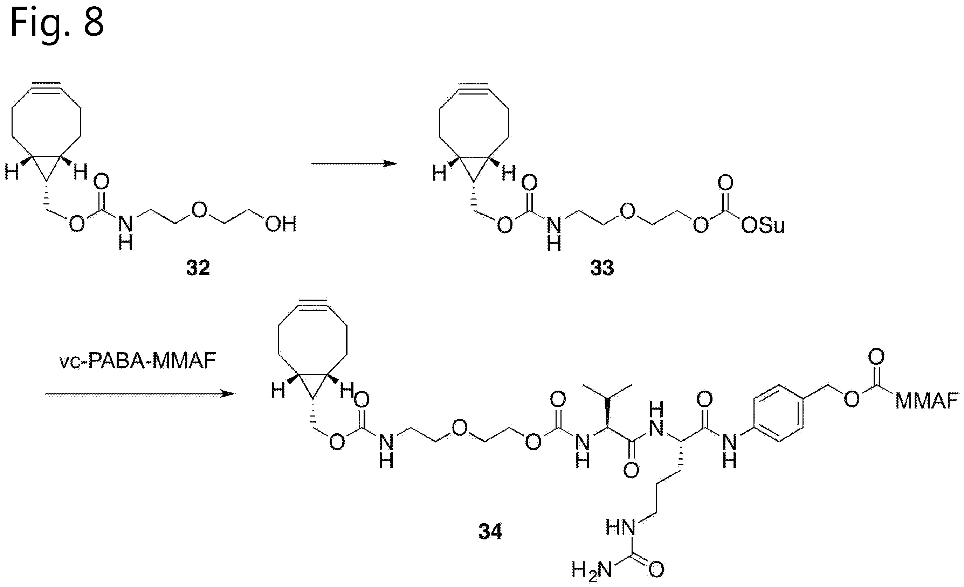

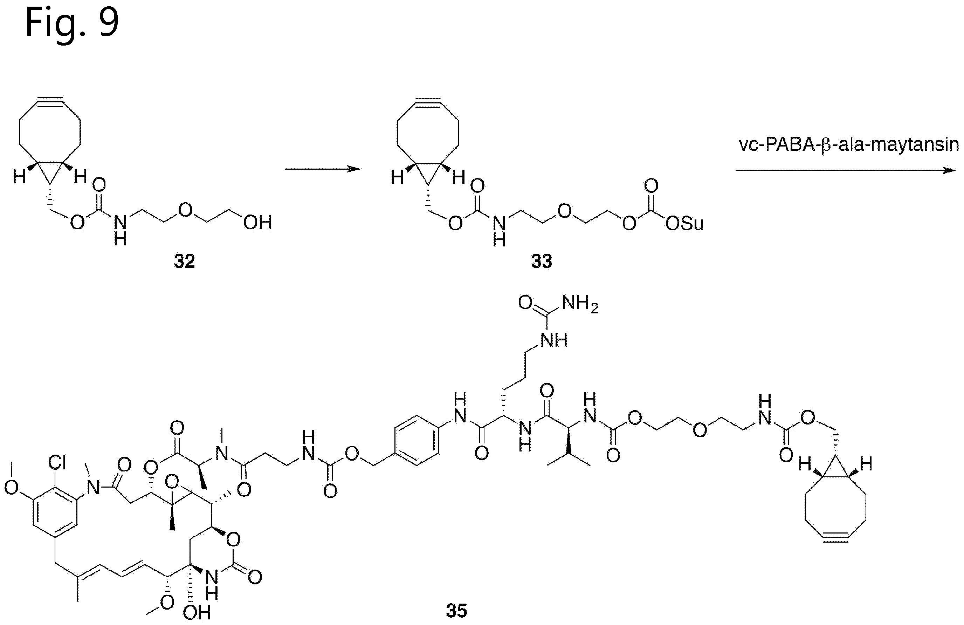

Examples of a linker-conjugate according to formula **(152)** are shown in Figure 8 and Figure 9. Figure 8 shows the reaction scheme for the synthesis of BCN-MMAF conjugate **(34).** Figure 9 shows the reaction scheme for the synthesis of BCN-maytansinoid conjugate **(35).**

In another preferred embodiment, said linker-conjugate has the Formula **(153):** 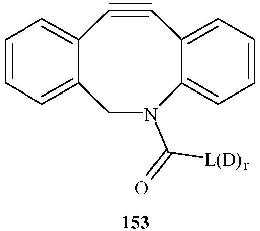 wherein L, D and r are as defined above. Preferably, r is 1 or 2, more preferably r is 1.

In a preferred embodiment of the process for the preparation of an antibody-conjugate according to the invention, an azide-modified antibody is conjugated to a linker-conjugate according to formula **(142a), (142b), (146), (147), (148), (149), (150), (151), (152)** or **(153),** or to a linker-conjugate according to formula (142a), (146), **(147), (148), (149), (150), (151), (152)** or **(153).** Preferably, the linker-conjugate is according to formula **(142a), (142b), (151), (152)** or **(153),** or according to formula **(142a), (151), (152)** or **(153).** In a further preferred embodiment, the linker-conjugate is according to formula **(151), (152)** or **(153),** more preferably (152) or **(153)** and even more preferably **(152).**

As described above, in a preferred embodiment the modified antibody is a thiol-modified antibody and functional group B comprises an N-maleimide group or a halogenated acetamido group.

In a specific embodiment of the process for the preparation of an antibody-conjugate, said process comprises the steps of:
(1) providing an IgG antibody comprising one N-linked glycosylation site on the combination of a single heavy chain and single light chain, wherein the N-linked glycosylation site is a mutant N-linked glycosylation site as compared to its wild type counterpart; and
(2) trimming the oligosaccharide that is attached to said glycosylation site, by the action of an endo-β-N-acetylglucosaminidase, in order to obtain a proximal N-linked GlcNAc-residue at said glycosylation site; and
(3) attaching a monosaccharide derivative Su(A)ₓ to said proximal N-linked GlcNAc-residue, in the presence of a suitable catalyst; wherein Su(A)ₓ is defined as a monosaccharide derivative Su comprising x functional groups A wherein x is 1, 2, 3 or 4 and wherein A is selected from the group consisting of an azido group, a keto group, an alkynyl group, a thiol group or a precursor thereof, a halogen, a sulfonyloxy group, a halogenated acetamido group, a mercaptoacetamido group and a sulfonylated hydroxyacetamido group;
(4) reacting the proximal N-linked GlcNAc-Su(A)ₓ substituent with a linker-conjugate, wherein said linker-conjugate comprises a functional group (B) and a molecule of interest (D), wherein said functional group (B) is a functional group that is capable of reacting with a functional group (A) of said GlcNAc-Su(A)ₓ substituent, and wherein Su(A)ₓ is defined as above, with the proviso that A is not a thiol group precursor; and
wherein the proximal N-linked GlcNAc-residue in steps (2), (3) and (4) is optionally fucosylated.

Steps (1), (2), (3) and (4) of this process are described in detail above.

### Antibody-conjugate

The present invention further relates to an antibody-conjugate obtainable by the process for the preparation of an antibody-conjugate according to the invention.

In a preferred embodiment of the antibody-conjugate according to the invention, x is 1 or 2, more preferably x is 1.

In a preferred embodiment, the antibody-conjugate is obtained by reaction of a thiol-modified antibody with a linker-conjugate comprising a maleimide functional group B. In a further preferred embodiment, the antibody-conjugate is according to formula **(154):**

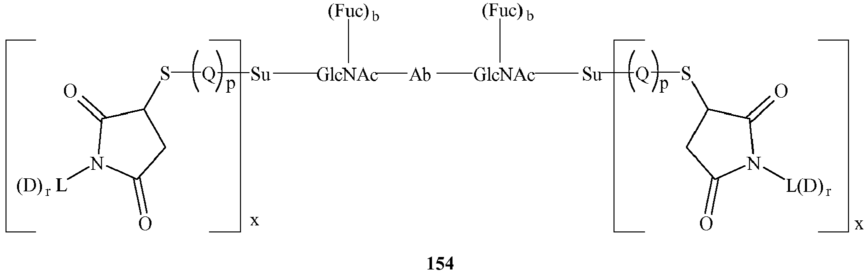

In another preferred embodiment, the antibody-conjugate is obtained by reaction of a thiol-modified antibody with a linker-conjugate comprising a functional group B that comprises a halogenated acetamido group. In a further preferred embodiment, the antibody-conjugate is according to formula **(155):** 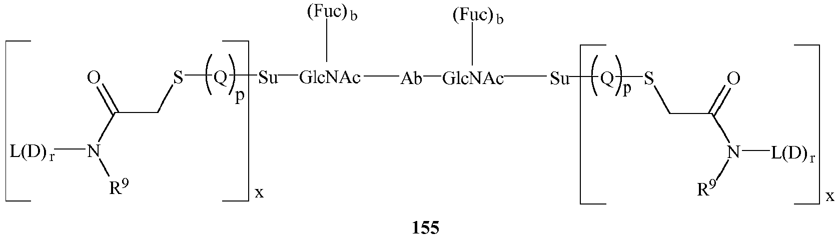 wherein:
Ab is an antibodyt;
Su is a sugar derivative;
L is a linker;
D is a molecule of interest;
b is 0 or 1;
r is 1 to 20;
x is 1, 2, 3 or 4;
p is 0 or 1;
Q is -N(H)C(O)CH₂- or -CH₂-; and
R⁹ is selected from the group consisting of L(D)ᵣ, hydrogen, C₁ - C₂₄ alkyl groups, C₆ - C₂₄ aryl groups,
C₇ - C₂₄ alkylaryl groups and C₇ - C₂₄ arylalkyl groups, the C₁ - C₂₄ alkyl groups, C₆ - C₂₄ aryl groups,
C₇ - C₂₄ alkylaryl groups and C₇ - C₂₄ arylalkyl groups optionally being substituted. Preferably, R⁹ is selected from the group consisting of L(D)ᵣ, hydrogen, C₁ - C₁₂ alkyl groups, C₆ - C₁₂ aryl groups, C₇ - C₁₂ alkylaryl groups and C₇ - C₁₂ arylalkyl groups, the C₁ - C₁₂ alkyl groups, C₆ - C₁₂ aryl groups, C₇ - C₁₂ alkylaryl groups and C₇ - C₁₂ arylalkyl groups optionally being substituted. More preferably, R⁹ is selected from the group consisting of L(D)ᵣ, hydrogen, C₁ - C₆ alkyl groups, C₆ - C₁₂ aryl groups,
C₇ - C₁₂ alkylaryl groups and C₇ - C₁₂ arylalkyl groups, the C₁ - C₆ alkyl groups, C₆ - C₁₂ aryl groups, C₇ - C₁₂ alkylaryl groups and C₇ - C₁₂ arylalkyl groups optionally being substituted. Even more preferably, R⁹ is H, C₁, C₂, C₄ or C₄ alkyl or C₆ - C₁₂ aryl. Most preferably, R⁹ is H or methyl.

In another preferred embodiment, the antibody-conjugate is obtained by reaction of an azide-modified antibody with a linker-conjugate comprising a functional group B that comprises a (hetero)cyclooctyne group, preferably a linker-conjugate according to formula (142a) or (142b). In a further preferred embodiment, the antibody-conjugate is according to formula (156): 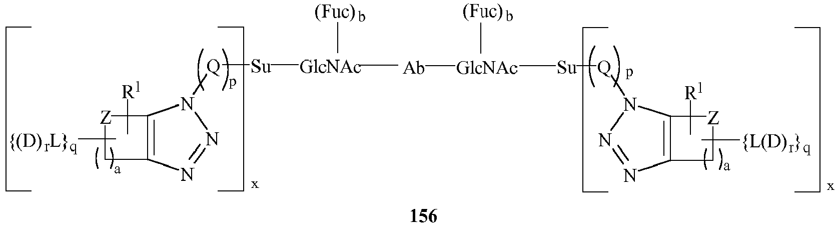 wherein:
Ab is an antibody;
Su is a sugar derivative;
L is a linker;
D is a molecule of interest;
b is 0 or 1;
r is 1 - 20;
x is 1, 2, 3 or 4;
y is y is 1 - 20;
R¹ is independently selected from the group consisting of hydrogen, halogen, -OR⁵, -NO₂, -CN, -S(O)₂R⁵, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups and wherein the alkyl groups, (hetero)aryl groups, alkyl(hetero)aryl groups and (hetero)arylalkyl groups are optionally substituted, wherein two substituents R¹ may be linked together to form an annelated cycloalkyl or an annelated (hetero)arene substituent, and wherein R⁵ is independently selected from the group consisting of hydrogen, halogen, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups;
Z is C(R¹)₂, O, S or NR², wherein R² is R¹ or L(D)ᵣ, and wherein L, D and r are as defined above;
p is 0 or 1;
Q is -N(H)C(O)CH₂- or -CH₂-;
q is 0 or 1, with the proviso that if q is 0 then Z is N-L(D)ᵣ;
a is 1, 2, 3, 4, 5, 6, 7, 8, 9 or 10.

In another further preferred embodiment, the antibody-conjugate is according to formula **(157):** 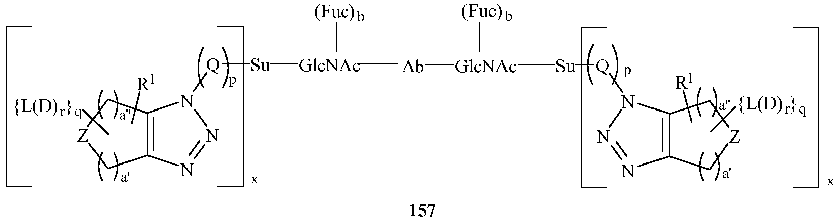 wherein:
Ab is an antibody;
Su is a sugar derivative;
L is a linker;
D is a molecule of interest;
b is 0 or 1;
r is 1 - 20;
x is 1, 2, 3 or 4;
y is y is 1 - 20;
R¹ is independently selected from the group consisting of hydrogen, halogen, -OR⁵, -NO₂, -CN, -S(O)₂R⁵, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups and wherein the alkyl groups, (hetero)aryl groups, alkyl(hetero)aryl groups and (hetero)arylalkyl groups are optionally substituted, wherein two substituents R¹ may be linked together to form an annelated cycloalkyl or an annelated (hetero)arene substituent, and wherein R⁵ is independently selected from the group consisting of hydrogen, halogen, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups;
Z is C(R¹)₂, O, S or NR², wherein R² is R¹ or L(D)ᵣ, and wherein L, D and r are as defined above;
p is 0 or 1;
Q is -N(H)C(O)CH₂- or -CH₂-;
q is 0 or 1, with the proviso that if q is 0 then Z is N-L(D)ᵣ;
a' is 0, 1, 2, 3, 4, 5, 6, 7 or 8;
a" is 0,1, 2, 3, 4, 5, 6, 7 or 8; and
a'+a"< 10.

In a further preferred embodiment, the antibody-conjugate according to formula **(156)** or **(157)** is obtained via reaction of an azide-modified antibody with a linker-conjugate comprising a cyclooctyne group functional group according to formula **(142a)** or **(142b), (146), (147), (148), (149), (150), (151), (152)** or **(153),** more preferably according to formula **(146), (147), (148), (149), (150), (151), (152)** or **(153).** Preferably, the linker-conjugate is according to formula **(142a)** or **(142b), (151), (152)** or **(153),** more preferably the linker-conjugate is according to formula **(151), (152)** or **(153),** more preferably **(152)** or **(153)** and even more preferably **(152).**

In a further preferred embodiment, x in antibody-conjugates according to formula **(154), (155), (156)** and **(157)** is 1 or 2, more preferably x is 1. In a further preferred embodiment, r is 1 or 2, preferably 1. In a further preferred embodiment, x is 1 and r is 1 or 2, more preferably x is 1 and r is 1. In another preferred embodiment, x is 2 and r is 1 or 2, more preferably x is 2 and r is 1.

### Antibody-drug conjugate

In a particularly preferred embodiment of the antibody-conjugate according to the invention, the molecule of interest is selected from the group of pharmaceutically active substances. In a further preferred embodiment the active substance is selected from the group of drugs and prodrugs. Even more preferably, the molecule of interest is selected from the group consisting of low to medium molecular weight compounds. More preferably, the molecule of interest is selected from the group consisting of cytotoxins, antiviral agents, antibacterial agents, peptides and oligonucleotides, and most preferably the molecule of interest is a cytotoxin. In a further preferred embodiment, the molecule of interest is selected from the group consisting of colchicine, vinca alkaloids, camptothecins, doxorubicin, daunorubicin, taxanes, calicheamycins, tubulysins, irinotecans, an inhibitory peptide, amanitin, deBouganin, duocarmycins, maytansines, auristatins or pyrrolobenzodiazepines (PBDs). In a preferred embodiment, the cytotoxin is selected from the group consisting of camptothecins, doxorubicin, daunorubicin, taxanes, calicheamycins, duocarmycins, maytansines, auristatins and pyrrolobenzodiazepines (PBDs). In another preferred embodiment, the cytotoxin is selected from the group consisting of colchicine, vinca alkaloids, tubulysins, irinotecans, an inhibitory peptide, amanitin and deBouganin.

When the molecule of interest in the antibody-conjugate according to the invention is an active substance, the antibody-conjugate may also be referred to as "antibody-drug conjugate" (ADC).

As an example, Figure 11(B) shows the mass spectral profile of an antibody drug conjugate according to the invention, resulting from conjugation of azide-charged trastuzumab mutant N297Q, L196N after treatment with BCN-vc-PABA-MMAF 34.

The invention further relates to an antibody-conjugate according to the invention, wherein the molecule of interest is an active substance, for use as a medicament.

The invention also relates to the use of an antibody-conjugate according to the invention, wherein the molecule of interest is an active substance, for use in the treatment of cancer.

The invention further relates to an antibody-conjugate according to the invention, wherein the molecule of interest is an active substance, for use in the treatment of breast cancer, more preferably for use in the treatment of HER2-positive breast cancer.

The invention also relates to a method treating cancer by administering an antibody-drug conjugate according to the invention.

The invention also relates to a method treating breast cancer by administering an antibody-drug conjugate according to the invention.

The invention also relates to a method treating HER2-positive breast cancer by administering an antibody-drug conjugate according to the invention.

As described above, the antibody-conjugates according to the invention have several advantages over antibody-conjugates known in the prior art. One of the advantages of the modified antibodies, the antibody-conjugates and the process for their preparation according to the invention is that these antibodies and antibody-conjugates are homogeneous, both in site-specificity and stoichiometry. The modified antibodies and antibody-conjugates according to the invention are obtained with a DAR very near to the theoretical value, and with a very low standard deviation. This also means that the antibody-conjugates according to the invention result in a more consistent product for preclinical testing.

The properties of an antibody conjugate according to the invention are modulated by designing, expressing, and processing into antibody-drug conjugates the monoclonal antibodies with different glycosylation profiles. The properties that may be modulated are e.g. anti-tumor activity, the maximum tolerated dose, pharmacokinetics such as plasma clearance, therapeutic index, both in terms of efficacy and toxicity, attenuation of the drug, tendency to aggregate, stability of the attachment of the drug and release of the drug after reaching the target. In particular, there is a correlation between location of drug and the *in vivo* efficacy of an antibody-drug conjugate (ADC).

For example, as described in example 17 and tabulated in Table 2, there is a clear difference in aggregation propensity of different glycomutants, keeping all parameters (antibody, linker, payload) constant, except for the glycosylation site.

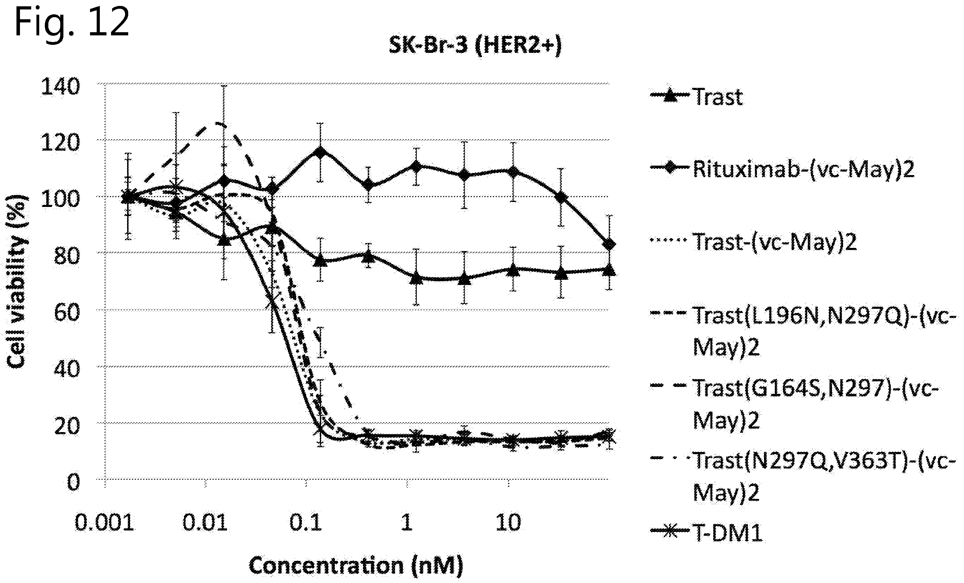

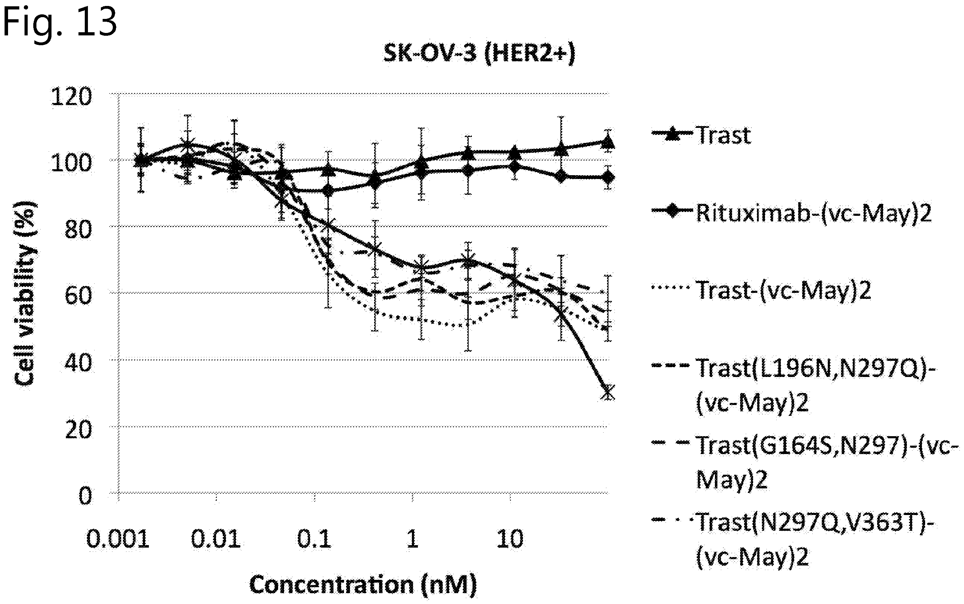

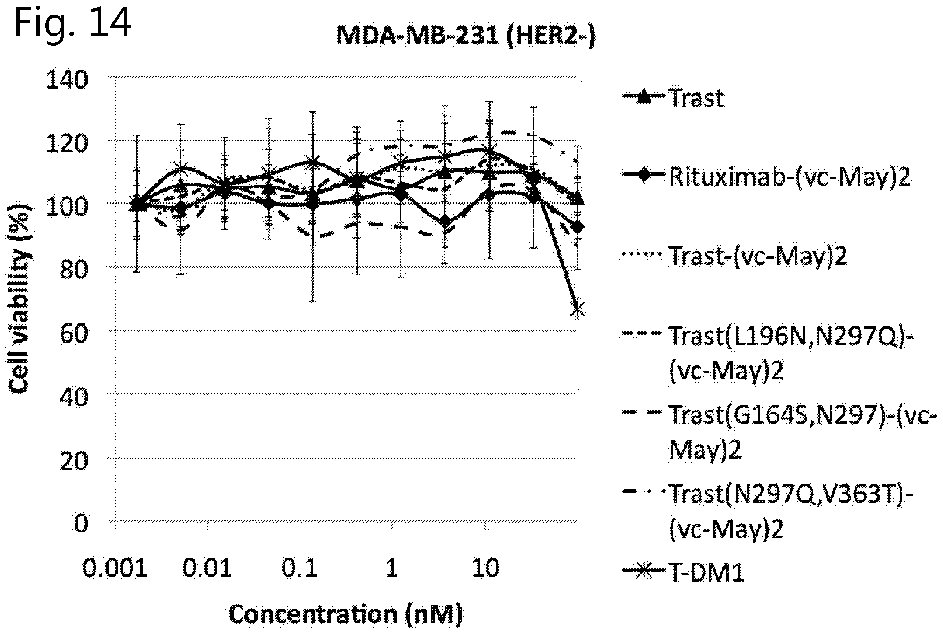

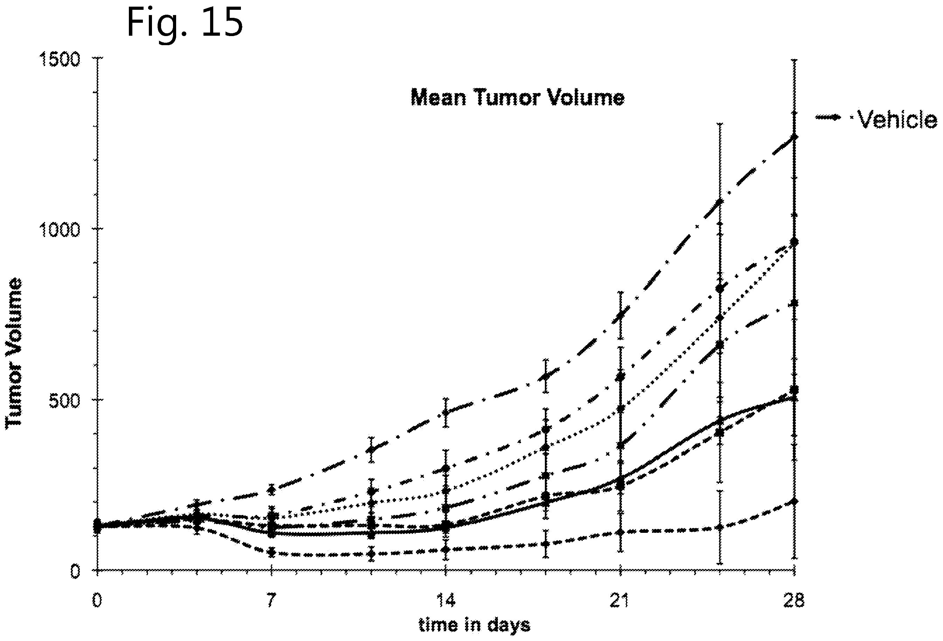

The difference between glycomutants of trastuzumab also becomes apparent from the in vivo efficacy study described in example 18 and graphically illustrated in Figure 15, ranging from little effect for some glycomutants to near tumor erradication with one other glycomutant. An, albeit smaller, difference in efficacy between different glycomutants also become apparent from the in vitro efficacy studies described in example 15 and graphically depicted in Figures 12 and 13, and negative control Figure 14.

### Examples

### Synthesis

### Example 1. Synthesis of BCN-PEG₂-OSu carbonate (33)

A solution of *N*,*N*-disuccinimidyl carbonate (1.82 g, 7.11 mmol) in MeCN (50 mL) was prepared under argon. A solution of BCN-PEG₂-OH (1.0 g, 3.55 mmol) in MeCN (50 mL) was added dropwise over 3 h. After 1 h of additional stirring, the reaction mixture was poured out in a mixture of EtOAc/H₂O (150 mL/150 mL). The layers were separated and the aqueous layer was extracted with EtOAc (150 mL). The combined organic layers were dried (Na₂SO₄) and concentrated. The residue was purified *via* column chromatography and the desired product was obtained as a colorless oil (0.79 g, 2.81 mmol, 79%). ¹H NMR (CDCl₃, 400 MHz) δ (ppm) 5.19 (bs, 1H), 4.50-4.42 (m, 2H), 4.16 (d, *J* = 8.0 Hz, 2H), 3.77-3.71 (m, 2H), 3.57 (t, *J* = 5.1 Hz, 2H), 3.39 (dd, *J* = 10.5, 5.4 Hz, 2H), 2.85 (s, 4H), 2.35-2.16 (m, 6H), 1.65-1.51 (m, 2H), 1.41-1.34 (m, 1H).

### Example 2. Synthesis of BCN-vc-PABA-MMAF (34)

To a solution of Val-Cit-PAB-MMAF.TFA (17.9 mg, 14.3 µmol) in DMF (2 mL) was added BCN-PEG₂-C(O)OSu (17.9 mg, 14.3 µmol) **(33)** as a solution in DMF (0.78 mL) and triethylamine (6.0 µL). The product (7 mg, 5 µmol, 35%) was obtained after purification *via* reversed phase HPLC (C18, gradient H₂O/MeCN 1% AcOH). LRMS (HPLC, ESI+) calcd for C₇₄H₁₁₄N₁₁O₁₈ (*M*+H⁺) 1444.83, found 1445.44.

### Example 3. Synthesis of BCN-vc-PABA-β-ala-maytansinoid (35)

To a suspension of Val-Cit-PABA-β-alaninoyl-maytansinoid (commercially available from Concortis) (27 mg, 0.022 mmol) in MeCN (2 mL) was added triethylamine (9.2 µL, 6.7 mg, 0.066 mmol) and a solution of BCN-PEG₂-OSu carbonate **33** (9.2 mg, 0.022 mmol) in MeCN (1 mL). After 23 h, the mixture was poured out in a mixture of EtOAc (20 mL) and water (20 mL). After separation, the organic phase was dried (Na₂SO₄) and concentrated. After purification *via* column chromatography (EtOAc → MeOH/EtOAc 1/4) 22 mg (0.015 mmol, 70%) of the desired product **35** was obtained. LRMS (ESI+) calcd for C₇₀H₉₇ClN₁₀O₂₀ (*M*+H⁺) 1432.66, found 1434.64.

### Antibody glycosylation mutants

Both native trastuzumab and mutant antibodies were transiently expressed in CHO K1 cells by Evitria (Zurich, Switzerland), purified using protein A sepharose and analyzed by mass spectrometry.

Specific mutants of trastuzumab were derived from literature (Qu et al., J. Immunol. Methods 1998, 213, 131), in particular L196N and G164S) or *de novo* designed, in particular V363T. In all three cases, a second mutation involved the mutation of asparagine 297 to glutamine (N297Q) to remove the native glycosylation site. All mutations were localized on the heavy chain of trastuzumab.

### General protocol for mass spectral analysis of IgG

A solution of 50 µg (modified) IgG, 1 M Tris-HCl pH 8.0, 1 mM EDTA and 30 mM DTT with a total volume of approximately 70 µL was incubated for 20 minutes at 37 °C to reduce the disulfide bridges allowing to analyze both light and heavy chain. If present, azide-functionalities are reduced to amines under these conditions. Reduced samples were washed trice with milliQ using an Amicon Ultra-0.5, Ultracel-10 Membrane (Millipore) and concentrated to 10 µM (modified) IgG. The reduced IgG was analyzed by electrospray ionization time-of-flight (ESI-TOF) on a JEOL AccuTOF. Deconvoluted spectra were obtained using Magtran software.

### General Protocol for trimming of IgG glycans using EndoS

Trimming of IgG glycans was performed using endo S from *Streptococcus pyogenes* (commercially available from Genovis, Sweden). The IgG (10 mg/mL) was incubated with endo S (40 U/mL final concentration) in 25 mM Tris pH 8.0 for 16 hours at 37 °C.

### General Protocol for trimming of IgG glycans using EndoF2 or EndoF3

Trimming of IgG glycans was performed using endoF2 from *Elizabethkingia miricola* (commercially available from QA Bio) or endoF3 from *Elizabethkingia meningosepticum* (commercially available from QA Bio). The IgG (10 mg/mL) was incubated with endo F2 (100 mU/mg IgG) or EndoF3 (25 mU/mg IgG) in 100 mM sodium Citrate pH 4.5 for 16 hours at 37 °C. The deglycosylated IgG was concentrated and washed with 25 mM Tris-HCl pH 8.0 using an Amicon Ultra-0.5, Ultracel-10 Membrane (Millipore).

### Example 4. Trimming of native trastuzumab

Trastuzumab with base MS peaks at 50444, 50591 and 50753 and 50914 Da, corresponding to G0, G0F, G1F and G2F isoforms of glycosylation, was subjected to the trimming protocol with endo S above. After deconvolution of peaks, the mass spectrum showed one peak of the light chain and two peaks of the heavy chain. The two peaks of heavy chain belonged to one major product (49496 Da, 90% of total heavy chain), resulting from core GlcNAc(Fuc)-substituted trastuzumab, and a minor product (49351 Da, ±10% of total heavy chain), resulting from core GlcNAcsubstituted trastuzumab.

### Example 5. Trimming of trastuzumab-mutant N297Q,L196N

Trastuzumab-(N297Q, L196N) mutant with major base MS peaks at 50934, 51225 and 51516 Da, corresponding to G2F, G2FS and G2FS2 glycosylation isoforms, was subjected to the trimming protocol with endo F3 as described above. After deconvolution of peaks, the mass spectrum showed one peak of the light chain and one major peak of the heavy chain (49514 Da, 90% of total heavy chain), resulting from core GlcNAc(Fuc)-substituted trastuzumab-(N297Q, L196N) mutant. Incubation with endo F2 instead of endo F3 as described above gave the identical core GlcNAc(Fuc)-substituted heavy chain product. On the other hand, trastuzumab-(N297Q, L196N) mutant was completely inert to hydrolysis by the action of endo S.

### Example 6. Trimming of trastuzumab-mutant N297Q,G164S

Trastuzumab-(N297Q, G164S) mutant with major base MS peaks at 50961, 51251 and 51542 Da, corresponding to G2F, G2FS and G2FS2 glycosylation isoforms, was subjected to the trimming protocol using EndoF3 as described above. After deconvolution of peaks, the mass spectrum showed one peak of the light chain and one major peak of the heavy chain (49537 Da, 90% of total heavy chain), resulting from core GlcNAc(Fuc)-substituted trastuzumab-(N297Q, G164S).

### Example 7. Trimming of trastuzumab-mutant N297Q, V363T

Trastuzumab-(N297Q, V363T) mutant with major base MS peaks at 50934, 51227 and 51517 Da, corresponding to G2F, G2FS and G2FS2 glycosylation isoforms, was subjected to the trimming protocol with EndoF3 as described above. After deconvolution of peaks, the mass spectrum showed one peak of the light chain and one peak of the light chain and one major peak of the heavy chain (49515 Da, 90% of total heavy chain), resulting from core GlcNAc(Fuc)-substituted trastuzumab-(N297Q, V363T) mutant. Also incubation with EndoF2 as described above gave the core GlcNAc(Fuc)-substituted heavy chain product.

### General protocol for glycosyltransfer of modified sugar to IgG

Enzymatic introduction of modified sugar onto IgG was effected with a mutant of bovine β(1,4)-galactosyltransferase, β(1,4)-Gal-T1(Y289L. The deglycosylated IgG (prepared as described above, 10 mg/mL) was incubated with a modified UDP-sugar derivative (0.4 mM) from one of the UDP-sugars **28-31** and β(1,4)-Gal-T1(Y289L) (1 mg/mL) in 10 mM MnCl₂ and 25 mM Tris-HCl pH 8.0 for 15 hours at 30 °C.
The modified IgG was incubated with protein A agarose (40 µL per mg IgG) for 2 hours at 4 °C. The protein A agarose was washed three times with PBS and the IgG was eluted with 100 mM glycine-HCl pH 2.7. The eluted IgG was neutralized with 1 M Tris-HCl pH 8.0 and concentrated and washed with PBS using an Amicon Ultra-0.5, Ultracel-10 Membrane (Millipore) to a concentration of 10 mg/mL.

### Example 8. Glycosyltransfer of UDP-sugar 28 (UDP-GalNAz) to deglycosylated trastuzumab

Deglycosylated trastuzumab was subjected to the glycosyltransfer protocol described above with UDP-GalNAz **28.** After protein A affinity purification, mass spectral analysis indicated the formation of a one major product of (49713 Da, 90% of total heavy chain), resulting from GalNAz transfer to core GlcNAc(Fuc) substituted trastuzumab, and a minor product (49566 Da, ±10% of total heavy chain), resulting from GalNAz transfer to core GlcNAc substituted trastuzumab.

### Example 9. Glycosyltransfer of UDP-sugar 28 (UDP-GalNAz) to deglycosylated trastuzumab mutants

Trastuzumab-(N297Q, L196N), trastuzumab-(N297Q, G164S) and trastuzumab-(N297Q, V363T) were subjected to the glycosyltransfer protocol described above with UDP-GalNAz **28.** One major peak of the heavy chain was observed for trastuzumab-(N297Q, L196N) (49733 Da, 90% of total heavy chain), trastuzumab-(N297Q, G164S) (49757, 90% of total heavy chain) and trastuzumab-(N297Q, V363T) (49734 Da, 90% of total heavy chain). For all three mutants this corresponds to the core GlcNAc(Fuc)GalNaz-substituted heavy chain.

### Example 10. Glycosyltransfer of UDP-sugar 29 (UDP-GalNAcSAc) to deglycosylated trastuzumab

Trimmed trastuzumab (250 µL, 10 mg/mL, 16.5 nmol) was incubated with UDP-GalNAcSAc **(29)** (18.5 µL, 10 mM) and β(1,4)-Gal-T1(Y289L) (12.5 µL, 2 mg/mL) in 10 mM MnCl₂ and 25 mM Tris-HCl pH 6.0 for 16 hours at 30 °C. The crude mixture was purified with ProtA and in order to maintain the low pH the column was washed with 25 mM Tris-HCl pH 6.0 and after elution with glycine.HCl buffer (pH 2.7, 0.1 M), the elution buffer was neutralized with Tris-HCl (pH 7.2, 1 M).
Via this protocol 1.1 mg of an IgG was obtained, AccuTOF analysis of which showed the 95% conversion of trast-(GalNAcSH)₂ into a single desired product (GalNAcSAc minus the acetate group, probably due to *in situ* deprotection with DTT (mass 49729, expected mass 49730). The other 5% was remaining starting material (mass 49494).

### Example 11. Glycosyltransfer of UDP-sugar 29 (UDP-GaINAcSAc) to deglycosylated trastuzumab(N297Q, V363T)

Trimmed trastuzumab(N297Q,V363T) (100 µL, 10 mg/mL, 6.6 nmol) was incubated with UDP-GalNAcSAc **(29)** (14 µL, 10 mM) and β(1,4)-Gal-T1(Y289L) (10 µL, 2 mg/mL) in 10 mM MnCl₂ and 25 mM Tris-HCl pH 6.0 for 16 hours at 30 °C. The crude mixture was purified with ProtA and in order to maintain the low pH the column was washed with 25 mM Tris-HCl pH 6.0 and after elution with glycine.HCl buffer (pH 2.7, 0.1 M), the elution buffer was neutralized with Tris-HCl (pH 7.2, 1 M). Via this protocol 0.25 mg of an IgG was obtained, AccuTOF analysis of which showed the presence of trast(N297Q,V363T)-(GalNAcSH)₂ as a single desired product (GalNAcSAc minus the acetate group, probably due to *in situ* deprotection with DTT (mass 49744, expected mass 49748).

### Example 12. Glycosyltransfer of UDP-sugar 30 (UDP-GalNAcCl) to deglycosylated trastuzumab

Trimmed trastuzumab (100 µL, 10 mg/mL, 6.6 nmol) was incubated with UDP-GalNAcCl **(30)** (5 µL, 10 mM) and β(1,4)-Gal-T1(Y289L) (5 µL, 2 mg/mL) in 10 mM MnCl₂ and 25 mM Tris-HCl pH 6.0 for 16 hours at 30 °C. The crude mixture was purified with ProtA according to the protocol for GalNAcSAc to afford trast-(GalNAcCl)₂ (0.36 mg). AccuTOF analysis showed complete conversion to the desired product (mass 49731, expected mass 49732).

### Example 13. Glycosyltransfer of UDP-sugar 30 (UDP-GalNAcCl) to deglycosylated trastuzumab(N297Q, V363T)

Trimmed trastuzumab N297Q V363T mutant (100 µL, 10 mg/mL, 6.6 nmol) was incubated with UDP-GalNAcCl **(30)** (5 µL, 10 mM) and β(1,4)-Gal-T1(Y289L) (5 µL, 2 mg/mL) in 10 mM MnCl₂ and 25 mM Tris-HCl pH 6.0 for 16 hours at 30 °C. The crude mixture was purified with ProtA according to the protocol for GalNAcSAc to afford trast-(GalNAcCl)₂ (0.35 mg). AccuTOF analysis showed complete conversion to the desired product (mass 49750, expected mass 49750).

### Example 14. Conjugation of BCN-vc-PABA-Maytansinoid 35 to trastuzumab mutants

After protein A purification trastuzumab-(N297Q, L196N), trastuzumab-(N297Q, G164S) and trastuzumab-(N297Q, V363T) were conjugated to BCN-vc-PABA-maytansinoid **35.** All three trastuzumab mutants were concentrated to 100 µM in PBS, added to BCN-vc-PABA-maytansinoid (4 eq in DMF) and incubated overnight at room temperature. To obtain at least 90% conversion, this step was repeated 5 times to obtain trastuzumab(L196N,N297Q)-(vc-PABA-maytansinoid)₂ (heavy chain products between 51100 and 51500 Da with major peak of 51187 Da corresponding to heavy chain conjugated to BCN-vc-PABA-maytansinoid, ±95% of total heavy chain), trastuzumab(*N297Q*, *V3631*)-(vc-PABA-maytansinoid)₂ (heavy chain products between 51100 and 51500 Da with major peak of 51189 Da corresponding to heavy chain conjugated to BCN-vc-PABA-maytansinoid, ±80% of total heavy chain) and trastuzumab(G164S,*N297Q*)-(vc-PABA-maytansinoid)₂ (heavy chain products between 51100 and 51500 Da with major peak of 51218 Da corresponding to heavy chain conjugated to BCN-vc-PABA-maytansinoid, ±90% of total heavy chain).

### Example 15: In vitro efficacy

SK-Br-3 (Her2+), SK-OV-3 (Her2+) and MDA-MB-231 (Her2-) cells were plated in 96-wells plates (5000 cells/well) in RPMI 1640 GlutaMAX (Invitrogen) supplemented with 10% fetal calf serum (FCS) (Invitrogen, 200 µL/well) and incubated overnight at 37°C and 5% CO₂. A three-fold dilution series (ranging from ±0.002 to 100 nM) of the sterile-filtered compounds was prepared in RPMI 1640 GlutaMAX supplemented with 10% FCS. After removal of the culture medium, the concentration series were added in quadruplo and incubated for three days at 37°C and 5% CO₂. The culture medium was replaced by 0.01 mg/mL resazurin (Sigma Aldrich) in RPMI 1640 GlutaMAX supplemented with 10% FCS. After 4 to 6 hours at 37°C and 5% CO₂ fluorescence was detected with a fluorescence plate reader (Tecan Infinite 200) at 540 nm excitation and 590 nm emission. The relative fluorescent units (RFU) were normalized to cell viability percentage by setting wells without cells at 0% viability and wells with lowest dose of compound at 100% viability. For each conditions the average cell viability percentage ± sem is shown.

The in vitro cytotoxicity of trastuzumab-(vc-PABA-maytansinoid)₂, trastuzumab(L196N,N297Q)-(vc-PABA-Maytansinoid)₂, trastuzumab(G164S,N297Q)-(vc-PABA-Maytansinoid)₂, trastuzumab(N297Q,V363T)-(vc-PABA-Maytansinoid)₂ were compared to T-DM1 as a positive control and trastuzumab and rituximab-(vc-PABA-maytansinoid)₂ as negative controls (Figures 12-14). All trastuzumab-based ADCs affect the viability of the Her positive cell lines SK-Br-3 and SK-OV-3, but not of the Her2-negative cell line MDA-MB-231, which shows that these ADCs specifically target Her2 positive cells. In the Her2 negative cell line MDA-MB-231, only T-DM1 shows a slight decrease in cell viability at the highest concentration (100 nM).

### Example 16: Cloning and expression of GalT mutants Y289N, Y289F, Y289M, Y289V, Y289A, Y289G and Y289I.

The GalT mutant genes were amplified from a construct containing the sequence encoding the catalytic domain of GalT consisting of 130-402 aa residues, by the overlap extension PCR method. The wild type enzyme is represented by SEQ ID NO: 17. For Y289N mutant (represented by SEQ ID NO: 18), the first DNA fragment was amplified with a pair of primers: Oligo38_GalT_Extemal_Fw (CAG CGA CAT ATG TCG CTG ACC GCA TGC CCT GAG GAG TCC represented by SEQ ID NO: 1) and Oligo19_GalT_Y289N_Rw (GAC ACC TCC AAA **GTT** CTG CAC GTA AGG TAG GCT AAA represented by SEQ ID NO: 2). The *NdeI* restriction site is underlined, while the mutation site is highlighted in bold. The second fragment was amplified with a pair of primers: Oligo29_GalT_External_Rw (CTG ATG GAT GGA TCC CTA GCT CGG CGT CCC GAT GTC CAC represented by SEQ ID NO: 3) and Oligo18_GalT_Y289N_Fw (CCT TAC GTG CAG **AAC** TTT GGA GGT GTC TCT GCT CTA represented by SEQ ID NO: 4). The *BamHI* restriction site is underlined, while the mutation site is highlighted in bold. The two fragments generated in the first round of PCR were fused in the second round using Oligo38_GalT_External_Fw and Oligo29_GalT_External_Rw primers. After digestion with *NdeI* and *BamHI.* This fragment was ligated into the pET16b vector cleaved with the same restriction enzymes. The newly constructed expression vector contained the gene encoding Y289N mutant and the sequence encoding for the His-tag from pET16b vector, which was confirmed by DNA sequencing results. For the construction of Y289F (represented by SEQ ID NO: 19), Y289M (represented by SEQ ID NO: 20), Y289I (represented by SEQ ID NO: 21), Y289V (represented by SEQ ID NO: 22), Y289A (represented by SEQ ID NO: 23) and Y289G (represented by SEQ ID NO: 24) mutants the same procedure was used, with the mutation sites changed to **TTT, ATG, ATT, GTG, GCG** or **GGC** triplets encoding for phenylalanine, methionine, isoleucine, valine, alanine or glycine, respectively. More specifically, for the construction of Y289F the first DNA fragment was amplified with a pair of primers defined herein as SEQ ID NO: 1 and SEQ ID NO: 5 and the second fragment was amplified with a pair of primers defined herein as SEQ ID NO: 3 and SEQ ID NO: 6 (be referred to Table 1 for the related sequences). Furthermore, for the construction of Y289M the first DNA fragment was amplified with a pair of primers defined herein as SEQ ID NO: 1 and SEQ ID NO: 7 and the second fragment was amplified with a pair of primers defined herein as SEQ ID NO: 3 and SEQ ID NO: 8. For the construction of Y289I the first DNA fragment was amplified with a pair of primers defined herein as SEQ ID NO: 1 and SEQ ID NO: 9 and the second fragment was amplified with a pair of primers defined herein as SEQ ID NO: 3 and SEQ ID NO: 10. For the construction of Y289V the first DNA fragment was amplified with a pair of primers defined herein as SEQ ID NO: 1 and SEQ ID NO: 11 and the second fragment was amplified with a pair of primers defined herein as SEQ ID NO: 3 and SEQ ID NO: 12. for the construction of Y289A the first DNA fragment was amplified with a pair of primers defined herein as SEQ ID NO: 1 and SEQ ID NO: 13 and the second fragment was amplified with a pair of primers defined herein as SEQ ID NO: 3 and SEQ ID NO: 14. For the construction of Y289G the first DNA fragment was amplified with a pair of primers defined herein as SEQ ID NO: 1 and SEQ ID NO: 15 and the second fragment was amplified with a pair of primers defined herein as SEQ ID NO: 3 and SEQ ID NO: 16 (be referred to Table 1 for the related sequences).

GalT mutants were expressed, isolated and refolded from inclusion bodies according to the reported procedure by Qasba et al. (Prot. Expr. Pur. 2003, 30, 219-229). After refolding, the precipitate was removed and the soluble and folded protein was isolated using a Ni-NTA column (HisTrap excel 1 mL column, GE Healthcare). After elution with 25 mM Tris-HCl pH 8.0, 300 mM NaCl and 200 mM imidazole, the protein was dialyzed against 25 mM Tris-HCl pH 8.0 and concentrated to 2 mg/mL using a spinfilter (Amicon Ultra-15 Centrifugal Filter Unit with Ultracel-10 membrane, Merck Millipore).

**Table 1 Sequence identification of the primers used**

| **SEQ ID NO** | **Nucleotide sequence** |
|---|---|
| SEQ ID NO: 1 | CAG CGA CAT ATG TCG CTG ACC GCA TGC CCT GAG GAG TCC |
| SEQ ID NO: 2 | GAC ACC TCC AAA **GTT** CTG CAC GTA AGG TAG GCT AAA |
| SEQ ID NO: 3 | CTG ATG GAT GGA TCC CTA GCT CGG CGT CCC GAT GTC CAC |
| SEQ ID NO: 4 | CCT TAC GTG CAG **AAC** TTT GGA GGT GTC TCT GCT CTA |
| SEQ ID NO: 5 | GAC ACC TCC AAA **AAA** CTG CAC GTA AGG TAG GCT AAA |
| SEQ ID NO: 6 | CCT TAC GTG CAG **TTT** TTT GGA GGT GTC TCT GCT CTA |
| SEQ ID NO: 7 | GAC ACC TCC AAA **CAT** CTG CAC GTA AGG TAG GCT AAA |
| SEQ ID NO: 8 | CCT TAC GTG CAG **ATG** TTT GGA GGT GTC TCT GCT CTA |
| SEQ ID NO: 9 | GAC ACC TCC AAA **AAT** CTG CAC GTA AGG TAG GCT AAA |
| SEQ ID NO: 10 | CCT TAC GTG CAG **ATT** TTT GGA GGT GTC TCT GCT CTA |
| SEQ ID NO: 11 | GAC ACC TCC AAA **CAC** CTG CAC GTA AGG TAG GCT AAA |
| SEQ ID NO: 12 | CCT TAC GTG CAG **GTG** TTT GGA GGT GTC TCT GCT CTA |
| SEQ ID NO: 13 | GAC ACC TCC AAA **CGC** CTG CAC GTA AGG TAG GCT AAA |
| SEQ ID NO: 14 | CCT TAC GTG CAG **GCG** TTT GGA GGT GTC TCT GCT CTA |
| SEQ ID NO: 15 | GAC ACC TCC AAA **GCC** CTG CAC GTA AGG TAG GCT AAA |
| SEQ ID NO: 16 | CCT TAC GTG CAG **GGC** TTT GGA GGT GTC TCT GCT CTA |

### Example 17: Aggregation study of ADCs with different conjugation sites

The effect of the conjugation site of maytansinoid on the aggregation of the corresponding ADCs was studied using five ADCs derived from different trastuzumab variants. These ADCs were prepared using native trastuzumab and four trastuzumab mutants each containing a single glycosylation site on the heavy chain (trastuzumab(G164S, N300Q), trastuzumab(L196N, N300Q), trastuzumab(K291T, N300Q) and trastuzumab(N300Q, V366T). The ADCs were prepared by enzymatic remodelling of the glycan with introduction of GalNAz, followed by conjugation to BCN-maytansin **35.** All ADCs were purified by size-exclusion chromatography on a Superdex200 10/300 GL column (GE Healthcare) to obtain the monomeric fraction. After purification, the ADCs were incubated in phosphate-buffered saline at a concentration of 1 mg/ml at 37 °C. The level of aggregation was analyzed using a Superdex200 PC 3.2/30 column (GE Healthcare) after 0, 1 and 2 weeks. The increase in aggregation after 1 and 2 weeks compared to T0 is shown in Table 2. ADCs derived from trastuzumab(G164S, N300Q) and trastuzumab(L196N, N300Q) show less aggregation compared to the ADC derived from native trastuzumab.

**Table 2. Aggregation studies on ADCs.**

| ADC prepared from | Increase in aggregation (%) | |
|---|---|---|
| | 1 week | 2 weeks |
| trastuzumab | 0.03 | 0.77 |
| trastuzumab(G164S, N300Q) | 0.16 | 0.29 |
| trastuzumab(L196N, N300Q) | -0.12 | -0.05 |
| trastuzumab(N300Q, K291T) | 0.36 | 0.89 |
| trastuzumab(N300Q, V366T) | 0.63 | 0.75 |

### Example 18: In vivo efficacy

Female SHO mice (Crl:SHO-Prkdcscid Hrhr, 6- to 9-week-old at the beginning of the experimental phase, obtained from *Charles River Laboratories,* L'Arbresles, France) were anaesthetized with ketamine/xylazine, the skin was aseptized with a chlorhexidine solution, incised at the level of the interscapular region, a 20 mm³ tumor fragment (HBCx-13B breast cancer patient-derived xenograft model) was placed in the subcutaneous tissue and the skin was closed with clips. When the tumor volume was in the range of 60 to 200 mm³, groups of ten mice were injected i.v. with either vehicle, with trastuzumab-(GalNAz-vc-PABA-maytansin)₂ or with one of the glycosylation mutants trastuzumab(G164S, N300Q), trastuzumab(L196N, N300Q), trastuzumab(K291T, N300Q), trastuzumab(N300Q, V366T), trastuzumab(L177N, N300Q) or trastuzumab (LC-R18N, HC-N300Q), all dosed at 6 mg/kg. Tumors were measured on day 0, 4, 7, 11, 14, 18, 21, 25 and 28.

## Claims

1. Process for the preparation of a modified antibody, the process comprising attaching a monosaccharide derivative Su(A)ₓ to a proximal N-linked GlcNAc-residue, in the presence of a catalyst selected from the group consisting of *β*(1,4)-galactosyltransferases, *β*(1,3)-N-galactosyltransferases, *β*(1,4)-galactosyltransferases comprising a mutant catalytic domain and *β*(1,3)-N-galactosyltransferases comprising a mutant catalytic domain; wherein Su(A)ₓ is defined as a monosaccharide derivative Su comprising x functional groups A wherein x is 1, 2, 3 or 4 and wherein A is selected from the group consisting of an azido group, a keto group, an alkynyl group, a thiol group or a precursor thereof, a halogen, a sulfonyloxy group, a halogenated acetamido group, a mercaptoacetamido group and a sulfonylated hydroxyacetamido group, wherein a precursor of a thiol group is -[C(R⁷)₂]ₒSC(O)CH₃, wherein R⁷ is independently selected from the group consisting of hydrogen, halogen and an optionally substituted C₁ - C₂₄ alkyl group, and o is 0 - 24; wherein said proximal N-linked GlcNAc-residue is attached to the N-linked glycosylation site of an IgG antibody comprising one N-linked glycosylation site on the combination of a single heavy chain and single light chain, wherein said N-linked glycosylation site is a mutant N-linked glycosylation site as compared to its wild type counterpart, and wherein the proximal N-linked GlcNAc-residue is according to Formula (**138**): 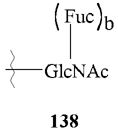 wherein b is 0 or 1.

2. Process according to claim 1, wherein the catalyst is selected from the group consisting of *β*(1,4)-galactosyltransferases comprising a mutant catalytic domain and *β*(1,3)-N-galactosyltransferases comprising a mutant catalytic domain.

3. Process according to claim 1 or claim 2, wherein the catalyst is a catalyst comprising a mutant catalytic domain from a *β*(1,4)-galactosyltransferase, selected from the group consisting of bovine *β*(1,4)-Gal-T1 GalT Y289L, GalT Y289N, GalT Y289I, GalT Y289F, GalT Y289M, GalT Y289V, GalT Y289G and GalT Y289A.

4. Process according to any one of the preceding claims, wherein Su(A)ₓ is selected from the group consisting of GalNAz-UDP, 6-AzGalNAc-UDP, 6-GalNAcCl-UDP, 6-GalNAcSH-UDP, 2-GalNAcCl-UDP, 2-GalNAcSH-UDP, 6-ClGal-UDP, 2-ClGal-UDP, 2-HSGal-UDP and 6-HSGal-UDP.

5. IgG antibody comprising one N-linked glycosylation site on the combination of a single heavy chain and single light chain, wherein the N-linked glycosylation site is a mutant N-linked glycosylation site as compared to its wild type counterpart, wherein a proximal N-linked GlcNAc-Su(A)ₓ-substituent is attached to the antibody, wherein the GlcNAc in the N-linked GlcNAc-Su(A)ₓ-substituent is optionally fucosilated, and wherein Su(A)ₓ is defined as a monosaccharide derivative Su comprising x functional groups A wherein x is 1, 2, 3 or 4 and wherein A is selected from the group consisting of an azido group, a keto group, an alkynyl group, a thiol group or a precursor thereof, a halogen, a sulfonyloxy group, a halogenated acetamido group, a mercaptoacetamido group and a sulfonylated hydroxyacetamido group, wherein a precursor of a thiol group is -[C(R⁷)₂]ₒSC(O)CH₃, wherein R⁷ is independently selected from the group consisting of hydrogen, halogen and an optionally substituted C₁ - C₂₄ alkyl group, and o is 0 - 24.

6. Antibody according to claim 5, wherein the antibody is according to formula (140): 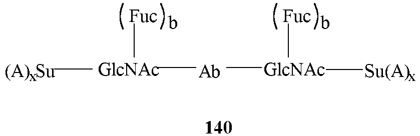 wherein:
Ab represents an IgG antibody comprising one N-linked glycosylation site on the combination of a single heavy chain and single light chain, wherein the N-linked glycosylation site is a mutant N-linked glycosylation site as compared to its wild type counterpart; Su(A) and x are as defined in claim 5; and b is 0 or 1.

7. Antibody according to claim 5 or claim 6, wherein A is an azide group, a thiol group or a halogen.

8. Use of the antibody according to any one of claims 5 - 7 in the preparation of an antibody-conjugate, wherein a antibody-conjugate is defined as an antibody that is conjugated to a molecule of interest D via a linker L.

9. Process for the preparation of an antibody-conjugate, said process comprising reacting a modified antibody according to any one of claims 5 - 7 with a linker-conjugate, wherein said linker-conjugate comprises a functional group B and one or more molecules of interest, wherein said functional group B is a functional group that is capable of reacting with a functional group A on a glycan of the modified glycoprotein, and wherein functional group A is as defined in claim 1.

10. Process according to claim 9, wherein:
(a) when the modified antibody is an azide-modified antibody, the linker-conjugate comprises a (hetero)cycloalkynyl group or an alkynyl group, and one or more molecules of interest; or
(b) when the modified antibody is a keto-modified antibody, the linker-conjugate comprises a primary amine group, an aminooxy group or a hydrazinyl group, and one or more molecules of interest; or
(c) when the modified antibody is an alkyne-modified antibody, the linker-conjugate comprises an azido group, and one or more molecules of interest.
(d) when the modified antibody is a thiol-modified antibody or a mercaptoacetamide-modified antibody, the linker-conjugate comprises an N-maleimide group or a halogenated acetamido group or an alkene, and one or more molecules of interest; or
(e) when the modified antibody is a halogen-modified antibody, a halogenated acetamide-modified antibody, a sulfonyloxy-modified antibody or a sulfonylated hydroxyacetamido-modified antibody, the linker-conjugate comprises a thiol group, and one or more molecules of interest.

11. Antibody-conjugate obtainable by the process according to any one of claims 9 - 10.

12. Antibody-conjugate according to claim 11, wherein the antibody is according to formula (**154**), (**155**), (**156**) or (**157**): 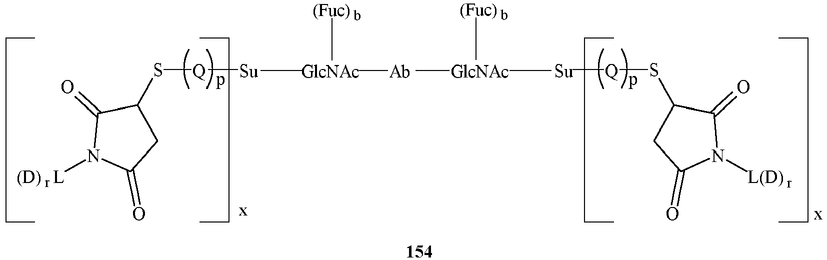 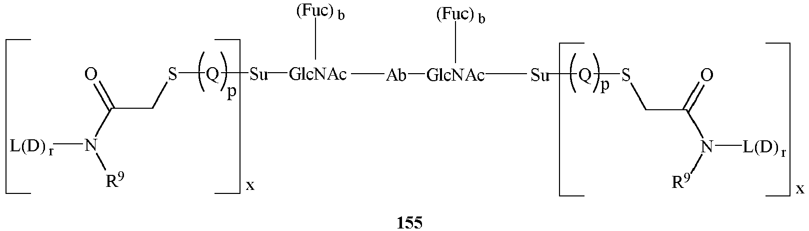 wherein:
Ab is as defined in claim 6;
Su is a sugar derivative;
L is a linker;
D is a molecule of interest;
b is 0 or 1;
r is 1 to 20;
x is 1, 2, 3 or 4;
p is 0 or 1;
Q is -N(H)C(O)CH₂- or -CH₂-; and
R⁹ is selected from the group consisting of L(D)ᵣ, hydrogen, C₁ - C₂₄ alkyl groups, C₆ - C₂₄ aryl groups, C₇ - C₂₄ alkylaryl groups and C₇ - C₂₄ arylalkyl groups, the C₁ - C₂₄ alkyl groups, C₆ - C₂₄ aryl groups, C₇ - C₂₄ alkylaryl groups and C₇ - C₂₄ arylalkyl groups optionally being substituted, preferably R⁹ is selected from the group consisting of L(D)ᵣ, hydrogen, C₁ - C₁₂ alkyl groups, C₆ - C₁₂ aryl groups, C₇ - C₁₂ alkylaryl groups and C₇ - C₁₂ arylalkyl groups, the C₁ - C₁₂ alkyl groups, C₆ - C₁₂ aryl groups, C₇ - C₁₂ alkylaryl groups and C₇ - C₁₂ arylalkyl groups optionally being substituted, more preferably R⁹ is selected from the group consisting of L(D)ᵣ, hydrogen, C₁ - C₆ alkyl groups, C₆ - C₁₂ aryl groups, C₇ - C₁₂ alkylaryl groups and C₇ - C₁₂ arylalkyl groups, the C₁ - C₆ alkyl groups, C₆ - C₁₂ aryl groups, C₇ - C₁₂ alkylaryl groups and C₇ - C₁₂ arylalkyl groups optionally being substituted, even more preferably R⁹ is H, C₁, C₂, C₄ or C₄ alkyl or C₆ - C₁₂ aryl, most preferably, R⁹ is H or methyl; 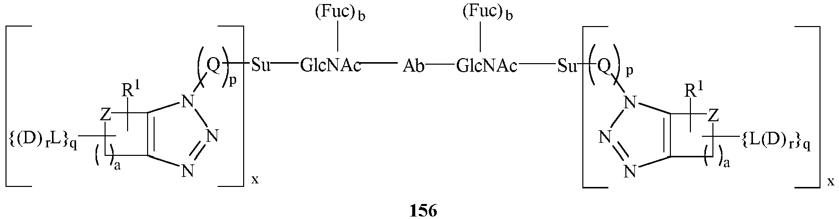
wherein:
Ab is as defined in claim 6;
Su is a sugar derivative;
L is a linker;
D is a molecule of interest;
b is 0 or 1;
r is 1 - 20;
x is 1, 2, 3 or 4;
y is y is 1 - 20;
R¹ is independently selected from the group consisting of hydrogen, halogen, - OR⁵, -NO₂, -CN, -S(O)₂R⁵, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups and wherein the alkyl groups, (hetero)aryl groups, alkyl(hetero)aryl groups and (hetero)arylalkyl groups are optionally substituted, wherein two substituents R¹ may be linked together to form an annelated cycloalkyl or an annelated (hetero)arene substituent, and wherein R⁵ is independently selected from the group consisting of hydrogen, halogen, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups;
Z is C(R¹)₂, O, S or NR², wherein R² is R¹ or L(D)ᵣ, and wherein L, D and r are as defined above;
p is 0 or 1;
Q is -N(H)C(O)CH₂- or -CH₂-;
q is 0 or 1, with the proviso that if q is 0 then Z is N-L(D)ᵣ;
a is 1, 2, 3, 4, 5, 6, 7, 8, 9 or 10; 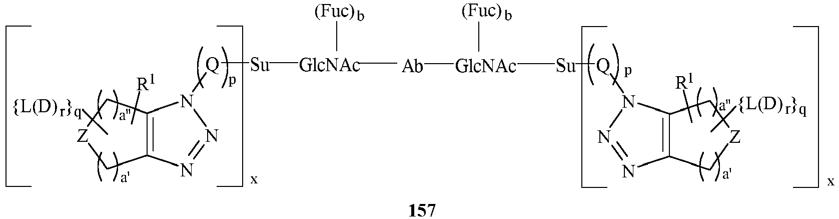
wherein:
Ab is as defined in claim 6;
Su is a sugar derivative;
L is a linker;
D is a molecule of interest;
b is 0 or 1;
r is 1 - 20;
x is 1, 2, 3 or 4;
y is y is 1 - 20;
R¹ is independently selected from the group consisting of hydrogen, halogen, - OR⁵, -NO₂, -CN, -S(O)₂R⁵, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups and wherein the alkyl groups, (hetero)aryl groups, alkyl(hetero)aryl groups and (hetero)arylalkyl groups are optionally substituted, wherein two substituents R¹ may be linked together to form an annelated cycloalkyl or an annelated (hetero)arene substituent, and wherein R⁵ is independently selected from the group consisting of hydrogen, halogen, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups; Z is C(R¹)₂, O, S or NR², wherein R² is R¹ or L(D)ᵣ, and wherein L, D and r are as defined above;
p is 0 or 1;
Q is -N(H)C(O)CH₂- or -CH₂-;
q is 0 or 1, with the proviso that if q is 0 then Z is N-L(D)ᵣ;
a' is 0, 1, 2, 3, 4, 5, 6, 7 or 8;
a" is 0,1, 2, 3, 4, 5, 6, 7 or 8; and
a' + a" < 10.

13. Antibody-conjugate according to any one of claims 11 - 12, wherein x is 1 or 2, and/or r is 1 or 2.

14. Antibody-conjugate according to any one of claims 11 - 13, wherein the molecule of interest is selected from the group of pharmaceutically active substances.

15. Antibody-conjugate according to any one of claims 11 - 14, for use as a medicament.

## Patentansprüche

1. Verfahren zur Herstellung eines modifizierten Antikörpers, wobei das Verfahren das Anhängen eines Monosaccharid-Derivats Su(A)ₓ an einen proximalen N-gebundenen GIcNAc-Rest in Gegenwart eines Katalysators, ausgewählt aus der Gruppe bestehend aus β(1,4)-Galactosyltransferasen, β(1,3)-N-Galactosyltransferasen, β(1,4)-Galactosyltransferasen umfassend eine mutierte katalytische Domäne, und β(1,3)-Galactosyltransferasen umfassend eine mutierte katalytische Domäne; wobei Su(A)ₓ definiert ist als ein Monosaccharid-Derivat Su, umfassend x funktionelle Gruppen A, wobei x 1, 2, 3 oder 4 ist und wobei A ausgewählt ist aus der Gruppe, bestehend aus einer Azidogruppe, einer Ketogruppe, einer Alkinylgruppe, einer Thiolgruppe oder einem Vorläufer davon, einem Halogen, einer Sulfonyloxygruppe einer halogenierten Acetamidogruppe, einer Mercaptoacetamidogruppe und einer sulfonylierten Hydroxyacetamidogruppe, wobei ein Vorläufer einer Thiolgruppe -[C(R⁷)₂]ₒSC(O)CH₃ ist, wobei R⁷ unabhängig ausgewählt ist aus der Gruppe bestehend aus Wasserstoff, Halogen und einer optional substituierten C₁ - C₂₄-Alkylgruppe, und o 0 - 24 ist; wobei der proximale N-gebundene GIcNAc-Rest an die N-verknüpfte Glykosylierungsstelle eines IgG-Antikörpers gebunden ist, umfassend eine N-verknüpfte Glykosylierungsstelle an der Kombination einer einzelnen schweren Kette und einer einzelnen leichten Kette, wobei die N-verknüpfte Glykosylierungsstelle eine mutierte N-verknüpfte Glykosylierungsstelle im Vergleich zu ihrem Wildtyp-Gegenstück ist, und wobei der proximal N-gebundene GIcNAc-Rest einer der Formel (138) ist, 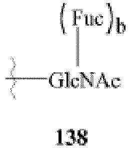 wobei b 0 oder 1 ist.

2. Verfahren nach Anspruch 1, wobei der Katalysator ausgewählt ist aus der Gruppe bestehend aus β(1,4)-Galactosyltransferasen umfassend eine mutierte katalytische Domäne, und β(1,3)-N-Galactosyltransferasen umfassend eine mutierte katalytische Domäne.

3. Verfahren nach Anspruch 1 oder Anspruch 2, wobei der Katalysator ein Katalysator umfassend eine mutierte katalytische Domäne aus einer β(1,4)-Galactosyltransferase, ist, ausgewählt aus der Gruppe bestehend aus bovinem β(1,4)-Gal-T1 GalT Y289L, GalTY289N, GalT Y289I, GalT Y289F, GalT Y289M, GalT Y289V, GalT Y289G und GalT Y289A.

4. Verfahren nach einem der vorangehenden Ansprüche, wobei Su(A)ₓ ausgewählt ist aus der Gruppe bestehend aus GalNAz-UDP, 6-AzGalNAc-UDP, 6-GalNAcCIUDP, 6-GalNAcSH-UDP, 2-GalNAcCI-UDP, 2-GalNAcSH-UDP, 6-CIGal-UDP, 2-ClGal-UDP, 2-HSGal-UDP und 6-HSGal-UDP.

5. IgG-Antikörper, umfassend eine N-verknüpfte Glykosylierungsstelle an der Kombination einer einzelnen schweren Kette und einer einzelnen leichten Kette, wobei die N-verknüpfte Glykosylierungsstelle eine mutierte N-verknüpfte Glykosylierungsstelle im Vergleich zu ihrem Wildtyp-Gegenstück ist, wobei ein proximal N-gebundener GlcNAc-Su(A)ₓ-Substituent an den Antikörper gebunden ist, wobei das GlcNAc in dem N-gebundenen GlcNAc-Su(A)ₓ-Substituenten optional fucosiliert ist, und wobei Su(A)ₓ definiert ist als ein Monosaccharidderivat Su umfassend x funktionelle Gruppen A, wobei x 1, 2, 3 oder 4 ist und wobei A ausgewählt ist aus der Gruppe, bestehend aus einer Azidogruppe, einer Ketogruppe, einer Alkinylgruppe, einer Thiolgruppe oder einem Vorläufer davon, einem Halogen, einer Sulfonyloxygruppe, einer halogenierten Acetamidogruppe, einer Mercaptoacetamidogruppe und einer sulfonylierten Hydroxyacetamidogruppe, wobei ein Vorläufer einer Thiolgruppe -[C(R⁷)₂]ₒSC(O)CH₃ ist, wobei R⁷ unabhängig ausgewählt ist aus der Gruppe bestehend aus Wasserstoff, Halogen und einer gegebenenfalls substituierten C₁ - C₂₄-Alkyl Gruppe, und o 0 - 24 ist.

6. Antikörper nach Anspruch 5, wobei der Antikörper einer der Formel (**140**) ist: 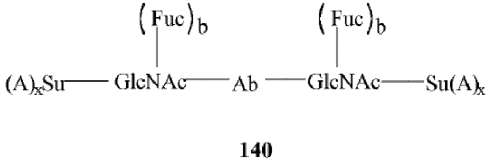 wobei:
Ab einen IgG-Antikörper umfassend eine N-verknüpfte Glykosilierungsstelle an der Kombination einer einzelnen schweren Kette und einer einzelnen leichten Kette darstellt, wobei die N-verknüpfte Glykosilierungsstelle eine mutierte N-verknüpfte Glykosilierungsstelle im Vergleich zu ihrem Wildtyp-Gegenstück ist; Su(A) und x sind wie in Anspruch 5 definiert; und b ist 0 oder 1.

7. Antikörper nach Anspruch 5 oder Anspruch 6, wobei A eine Azidgruppe, eine Thiolgruppe oder ein Halogen ist.

8. Verwendung des Antikörpers nach einem der Ansprüche 5 - 7 in der Herstellung eines Antikörper-Konjugats, wobei ein Antikörper-Konjugat definiert ist als ein Antikörper, welcher an ein Molekül von Interesse D via eines Linkers L konjugiert ist.

9. Verfahren zur Herstellung eines Antikörper-Konjugats, wobei das Verfahren das Umsetzen eines modifizierten Antikörpers nach einem der Ansprüche 5 - 7 mit einem Linker-Konjugat umfasst, wobei das Linker-Konjugat eine funktionelle Gruppe B und ein oder mehrere Moleküle von Interesse umfasst, wobei die funktionelle Gruppe B eine funktionelle Gruppe ist, die in der Lage ist, mit einer funktionellen Gruppe A auf einem Glykan des modifizierten Glykoproteins zu reagieren, und wobei die funktionelle Gruppe A wie in Anspruch 1 definiert ist.

10. Verfahren nach Anspruch 9, wobei:
(a) wenn der modifizierte Antikörper ein Azid-modifizierter Antikörper ist, umfasst das Linker-Konjugat eine (Hetero)Cycloalkinylgruppe oder eine Alkinylgruppe und ein oder mehrere Moleküle von Interesse; oder
(b) wenn der modifizierte Antikörper ein Keto-modifizierter Antikörper ist, umfasst das Linker-Konjugat eine primäre Amingruppe, eine Aminooxygruppe oder eine Hydrazinylgruppe und ein oder mehrere Moleküle von Interesse; oder
(c) wenn der modifizierte Antikörper ein Alkin-modifizierter Antikörper ist, umfasst das Linker-Konjugat eine Azidogruppe und ein oder mehrere Moleküle von Interesse.
(d) wenn der modifizierte Antikörper ein Thiol-modifizierter Antikörper oder ein Mercaptoamid-modifizierter Antikörper ist, umfasst das Linker-Konjugat eine N-Maleimid Gruppe oder eine halogenierte Acetamido-Gruppe oder ein Alken, und ein oder mehrere Moleküle von Interesse; oder
(e) wenn der modifizierte Antikörper ein Halogen-modifizierter Antikörper, ein halogeniertes Acetamid-modifizierter Antikörper, ein Sulfonyloxy-modifizierter Antikörper oder ein sulfonyliertes Hydroxyacetamido-modifizierter Antikörper ist, umfasst das Linker-Konjugat eine Thiolgruppe, und ein oder mehrere Moleküle von Interesse.

11. Antikörper-Konjugat erhältlich durch ein Verfahren nach einem der Ansprüche 9 - 10.

12. Antikörper-Konjugat nach Anspruch 11, wobei der Antikörper einer der Formeln (**154**), (**155**), (**156**) oder (**157**) ist: 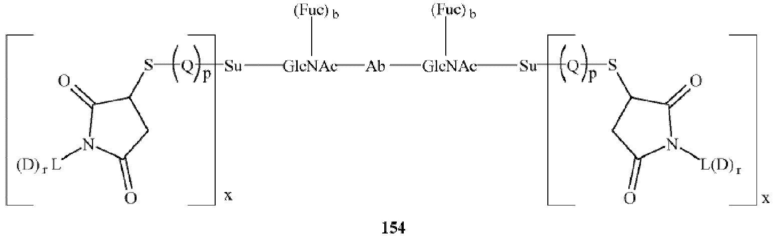 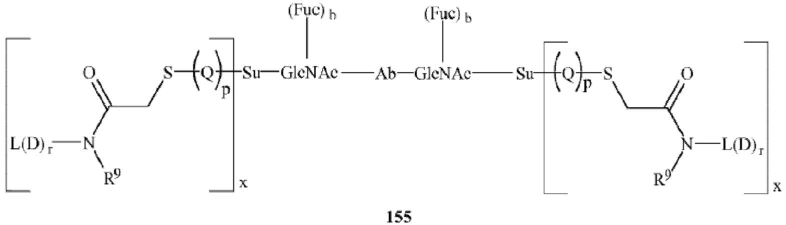 wobei:
Ab wie definiert in Anspruch 6 ist;
Su ein Zuckerderivat ist;
L ein Linker ist;
D ein Molekül von Interesse ist;
b 0 oder 1 ist;
r 1 bis 20 ist;
x 1, 2, 3 oder 4 ist;
p 0 oder 1 ist;
Q -N(H)C(O)CH₂- oder -CH₂- ist; und
R⁹ ausgewählt ist aus der Gruppe bestehend aus L(D)ᵣ, Wasserstoff, C₁ - C₂₄ Alkylgruppen, C₆ - C₂₄ Arylgruppen, C₇ - C₂₄ Alkylarylgruppen und C₇ - C₂₄ Arylalkylgruppen, die C₁ - C₂₄ Alkylgruppen, C₆ - C₂₄ Arylgruppen, C₇ - C₂₄ Alkylarylgruppen und C₇ - C₂₄ sind optional substituiert, vorzugsweise ist R⁹ ausgewählt aus der Gruppe bestehend aus L(D)ᵣ, Wasserstoff, C₁-C₁₂ Alkylgruppen, C₆ - C₁₂ Arylgruppen, C₇ - C₁₂ Alkylarylgruppen und C₇ - C₁₂ Arylalkylgruppen, die C₁ - C₁₂ Alkylgruppen, C₆ - C₁₂ Arylgruppen, C₇ - C₁₂ Alkylarylgruppen und C₇ - C₁₂ Arylalkylgruppen sind optional substituiert, weiter bevorzugt ist R⁹ ausgewählt aus der Gruppe bestehend aus L(D)ᵣ, Wasserstoff, C₁ - C₆ Alkylgruppen, C₆ - C₁₂ Arylgruppen, C₇ - C₁₂ Alkylarylgruppen und C₇ - C₁₂ Arylalkylgruppen, die C₁ - C₆ Alkylgruppen, C₆ -C₁₂ Arylgruppen, C₇ - C₁₂ Alkylarylgruppen und C₇ - C₁₂ Arylalkylgruppen sind optional substituier, noch weiter bevorzugt ist R⁹ H, C₁, C₂, C₄ oder C₄ Alkyl oder C₆ - C₁₂Aryl, am meisten bevorzugt ist R⁹ H oder Methyl; 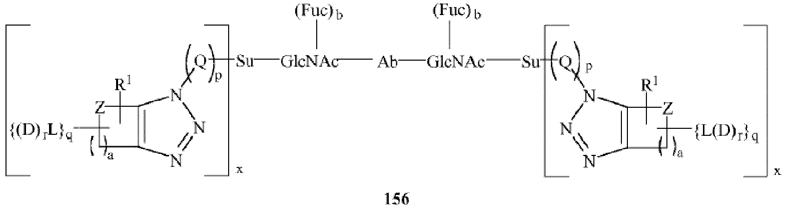
wobei:
Ab wie in Anspruch 6 definiert ist,
Su ein Zuckerderivat ist;
L ein Linker ist;
D ein Molekül von Interesse ist;
b 0 oder 1 ist;
r 1 - 20 ist;
x 1, 2 3 oder 4 ist;
y ist y ist 1 - 20;
R¹ unabhängig ausgewählt ist aus der Gruppe bestehend aus Wasserstoff, Halogen, -OR⁵, -NO₂, -CN, S(O₂)R⁵, C₁ - C₂₄ Alkylgruppen, C₆ - C₂₄ (Hetero)Arylgruppen, C₁ - C₂₄ Alkyl(hetero)arylgruppen und C₁ - C₂₄ (Hetero)Arylalkylgruppen und wobei die Alkylgruppen, (Hetero)Arylgruppen, Alkyl(hetero)Arylgruppen und (Hetero)Arylalkylgruppen optional substituiert sind, wobei zwei Substituenten R¹ miteinander verbunden sein können, um einen anellierten Cycloalkyl- oder einen anellierten (Hetero)Arensubstituenten zu bilden, und wobei R⁵ unabhängig ausgewählt ist aus der Gruppe bestehend aus Wasserstoff, Halogen, C₁ - C₂₄-Alkylgruppen, C₆ - C₂₄-(Hetero)Arylgruppen, C₁ - C₂₄-Alkyl(hetero)arylgruppen und C₁ - C₂₄-(Hetero)Arylalkylgruppen;
Z ist C(R¹)₂, O, S oder NR², wobei R² R¹ oder L(D)ᵣ ist, und wobei L, D und r wie oben definiert sind;
p ist 0 oder 1;
Q ist -N(H)C(O)CH₂- oder -CH₂-;
q ist 0 oder 1, mit der Maßgabe, dass, wenn q 0 ist, Z N-L(D)ᵣ ist;
a 1, 2, 3, 4, 5, 6, 7, 8, 9 oder 10 ist; 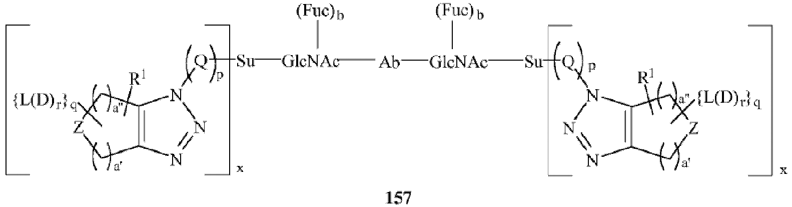
wobei
Ab wie in Anspruch 6 definiert ist;
Su ein Zuckerderivat ist;
L ein Linker ist;
D ein Molekül von Interesse ist;
b 0 oder 1 ist;
r 1 - 20 ist;
x 1, 2, 3 oder 4 ist;
y ist y ist 1 - 20;
R¹ unabhängig ausgewählt ist aus der Gruppe bestehend aus Wasserstoff, Halogen, -OR⁵, -NO₂, -CN, -S(O)₂R⁵, C₁-C₂₄-Alkylgruppen, C₆-C₂₄-(Hetero)Arylgruppen, C₇-C₂₄-Alkyl(hetero)Arylgruppen und C₇ - C₂₄-(Hetero)Arylalkylgruppen und wobei die Alkylgruppen, (Hetero)Arylgruppen, Alkyl(hetero)Arylgruppen und (Hetero)Arylalkylgruppen gegebenenfalls substituiert sind, wobei zwei Substituenten R¹ miteinander verbunden sein können, um einen anellierten Cycloalkyl- oder einen anellierten (Hetero)arensubstituenten zu bilden, und wobei R⁵ unabhängig ausgewählt ist aus der Gruppe bestehend aus Wasserstoff, Halogen, C₇ - C₂₄-Alkylgruppen, C₆ - C₂₄-(Hetero)arylgruppen, C₇ - C₂₄-Alkyl(hetero)Arylgruppen und C₇ - C₂₄ (Hetero)Arylalkylgruppen;
Z ist C(R¹)₂, O, S oder NR², wobei R² R¹ oder L(D)ᵣ ist, und wobei L, D und r wie oben definiert sind;
p ist 0 oder 1;
Q ist -N(H)C(O)CH₂- oder -CH₂-;
q 0 oder 1 ist, mit der Maßgabe, dass, wenn q 0 ist, Z N-L(D)ᵣ ist;
a' ist 0, 1, 2, 3, 4, 5, 6, 7 oder 8;
a" ist 0, 1, 2, 3, 4, 5, 6, 7 oder 8; und
a'+a"< 10.

13. Antikörper-Konjugat nach einem der Ansprüche 11 - 12, wobei x 1 oder 2 ist, und/oder r 1 oder 2 ist.

14. Antikörper-Konjugat nach einem der Ansprüche 11 - 13, wobei das Molekül von Interesse ausgewählt ist aus der Gruppe von pharmazeutisch aktiven Substanzen.

15. Antikörper-Konjugat nach einem der Ansprüche 11 - 14, zur Verwendung als Medikament.

## Revendications

1. Procédé pour la préparation d'un anticorps modifié, le procédé comprenant la fixation d'un dérivé de monosaccharide Su(A)ₓ à un résidu de GlcNAc N-lié proximal, en présence d'un catalyseur choisi dans le groupe comprenant *β*(1,4)-galactosyltransférases, *β*(1,3)-N-galactosyltransférases, *β*(1,4)-galactosyltransférases comprenant un domaine catalytique mutant et *β*(1,3)-N-galactosyltransférases comprenant un domaine catalytique mutant ; où Su(A)ₓ est défini comme un dérivé de monosaccharide Su comprenant x groupes fonctionnels A où x est 1, 2, 3 ou 4, et où A est choisi dans le groupe comprenant un groupe azido, un groupe céto, un groupe alcynyle, un groupe thiol ou un précurseur de ceux-ci, un halogène, un groupe sulfonyloxy, un groupe acétamido halogéné, un groupe mercaptoacétamido et un groupe hydroxyacétamido sulfonylé, où un précurseur d'un groupe thiol est -[C(R⁷)₂]ₒSC(O)CH₃, où R⁷ est choisi indépendamment dans le groupe comprenant hydrogène, halogène et un groupe alkyle en C₁-C₂₄ facultativement substitué, et o est 0 à 24 ; où ledit résidu de GlcNAc N-lié proximal est attaché au site de glycosylation N-lié d'un anticorps IgG comprenant un site de glycosylation N-lié sur la combinaison d'une chaîne lourde unique et d'une chaîne légère unique, où ledit site de glycosylation N-lié est un site de glycosylation N-lié mutant par comparaison à son homologue de type sauvage, et où le résidu de GlcNAc N-lié proximal est conforme à la formule (138) : 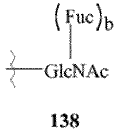 où b vaut 0 ou 1.

2. Procédé selon la revendication 1, où le catalyseur est choisi dans le groupe constitué de *β*(1,4)-galactosyltransférases comprenant un domaine catalytique mutant et de *β*(1,3)-N-galactosyltransférases comprenant un domaine catalytique mutant.

3. Procédé selon la revendication 1 ou la revendication 2, dans lequel le catalyseur est un catalyseur comprenant un domaine catalytique mutant provenant d'une *β*(1,4)-galactosyltransférase, choisie dans le groupe constitué par la *β*(1,4)-Gal-T1 bovine GAlT Y289L, GAlT Y289N, GalT Y289I, GalT Y289F, GalT Y289M, GalT Y289V, GalT Y289G et GalT Y289A.

4. Procédé selon l'une quelconque des revendications précédentes, dans lequel Su(A)ₓ est choisi dans le groupe comprenant GAINAz-UDP, 6-AzGAlNAc-UDP, 6-GAlNAcCl-UDP, 6-GalNAcSH-UDP, 2-GAlNAcCl-UDP, 2-GAlNAcSH-UDP, 6-ClGal-UDP, 2-ClGal-UDP, 2-HSGal-UDP et 6-HSGal-UDP.

5. Anticorps IgG comprenant un site de glycosylation N-lié sur la combinaison d'une chaîne lourde et d'une seule chaîne légère, où le site de glycosylation N-lié est un site de glycosylation N-lié mutant par rapport à son homologue de type sauvage, où un GlcNAc-Su(A)ₓ-substituant N-lié proximal est attaché à l'anticorps, où le GlcNAc dans le GlcNAc-Su(A)ₓ-substituant N-lié est facultativement fucosylé, et où Su(A)ₓ est défini comme un dérivé de monosaccharide Su comprenant x groupes fonctionnels A où x est 1, 2, 3 ou 4, et où A est choisi dans le groupe comprenant un groupe azido, un groupe céto, un groupe alcynyle, un groupe thiol ou un précurseur de ceux-ci, un halogène, un groupe sulfonyloxy, un groupe acétamido halogéné, un groupe mercaptoacétamido et un groupe hydroxyacétamido sulfonylé, où un précurseur d'un groupe thiol est -[C(R⁷)₂]ₒSC(O)CH₃, où R⁷ est choisi indépendamment dans le groupe comprenant hydrogène, halogène et un groupe alkyle en C₁-C₂₄ facultativement substitué, et o est 0 à 24.

6. Anticorps selon la revendication 5, où l'anticorps est selon la formule (140) : 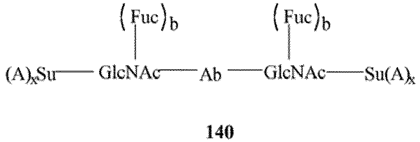 où :
Ab représente un anticorps IgG comprenant un site de glycosylation N-lié sur la combinaison d'une chaîne lourde unique et d'une chaîne légère unique, où le site de glycosylation N-lié est un site de glycosylation N-lié mutant par comparaison à son homologue de type sauvage ; Su(A) et x sont tels que définis dans la revendication 5 ; et b vaut 0 ou 1.

7. Anticorps selon la revendication 5 ou la revendication 6, dans lequel A est un groupe azide, un groupe thiol ou un halogène.

8. Utilisation de l'anticorps selon l'une quelconque des revendications 5 à 7 dans la préparation d'un anticorps conjugué, dans laquelle un anticorps conjugué est défini comme un anticorps qui est conjugué à une molécule d'intérêt D via un lieur L.

9. Procédé de préparation d'un anticorps conjugué, ledit procédé comprenant l'étape consistant à faire réagir un anticorps modifié selon l'une quelconque des revendications 5 à 7 avec un lieur conjugué, où ledit lieur conjugué comprend un groupe fonctionnel B et une ou plusieurs molécules de d'intérêt, où ledit groupe fonctionnel B est un groupe fonctionnel qui est capable de réagir avec un groupe fonctionnel A sur un glycane de la glycoprotéine modifiée, et où le groupe fonctionnel A est tel que défini dans la revendication 1.

10. Procédé selon la revendication 9, dans lequel :
(a) lorsque l'anticorps modifié est un anticorps modifié par un azide, le lieur conjugué comprend un groupe (hétéro)cycloalcynyle ou un groupe alcynyle, et une ou plusieurs molécules d'intérêt ; ou
(b) lorsque l'anticorps modifié est un anticorps céto-modifié, le lieur conjugué comprend un groupe amine primaire, un groupe aminooxy ou un groupe hydrazinyle, et une ou plusieurs molécules d'intérêt ; ou
(c) lorsque l'anticorps modifié est un anticorps modifié par un alcyne, le lieur conjugué comprend un groupe azido et une ou plusieurs molécules d'intérêt ;
(d) lorsque l'anticorps modifié est un anticorps modifié par un thiol ou un anticorps modifié par un mercaptoacétamide, le lieur conjugué comprend un groupe N-maléimide ou un groupe acétamido halogéné ou un alcène, et une ou plusieurs molécules d'intérêt ; ou
(e) lorsque l'anticorps modifié est un anticorps modifié par un halogène, un anticorps modifié par un acétamide halogéné, un anticorps modifié par un sulfonyloxy ou un anticorps modifié par un hydroxyacétamido sulfonylé, le lieur conjugué comprend un groupe thiol et une ou plusieurs molécules d'intérêt.

11. Anticorps conjugué pouvant être obtenu par le procédé selon l'une quelconque des revendications 9 à 10.

12. Anticorps conjugué selon la revendication 11, dans lequel l'anticorps est selon la formule (**154**), (**155**), (**156**) ou (**157**) : 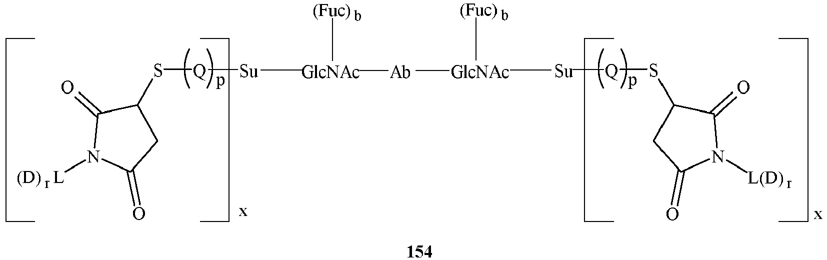 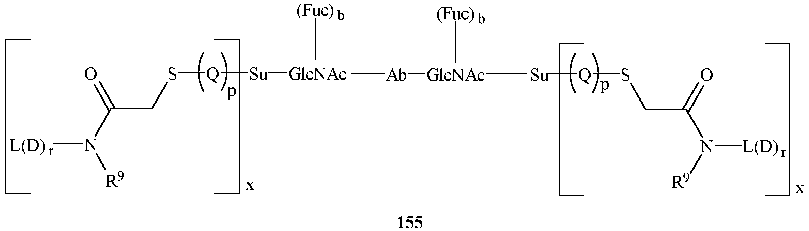 où :
Ab est tel que défini dans la revendication 6 ;
Su est un dérivé de sucre ;
L est un lieur ;
D est une molécule d'intérêt ;
b vaut 0 ou 1 ;
r vaut 1 à 20 ;
x vaut 1, 2, 3 ou 4 ;
p vaut 0 ou 1 ;
Q est -N(H)C(O)CH₂- ou -CH₂- ; et
R⁹ est choisi dans le groupe consistant en L(D)ᵣ, hydrogène, des groupes alkyle en C₁-C₂₄, des groupes aryle en C₆-C₂₄, des groupes alkylaryle en C₇-C₂₄ et des groupes arylalkyle en C₇-C₂₄, les groupes alkyle en C₁-C₂₄, les groupes aryle en C₆-C₂₄, les groupes alkylaryle en C₇-C₂₄ et les groupes arylalkyle en C₇-C₂₄ étant facultativement substitués ; de préférence, R⁹ est choisi dans le groupe consistant en L(D)ᵣ, hydrogène, des groupes alkyle en C₁-C₁₂, des groupes aryle en C₆-C₁₂, des groupes alkylaryle en C₇-C₁₂ et des groupes arylalkyle en C₇-C₁₂, les groupes alkyle en C₁-C₁₂, les groupes aryle en C₆-C₁₂, les groupes alkylaryle en C₇-C₁₂ et les groupes arylalkyle en C₇-C₁₂ étant facultativement substitués ; de manière encore plus préférée, R⁹ est choisi dans le groupe consistant en L(D)ᵣ, hydrogène, des groupes alkyle en C₁-C₆, des groupes aryle en C₆-C₁₂, des groupes alkylaryle en C₇-C₁₂ et des groupes arylalkyle en C₇-C₁₂, les groupes alkyle en C₁-C₆, les groupes aryle en C₆-C₁₂, les groupes alkylaryle en C₇-C₁₂ et les groupes arylalkyle en C₇-C₁₂ étant facultativement substitués ; de manière encore plus préférée, R⁹ est H, alkyle en C₁, C₂, C₄ ou C₄ ou aryle en C₆-C₁₂ ; de la manière la plus préférée, R⁹ est H ou méthyle ; 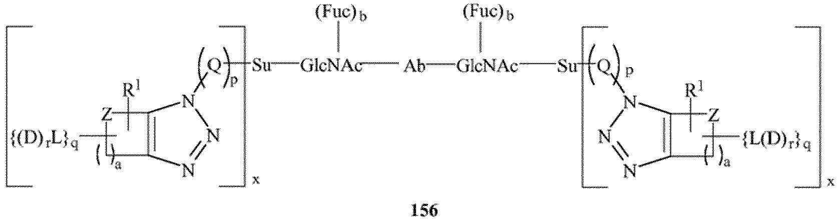
où :
Ab est tel que défini dans la revendication 6 ;
Su est un dérivé de sucre ;
L est un éditeur de liens ;
D est une molécule d'intérêt ;
b vaut 0 ou 1 ;
r vaut 1 à 20 ;
x vaut 1, 2, 3 ou 4 ;
y vaut 1 à 20 ;
R¹ est indépendamment sélectionné dans le groupe consistant en hydrogène, halogène, -OR⁵, -NO₂, -CN, -S(O)₂R⁵, des groupes alkyle en C₁-C₂₄, des groupes (hétéro)aryle en C₆-C₂₄, des groupes alkyl(hétéro)aryle en C₇-C₂₄ et des groupes (hétéro)arylalkyle en C₇-C₂₄ et où les groupes alkyle, les groupes (hétéro)aryle, les groupes alkyl(hétéro)aryle et les groupes (hétéro)arylalkyle sont facultativement substitués, où deux substituants R¹ peuvent être liés ensemble pour former un cycloalkyle annelé ou un substituant (hétéro)arène annelé, et où R⁵ est choisi indépendamment dans le groupe consistant en hydrogène, halogène, des groupes alkyle en C₁-C₂₄, des groupes(hétéro)aryle en C₆-C₂₄, des groupes alkyl(hétéro)aryle en C₇-C₂₄, et des groupes (hétéro)arylalkyle en C₇-C₂₄ ;
Z est C(R¹)₂, O, S ou NR², où R² est R¹ ou L(D)ᵣ, et où L, D et r sont tels que définis ci-dessus ;
p vaut 0 ou 1 ;
Q est -N(H)C(O)CH₂- ou -CH₂- ;
q vaut 0 ou 1, à condition que si q est 0 alors Z est N-L(D)ᵣ ;
a vaut 1, 2, 3, 4, 5, 6, 7, 8, 9 ou 10 ; 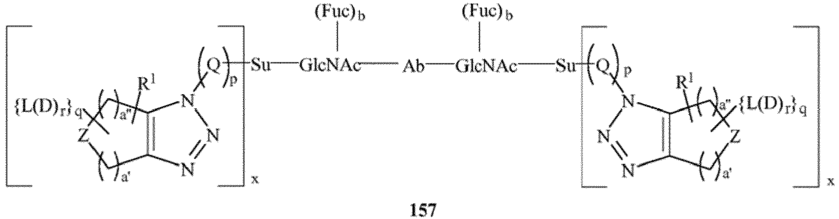
où :
Ab est tel que défini dans la revendication 6 ;
Su est un dérivé de sucre ;
L est un lieur ;
D est une molécule d'intérêt ;
b vaut 0 ou 1 ;
r vaut 1 à 20 ;
x vaut 1, 2, 3 ou 4 ;
y vaut 1 à 20 ;
R¹ est indépendamment sélectionné dans le groupe consistant en hydrogène, halogène, -OR⁵, -NO₂, -CN, -S(O)₂R⁵, des groupes alkyle en C₁-C₂₄, des groupes (hétéro)aryle en C₆-C₂₄, des groupes alkyl(hétéro)aryle en C₇-C₂₄, et des groupes (hétéro)arylalkyle en C₇-C₂₄ et où les groupes alkyle, les groupes (hétéro)aryle, les groupes alkyl(hétéro)aryle et les groupes (hétéro)arylalkyle sont facultativement substitués, où deux substituants R¹ peuvent être liés ensemble pour former un cycloalkyle annelé ou un substituant (hétéro)arène annelé, et où R⁵ est indépendamment choisi dans le groupe consistant en hydrogène, halogène, des groupes alkyle en C₁-C₂₄, des groupes (hétéro)aryle en C₆-C₂₄, des groupes alkyl(hétéro)aryle en C₇-C₂₄ des groupes et des groupes (hétéro)arylalkyle en C₇-C₂₄ ;
Z est C(R¹)₂, O, S ou NR², où R² est R¹ ou L(D)ᵣ, et où L, D et r sont tels que définis ci-dessus ;
p vaut 0 ou 1 ;
Q est -N(H)C(O)CH₂- ou -CH₂- ;
q vaut 0 ou 1, à condition que si q est 0 alors Z est N-L(D)ᵣ ;
a' vaut 0, 1, 2, 3, 4, 5, 6, 7 ou 8 ;
a" vaut 0, 1, 2, 3, 4, 5, 6, 7 ou 8 ; et
a' + a" < 10.

13. Anticorps selon l'une quelconque des revendications 11 à 12, dans lequel x vaut 1 ou 2, et/ou r vaut 1 ou 2.

14. Anticorps conjugué selon l'une quelconque des revendications 11 à 13, dans lequel la molécule d'intérêt est choisie dans le groupe de substances pharmaceutiquement actives.

15. Anticorps conjugué selon l'une quelconque des revendications 11 à 14, destiné à être utilisé comme médicament.
